# EUROPÄISCHE PATENTSCHRIFT

(11) **EP 1 261 609 B1**
(45) Veröffentlichungstag und Bekanntmachung des Hinweises auf die Patenterteilung: **03.11.2004**
(21) Anmeldenummer: 00993612.1
(22) Anmeldetag: 12.12.2000
(51) Int. Cl.: C07D 487/04, A61P 9/10

(54) **TRIAZOLOTRIAZINONE UND IHRE VERWENDUNG**
TRIAZOLOTRIAZINONES AND THE USE THEREOF
TRIAZOLOTRIAZINONES ET LEUR UTILISATION

(30) Priorität: 24.12.1999 DE 19962927; 27.01.2000 DE 10003296
(43) Veröffentlichungstag der Anmeldung: 04.12.2002
(73) Patentinhaber: Bayer HealthCare AG, 51368 Leverkusen (DE)
(72) Erfinder: NIEWÖHNER, Ulrich, 42929 Wermelskirchen (DE); HANING, Helmut, US Milford CT 06460 (US); LAMPE, Thomas, 42105 Wuppertal (DE); ES-SAYED, Mazen, 40764 Langenfeld (DE); SCHMIDT, Gunter, 42115 Wuppertal (DE); BISCHOFF, Erwin, 42115 Wuppertal (DE); DEMBOWSKY, Klaus, Boston, MA 02116 (US); PERZBORN, Elisabeth, 42327 Wuppertal (DE); SCHLEMMER, Karl-Heinz, 42113 Wuppertal (DE)
(86) Internationale Anmeldenummer: PCT/EP2000/012592
(87) Internationale Veröffentlichungsnummer: WO 2001/047929

(56) Entgegenhaltungen:
- EP-A- 0 463 756
- WO-A-93/06104
- WO-A-94/00453
- WO-A-98/49166
- WO-A-99/24433
- DE-A- 19 812 462
- US-A- 5 346 901
- WERMUTH C.G.: 'The Practise of Medicinal Chemistry' PRACTICE OF MEDICINAL CHEMISTRY 1996, Seiten 203 - 237, XP002190259
- 4 Dokumente in der mündlichen Verhandlung eingereicht (vgl. Annex)

## Beschreibung

Die vorliegende Erfindung betrifft neue Triazolotriazinone, Verfahren zu ihrer Herstellung und ihre Verwendung als Arzneimittel, insbesondere als Inhibitoren cGMP-metabolisierender Phosphodiesterasen.

In J. Heterocycl. Chem. (1993), 30(5), 1341-9, sowie in J. Heterocycl. Chem. (1984), 21(3), 697 -9 und in Nucleosides Nucleotides (1995), 14(7), 1601-12 werden 6-Amino-triazolotriazinone mit antiviraler Wirkung beschrieben.

In J. Med. Chem. (1986), 29(11), 2231-5 werden ebenfalls 6-Amino-triazolotriazinone als Nucleosidanaloga mit Antitumorwirkung beschrieben. Triazolotriazinone mit den in der vorliegenden Erfindung beschriebenen Substituenten und mit inhibitorischer Wirkung gegen cGMP-metabolisierende Phosphodiesterasen sind nicht bekannt.

Die erfindungsgemäßen Verbindungen sind potente Inhibitoren der cyclischen Guanosin 3',5'-monophophat metabolisierenden Phosphodiesterasen (cGMP -PDE's). Entsprechend der Nomenklatur von Beavo und Reifsnyder (Trends in Pharmacol. Sci. 11, 150-155, 1990) handelt es sich um die Phosphodiesterase Isoenzyme PDE-I, PDE-II und PDE-V.

Inhibitoren dieser Phosphodiesterasen sind aus WO 99/244 33 bekannt.

Ein Anstieg der cGMP-Konzentration kann zu heilsamen, antiaggregatorischen, antithrombotischen, antiproliferativen, antivasospastischen, vasodilatierenden, natriuretischen und diuretischen Effekten fuhren. Es kann die Kurz- oder Langzeitmodulation der vaskulären und kardialen Inotropie, den Herzrhythmus und die kardiale Erregungsleitung beeinflussen (J. C. Stoclet, T. Keravis, N. Komas and C. Kugnier, Exp. Opin. Invest. Drugs (1995), 4 (11), 1081-1100). Die Inhibition der cGMP-PDE's kann auch eine Verstärkung der Erektion bewirken. Daher sind solche Verbindungen zur Behandlung von erektilen Dysfunktionen geeignet.

Die vorliegende Erfindung betrifft nun Triazolotriazinone der allgemeinen Formel (I), 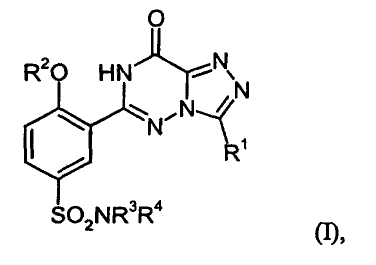 in welcher
- R¹: für (C₃-C₈)-Cycloalkyl steht,
- R²: für Wasserstoff oder für geradkettiges oder verzweigtes Alkyl mit bis zu 6 Kohlenstoffatomen steht,
- R³ und R⁴: gleich oder verschieden sind und für Wasserstoff, (C₁-C₆)-Alkoxy oder für (C₁-C₆)-Alkyl stehen, das gegebenenfalls bis zu 3-fach, gleich oder verschieden durch Hydroxy, (C₁-C₅)-Alkoxy, Phenoxy oder durch Reste der Formeln

―O-CO-NR⁵R⁶ ,

―NR⁷R⁸

oder 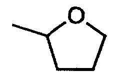 substituiert ist,
worin
R⁵, R⁶, R⁷ und R⁸ gleich oder verschieden sind und Wasserstoff, (C₁-C₆)-Alkyl oder Phenyl bedeuten, oder
R⁷ und R⁸ gemeinsam mit dem Stickstoffatom, an das sie gebunden sind, einen 5- bis 6-gliedrigen, gesättigten Heterocyclus bilden, der noch ein weiteres Heteroatom aus der Reihe S und O enthalten kann,
und/oder seinerseits (C₁-C₆)-Alkyl gegebenenfalls durch Phenyl substituiert ist, das gegebenenfalls bis zu 3-fach gleich oder verschieden durch Hydroxy, (C₁-C₆)-Alkoxy, Halogen oder durch (C₁-C₆)-Alkyl substituiert ist, das seinerseits wiederum durch Hydroxy oder (C₁-C₆)-Alkoxy substituiert ist, oder Phenyl gegebenenfalls durch Reste der Formeln -SO₂-NR⁹R¹⁰ oder -NR¹¹R¹² substituiert ist,
worin
R⁹, R¹⁰, R¹¹ und R¹² gleich oder verschieden sind und Wasserstoff, (C₁-C₆)-Alkyl oder Phenyl bedeuten,
oder
R¹¹ und R¹² gemeinsam mit dem Stickstoffatom, an das sie gebunden sind, einen 5- bis 6-gliedrigen, gesättigten Heterocyclus bilden, der noch ein weiteres Heteroatom aus der Reihe S und O enthalten kann,
oder
- R³: für Wasserstoff oder (C₁-C₆)-Alkyl steht,
und
- R⁴: für Reste der Formel 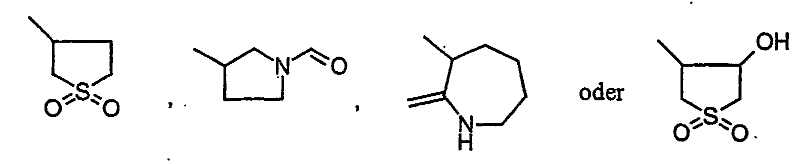 steht,
oder
für Phenyl steht, das gegebenenfalls bis zu 3-fach, gleich oder verschieden durch Halogen, (C₁-C₆)-Alkoxy, Hydroxy, durch einen Rest der Formel 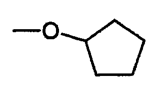 oder durch (C₁-C₆)-Alkyl substituiert ist, das seinerseits durch Hydroxy oder (C₁-C₆)-Alkoxy substituiert sein kann,
oder
- R³ und R⁴: gemeinsam mit dem Stickstoffatom, an das sie gebunden sind, einen Rest der Formel 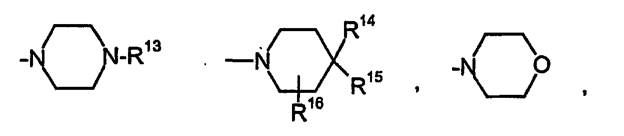 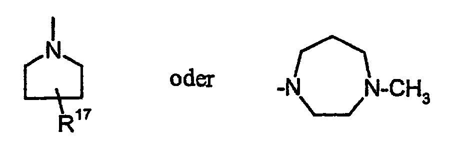 bilden,
worin
R¹³ Wasserstoff, (C₁-C₆)-Alkoxycarbonyl, (C₃-C₆)-Cycloalkyl, Pyridyl, Pyrimidyl oder (C₁-C₆)-Alkyl bedeutet, das gegebenenfalls durch Hydroxy substituiert ist,
R¹⁴ und R¹⁵ gleich oder verschieden sind und Wasserstoff, Hydroxy oder (C₁-C₆)-Alkyl bedeuten, das gegebenenfalls durch Hydroxy oder durch einen Rest der Formel -P(O)(OR¹⁸)(OR¹⁹) substituiert ist,
worin
R¹⁸ und R¹⁹ gleich oder verschieden sind und Wasserstoff oder (C₁-C₆)-Alkyl bedeuten, oder
R¹⁴ und R¹⁵ gemeinsam einen Rest der Formel =N-OH bilden,
R¹⁶ und R¹⁷ gleich oder verschieden sind und Wasserstoff oder (C₁-C₆)-Alkyl bedeuten, das gegebenenfalls durch Hydroxy substituiert ist,
und deren Salze, N-Oxide und stereo-isomere Formen.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Die Racemformen lassen sich ebenso wie die Diastereomeren in bekannter Weise in die stereoisomer einheitlichen Bestandteile trennen.

Die erfindungsgemäßen Stoffe können auch als Salze vorliegen. Im Rahmen der Erfindung sind physiologisch unbedenkliche Salze bevorzugt.

Physiologisch unbedenkliche Salze können Salze der erfindungsgemäßen Verbindungen mit anorganischen oder organischen Säuren sein. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Physiologisch unbedenkliche Salze können ebenso Metall- oder Ammoniumsalze der erfindungsgemäßen Verbindungen sein. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di-bzw. Triethylamin, Di- bzw. Triethanolamin, Dicyclohexylamin, Dimethylaminoethanol, Arginin, Lysin, Ethylendiamin oder 2-Phenylethylamin

(C₃-C₈)-Cycloalkyl bzw. (C₃-C₆)-Cycloalkyl steht für Cyclopropyl, Cyclopentyl, Cyclobutyl, Cyclohexyl, Cycloheptyl oder Cyclooctyl. Bevorzugt seien genannt: Cyclopropyl, Cyclopentyl und Cyclohexyl.

(C₁-C₆)-Alkyl steht für einen geradkettigen oder verzweigten Alkylrest mit 1 bis 6 Kohlenstoffatomen. Beispielsweise seien genannt: Methyl, Ethyl, n-Propyl, Isopropyl, n-Butyl, Isobutyl, tert.-Butyl, n-Pentyl und n-Hexyl. Bevorzugt ist ein geradkettiger oder verzweigter Alkylrest mit 1 bis 4 Kohlenstoffatomen. Besonders bevorzugt ist ein geradkettiger oder verzweigter Alkylrest mit 1 bis 3 Kohlenstoffatomen.

(C₁-C₆)-Alkoxy steht für einen geradkettigen oder verzweigten Alkoxyrest mit 1 bis 6 Kohlenstoffatomen. Beispielsweise seien genannt: Methoxy, Ethoxy, n-Propoxy, Isopropoxy, n-Butoxy, Isobutoxy, tert.-Butoxy, n-Pentoxy und n-Hexoxy. Bevorzugt ist ein geradkettiger oder verzweigter Alkoxyrest mit 1 bis 4 Kohlenstoffatomen. Besonders bevorzugt ist ein geradkettiger oder verzweigter Alkoxyrest mit 1 bis 3 Kohlenstoffatomen.

(C₁-C₆)-Alkoxycarbonyl steht im Rahmen der Erfindung für einen geradkettigen oder verzweigten Alkoxycarbonylrest mit 1 bis 6 Kohlenstoffatomen. Beispielsweise seien genannt: Methoxycarbonyl, Ethoxycarbonyl, n-Propoxycarbonyl, Isopropoxycarbonyl und tert.Butoxycarbonyl. Bevorzugt ist ein geradkettiger oder verzweigter Alkoxycarbonylrest mit 1 bis 4 Kohlenstoffatomen. Besonders bevorzugt ist ein geradkettiger oder verzweigter Alkoxycarbonylrest mit 1 bis 3 Kohlenstoffatomen.

Halogen steht im allgemeinen für Fluor, Chlor, Brom und Jod. Bevorzugt sind Fluor, Chlor und Brom. Besonders bevorzugt sind Fluor und Chlor.

Ein 5- bis 6-gliedriger aromatischer Heterocyclus mit bis zu 3 Heteroatomen aus der Reihe S, O und/oder N steht beispielsweise für Pyridyl, Pyrimidyl, Pyridazinyl, Thienyl, Furyl, Pyrrolyl, Thiazolyl, Oxazolyl oder Imidazolyl. Bevorzugt sind Pyridyl, Pyrimidyl, Pyridazinyl, Furyl und Thienyl.

Bevorzugt sind erfindungsgemäße Verbindungen der allgemeinen Formel (I),
in welcher
- R¹: für Cyclopropyl, Cyclopentyl oder Cyclohexyl steht,
- R²: für geradkettiges oder verzweigtes Alkyl mit bis zu 4 Kohlenstoffatomen steht,
- R³ und R⁴: gleich oder verschieden sind und für Wasserstoff, Methoxy oder für (C₁-C₅)-Alkyl stehen, das gegebenenfalls bis zu 3-fach, gleich oder verschieden durch Hydroxy, (C₁-C₄)-Alkoxy, Phenoxy oder durch Gruppen der Formeln

―O-CO-NR⁵R⁶ ,

―NR⁷R⁸

oder 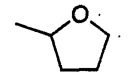 substituiert ist,
worin
R⁵, R⁶, R⁷ und R⁸ gleich oder verschieden sind und Wasserstoff, (C₁-C₄)-Alkyl oder Phenyl bedeuten, oder
R⁷ und R⁸ gemeinsam mit dem Stickstoffatom, an das sie gebunden sind, einen Morpholin-, Piperidin- oder Pyrrolidinring bilden,
und/oder seinerseits (C₁-C₅)-Alkyl gegebenenfalls durch Phenyl substituiert ist, das gegebenenfalls bis zu 3-fach gleich oder verschieden durch Hydroxy, (C₁-C₄)-Alkoxy oder durch (C₁-C₄)-Alkyl substituiert sein kann, das seinerseits wiederum durch Hydroxy oder (C₁-C₄)-Alkoxy substituiert ist, oder Phenyl gegebenenfalls durch Reste der Formeln -SO₂-NR⁹R¹⁰ oder -NR¹¹R¹² substituiert ist,
worin
R⁹, R¹⁰, R¹¹ und R¹² gleich oder verschieden sind und Wasserstoff, (C₁-C₄)-Alkyl oder Phenyl bedeuten, oder
R¹¹ und R¹² gemeinsam mit dem Stickstoffatom, an das sie gebunden sind, einen Morpholin-, Piperidin- oder Pyrrolidinring bilden,
oder
- R³: für Wasserstoff oder (C₁-C₄)-Alkyl steht,
und
- R⁴: für Reste der Formel 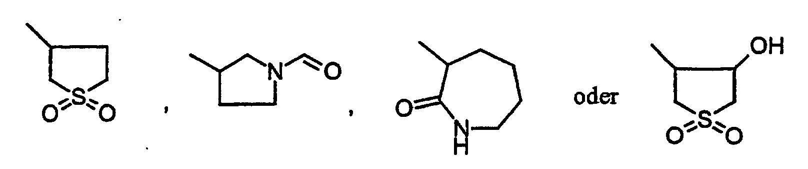 steht,
oder
für Phenyl steht, das gegebenenfalls bis zu 3-fach, gleich oder verschieden durch Fluor, (C₁-C₄)-Alkoxy, Hydroxy, durch einen Rest der Formel 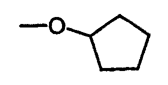 oder durch (C₁-C₄)-Alkyl substituiert ist, das seinerseits durch Hydroxy oder (C₁-C₃)-Alkoxy substituiert sein kann,
oder
- R³ und R⁴: gemeinsam mit dem Stickstoffatom, an das sie gebunden sind, einen Rest der Formel 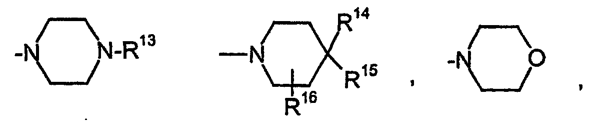 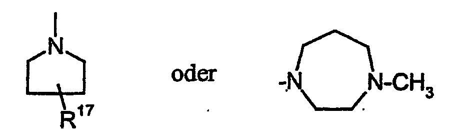 bilden,
worin
R¹³ Wasserstoff, (C₁-C₄)-Alkoxycarbonyl, Cyclopentyl, Cyclohexyl, Pyridyl, Pyrimidyl oder (C₁-C₅)-Alkyl bedeutet, das gegebenenfalls durch Hydroxy substituiert ist,
R¹⁴ und R¹⁵ gleich oder verschieden sind und Wasserstoff oder (C₁-C₅)-Alkyl bedeuten, das gegebenenfalls durch Hydroxy oder durch einen Rest der Formel -P(O)(OR¹⁸)(OR¹⁹) substituiert ist,
worin
R¹⁸ und R¹⁹ gleich oder verschieden sind und Wasserstoff , Methyl oder Ethyl bedeuten, oder
R¹⁴ und R¹⁵ gemeinsam einen Rest der Formel =N-OH bilden,
R¹⁶ und R¹⁷ gleich oder verschieden sind und Wasserstoff, Hydroxy oder (C₁-C₃)-Alkyl bedeuten, das gegebenenfalls durch Hydroxy substituiert ist,
und deren Salze, N-Oxide und stereo-isomere Formen.

Besonders bevorzugt sind Verbindungen der allgemeinen Formel (I),
in welcher
- R¹: für Cyclopentyl steht,
- R²: für geradkettiges oder verzweigtes Alkyl mit bis zu 3 Kohlenstoffatomen steht,
- R³ und R⁴: gleich oder verschieden sind und für Wasserstoff, Methoxy oder für (C₁-C₄)-Alkyl stehen, das gegebenenfalls bis zu 3-fach, gleich oder verschieden durch Hydroxy, (C₁-C₄)-Alkoxy, Phenoxy oder durch Gruppen der Formeln

―O-CO-NR⁵R⁶ ,

―NR⁷R⁸

oder 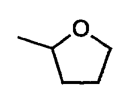 substituiert ist,
worin
R⁵, R⁶, R⁷ und R⁸ gleich oder verschieden sind und Wasserstoff, (C₁-C₃)-Alkyl oder Phenyl bedeuten, oder
R⁷ und R⁸ gemeinsam mit dem Stickstoffatom, an das sie gebunden sind, einen Morpholin-, Piperidin- oder Pyrrolidinring bilden,
und/oder seinerseits (C₁-C₄)-Alkyl gegebenenfalls durch Phenyl substituiert ist, das gegebenenfalls bis zu 3-fach gleich oder verschieden durch Hydroxy, (C₁-C₃)-Alkoxy, Fluor oder durch (C₁-C₃)-Alkyl substituiert ist, das seinerseits wiederum durch Hydroxy oder (C₁-C₄)-Alkoxy substituiert ist, oder Phenyl gegebenenfalls durch Reste der Formeln -SO₂-NR⁹R¹⁰ oder -NR¹¹R¹² substituiert ist,
worin
R⁹, R¹⁰, R¹¹ und R¹² gleich oder verschieden sind und Wasserstoff, (C₁-C₃)-Alkyl oder Phenyl bedeuten, oder
R¹¹ und R¹² gemeinsam mit dem Stickstoffatom, an das sie gebunden sind, einen Morpholin-, Piperidin- oder Pyrrolidinring bilden,
oder
- R³: für Wasserstoff oder Methyl steht,
und
- R⁴: für Reste der Formel 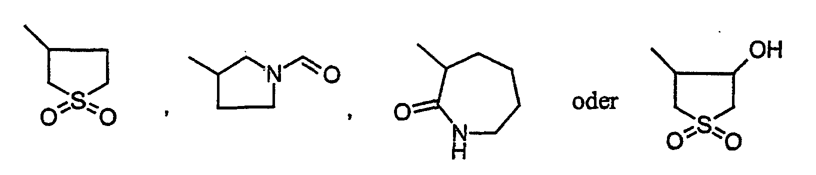 steht,
oder
für Phenyl steht, das gegebenenfalls bis zu 3-fach, gleich oder verschieden durch Fluor, Methoxy, Hydroxy, durch einen Rest der Formel 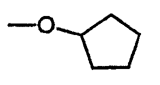 oder durch (C₁-C₄)-Alkyl substituiert ist, das seinerseits durch Hydroxy oder Methoxy oder Ethoxy substituiert sein kann,
oder
- R³ und R⁴: gemeinsam mit dem Stickstoffatom, an das sie gebunden sind, einen Rest der Formel 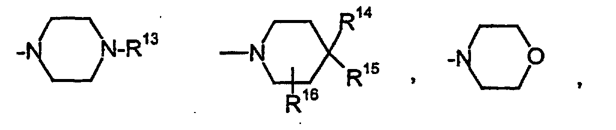 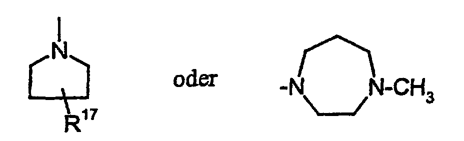 bilden,
worin
R¹³ Wasserstoff, (C₁-C₄)-Alkoxycarbonyl, Cyclopentyl, Pyrimidyl oder (C₁-C₃)-Alkyl bedeutet, das gegebenenfalls durch Hydroxy substituiert ist,
R¹⁴ und R¹⁵ gleich oder verschieden sind und (C₁-C₃)-Alkyl bedeuten, das gegebenenfalls durch Hydroxy oder durch einen Rest der Formel -P(O)(OR¹⁸)(OR¹⁹) substituiert ist,
worin
R¹⁸ und R¹⁹ Ethyl bedeuten, oder
R¹⁴ und R¹⁵ gemeinsam einen Rest der Formel =N-OH bilden,
R¹⁶ und R¹⁷ gleich oder verschieden sind und Wasserstoff oder (C₁-C₃)-Alkyl bedeuten, das gegebenenfalls durch Hydroxy substituiert ist,
und deren Salze, N-Oxide und stereo-isomere Formen.

Ganz besonders bevorzugt sind die in der folgenden Tabelle aufgeführten Verbindungen:

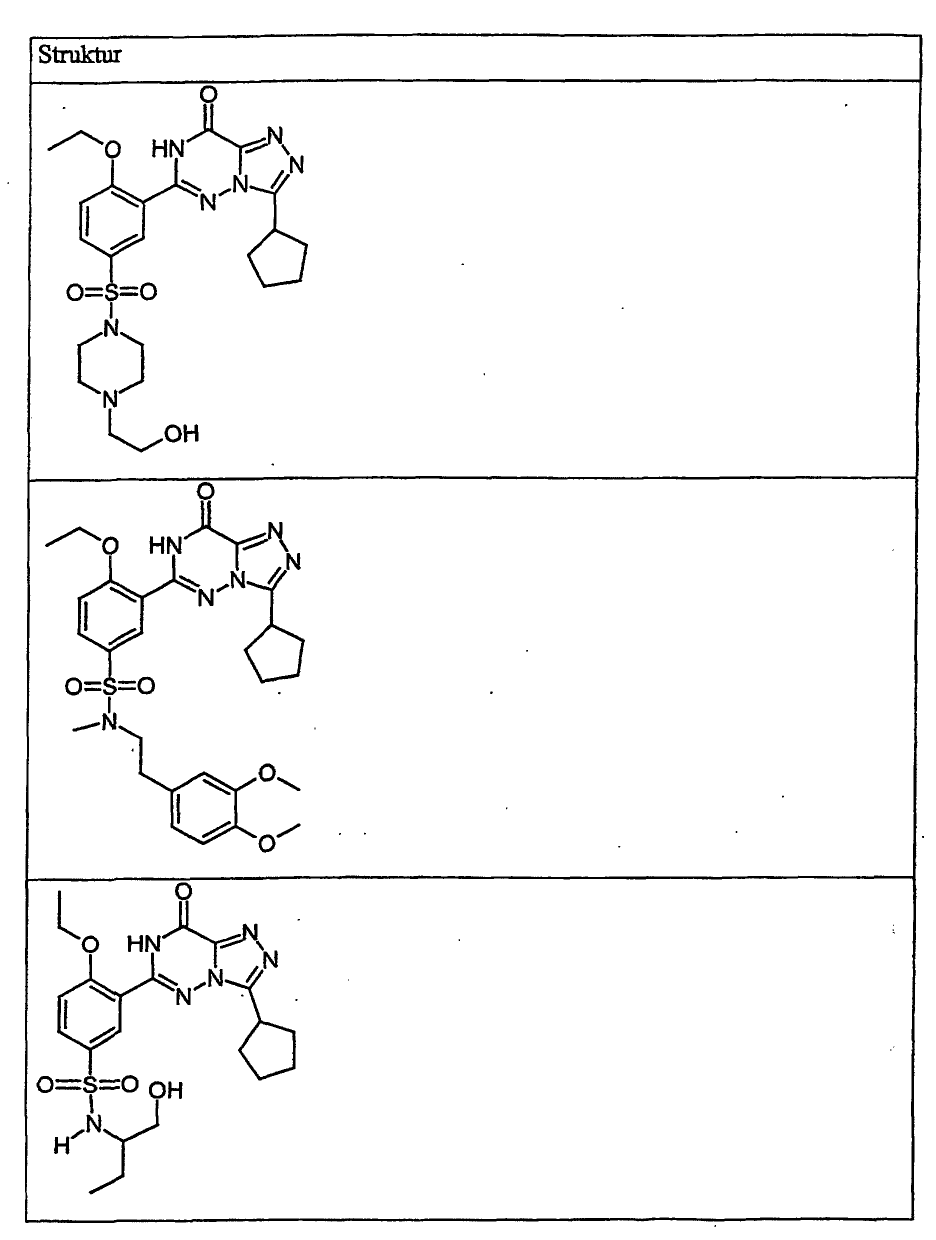

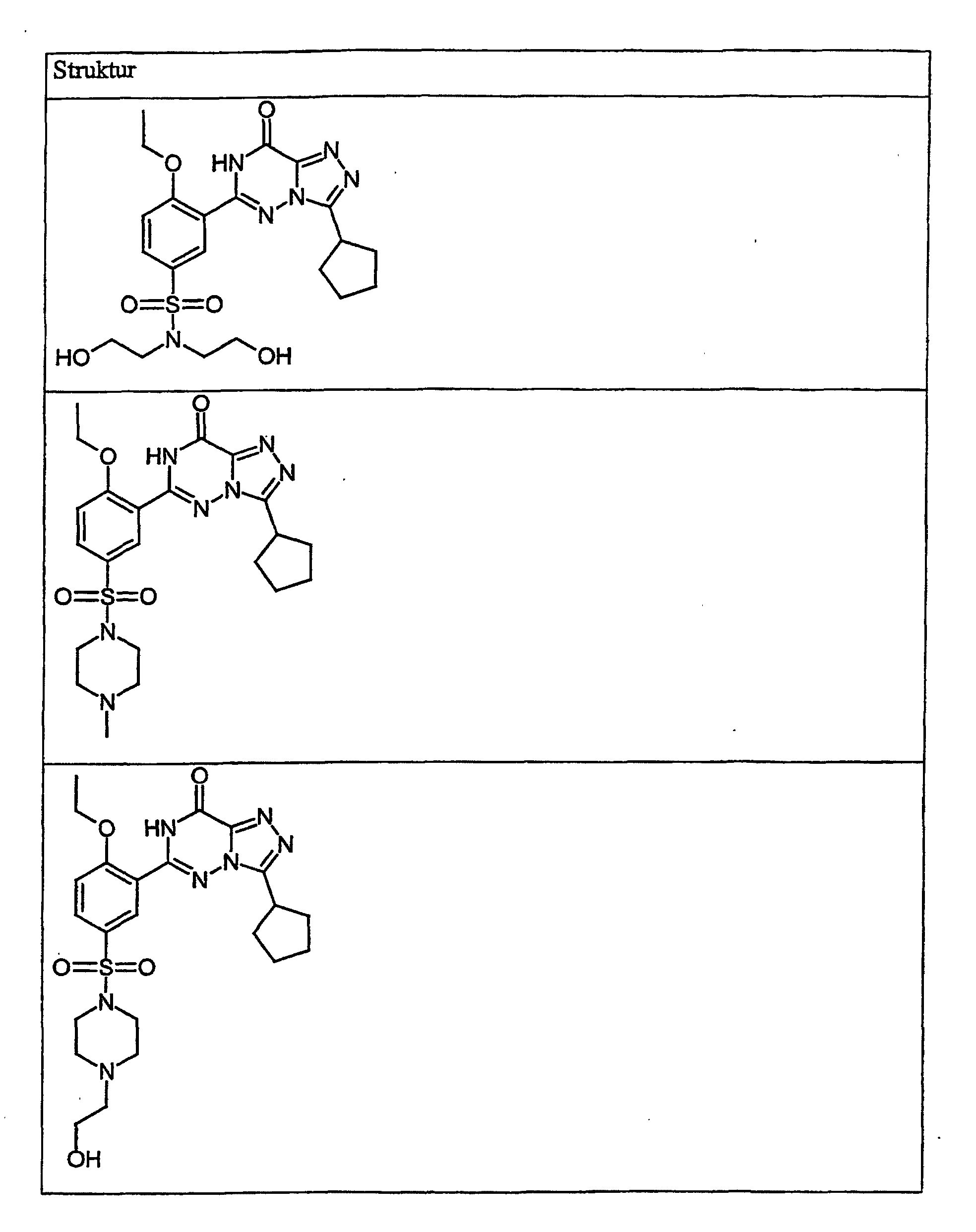

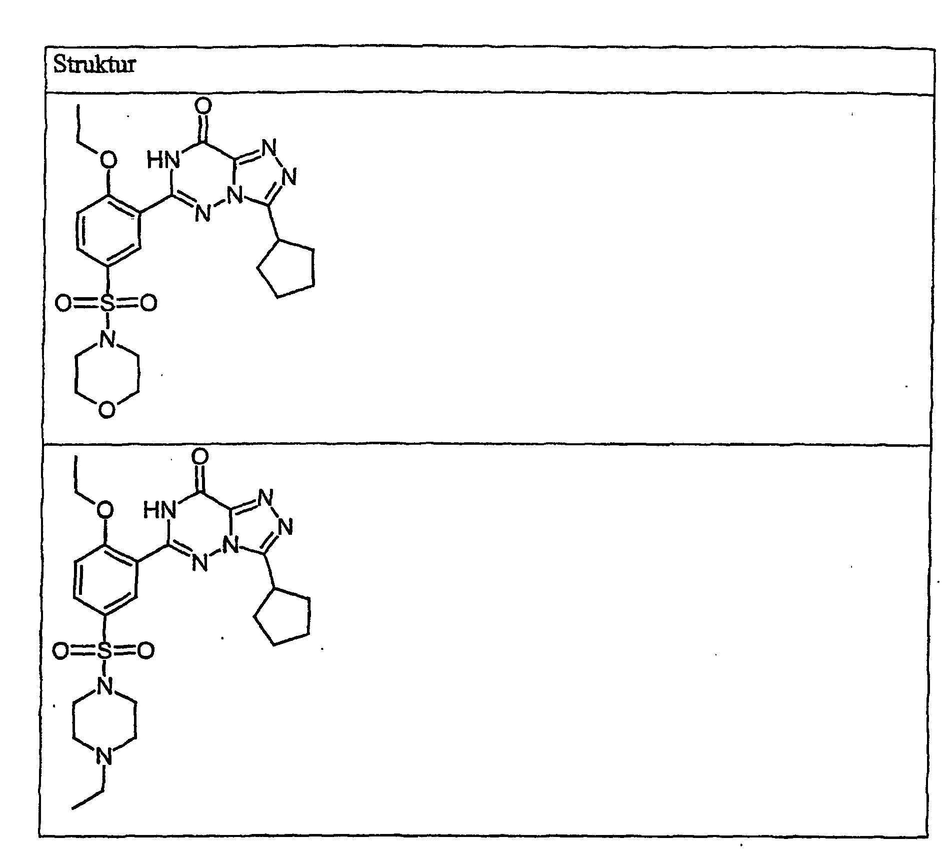

Außerdem wurde ein Verfahren zur Herstellung der erfindungsgemäßen Verbindungen der allgemeinen Formel (I) gefunden, dadurch gekennzeichnet, dass man Verbindungen der allgemeinen Formel (II) 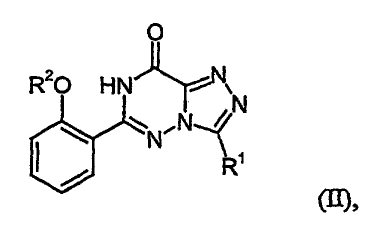 in welcher
R¹ und R² die oben angegebene Bedeutung haben,
mit Chlorsulfonsäure (ClSO₃H) gegebenenfalls in inerten Lösemitteln, gegebenenfalls in Anwesenheit einer Base zu den Verbindungen der allgemeinen Formel (III) 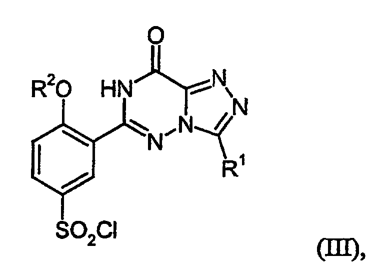 in welcher
R¹ und R² die oben angegebene Bedeutung haben,
umsetzt und abschließend mit Aminen der allgemeinen Formel (IV)

HNR³R⁴ (IV),

in welcher
- R³ und R⁴: die oben angegebene Bedeutung haben,
umsetzt.

Das erfindungsgemäße Verfahren kann durch folgendes Formelschema beispielhaft erläutert werden:

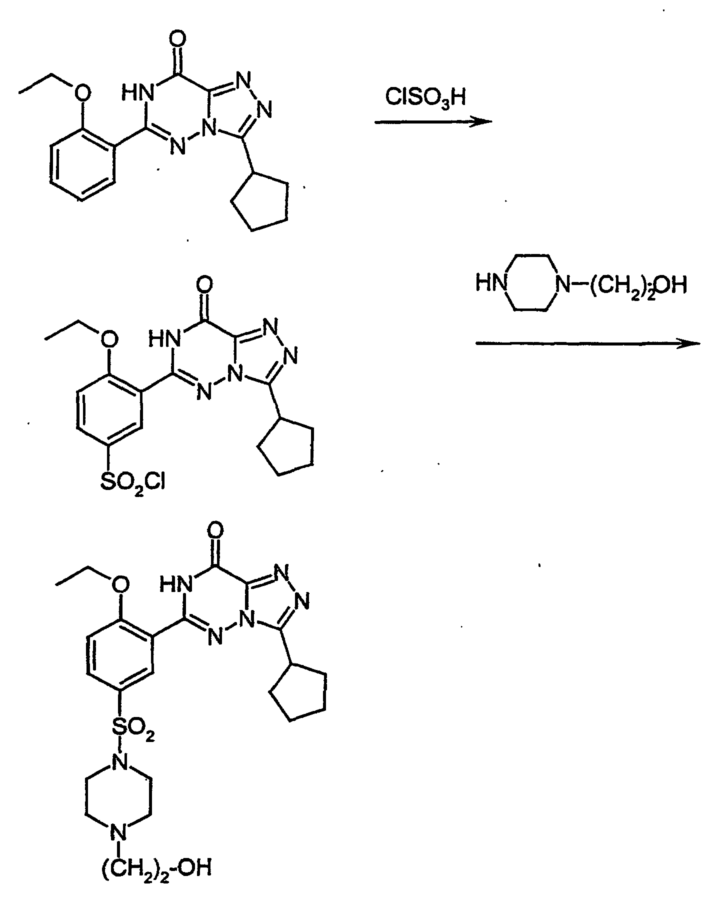

Als Lösemittel für die einzelnen Schritte eignen sich die üblichen organischen Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfrakionen, oder Halogenkohlenwasserstoffe wie Dichlormethan, Trichlormethan, Tetrachlormethan, Dichlorethan, Trichlorethylen oder Chlorbenzol, oder Essigester, Dimethylformamid, Hexamethylphosphorsäuretriamid, Acetonitril, Aceton, Dimethoxyethan oder Pyridin. Ebenso ist es möglich, Gemische der genannten Lösemittel zu verwenden.

Die Reaktionstemperaturen können im allgemeinen in einem größeren Bereich variieren. Im allgemeinen arbeitet man in einem Bereich von -20 °C bis 200 °C, bevorzugt von 0 °C bis 70 °C.

Die erfindungsgemäßen Verfahrensschritte werden im allgemeinen bei Normaldruck durchgeführt. Es ist aber auch möglich, bei Überdruck oder bei Unterdruck durchzuführen (z. B. in einem Bereich von 0,5 bis 5 bar).

Die Umsetzungen können beispielsweise in einem Temperaturbereich von 0°C bis Raumtemperatur und bei Normaldruck erfolgen.

Die Verbindungen der allgemeinen Formel (II) sind neu und können hergestellt werden, indem man durch Umsetzung der Verbindungen der allgemeinen Formel (V)

R¹-CO₂H (V),

in welcher
- R¹: die oben angegebene Bedeutung hat,
mit Thiocarbohydrazid 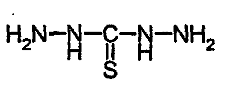 die Verbindungen der allgemeinen Formel (VI) 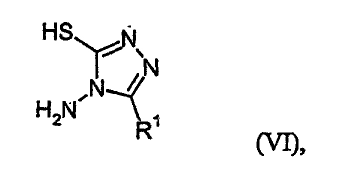 in welcher
- R¹: die oben angegebene Bedeutung hat,
herstellt, diese durch Umsetzung mit H₂O₂ / CH₃CO₂H in die Verbindungen der allgemeinen Formel (VII) 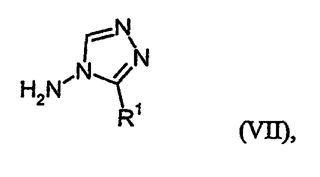 in welcher
- R¹: die oben angegebene Bedeutung hat,
überführt, in einem weiteren Schritt durch Umsetzung mit Verbindungen der allgemeinen Formel (VIII) 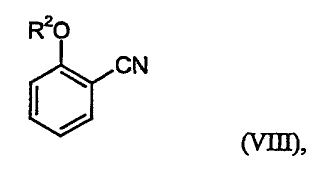 in welcher
- R²: die oben angegebene Bedeutung hat,
die Verbindungen der allgemeinen Formel (IX) 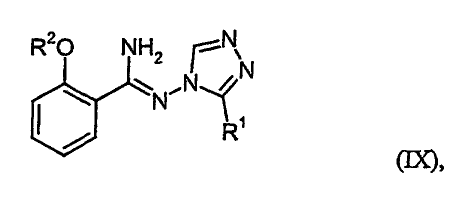 in welcher
- R¹ und R²: die oben angegebene Bedeutung haben,
herstellt, diese anschließend mit Diethylcarbonat in Verbindungen der allgemeinen Formel (X) 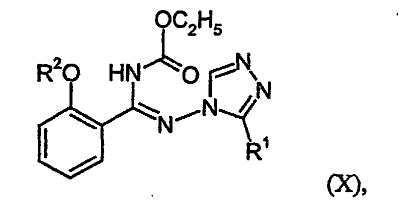 in welcher
- R¹ und R²: die oben angegebene Bedeutung haben,
überführt und abschließend durch Erhitzen zu den Verbindungen der allgemeinen Formel (II) cyclisiert.

Als Lösemittel für die einzelnen Schritte eignen sich die üblichen organischen Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfrakionen, oder Halogenkohlenwasserstoffe wie Dichlormethan, Trichlormethan, Tetrachlormethan, Dichlorethan, Trichlorethylen oder Chlorbenzol, oder Essigester, Dimethylformamid, Hexamethylphosphorsäuretriamid, Acetonitril, Aceton, Dimethoxyethan oder Pyridin. Ebenso ist es möglich, Gemische der genannten Lösemittel zu verwenden.

Die Reaktionstemperaturen können im allgemeinen in einem größeren Bereich variieren. Im allgemeinen arbeitet man in einem Bereich von -20 °C bis 200 °C, bevorzugt von 0 °C bis 70 °C.

Die erfindungsgemäßen Verfahrensschritte werden im allgemeinen bei Normaldruck durchgeführt. Es ist aber auch möglich, bei Überdruck oder bei Unterdruck durchzuführen (z. B. in einem Bereich von 0,5 bis 5 bar).

Die Umsetzungen können beispielsweise in einem Temperaturbereich von 0°C bis Raumtemperatur und bei Normaldruck erfolgen.

Die Verbindungen der allgemeinen Formeln (III), (IX) und (X) sind neu und können beispielsweise wie oben beschrieben hergestellt werden.

Die Verbindungen der allgemeinen Formeln (IV), (V), (VI), (VII) und (VIII) sind bekannt oder können nach üblichen Methoden hergestellt werden.

Die erfindungsgemäßen Verbindungen der allgemeinen Formel (I) zeigen ein nicht vorhersehbares, wertvolles pharmakologisches Wirkspektrum.

Sie inhibieren entweder eine oder mehrere der c-GMP metabolisierenden Phosphodiesterasen (PDE I, PDE II und PDE V). Dies führt zu einem Anstieg von c-GMP. Die differenzierte Expression der Phosphodiesterasen in verschiedenen Zellen, Geweben und Organen, ebenso wie die differenzierte subzelluläre Lokalisation dieser Enzyme, ermöglichen in Verbindung mit den erfindungsgemäßen selektiven Inhibitoren, eine selektive Adressierung der verschiedenen von cGMP regulierten Vorgänge.

Außerdem verstärken die erfindungsgemäßen Verbindungen die Wirkung von Substanzen, wie beispielsweise EDRF (Endothelium derived relaxing factor), ANP (atrial natriuretic peptide), von Nitrovasodilatoren und allen anderen Substanzen, die auf eine andere Art als Phosphodiesterase-Inhibitoren die cGMP-Konzentration erhöhen.

Daher sind die erfindungsgemäßen Verbindungen der allgemeine Formel (I) geeignet zur Prophylaxe und/oder Behandlung von Erkrankungen, bei denen ein Anstieg der cGMP-Konzentration heilsam ist, d.h. Erkrankungen, die im Zusammenhang mit cGMP-regulierten Vorgängen stehen (im Englischen meist einfach als 'cGMP-related diseases' bezeichnet). Hierzu zählen kardiovaskuläre Erkrankungen, Erkrankungen des Urogenitalsystems sowie cerebrovaskuläre Erkrankungen.

Unter dem Begriff "kardiovaskulären Erkrankungen" im Sinne der vorliegenden Erfindung fallen Erkrankungen wie beispielsweise Bluthochdruck, neuronale Hypertonie, stabile und instabile Angina, periphere und kardiale Gefäßerkrankungen, Arrhythmien, thromboembolische Erkrankungen und Ischämien wie Myokardinfarkt, Hirnschlag, transistorische und ischämische Attacken, Angina pectoris, periphere Durchblutungsstörungen, Verhinderung von Restenosen nach Thrombolysetherapie, percutaner transluminaler Angioplastie (PTA), percutan transluminaler Koronarangioplastien (PTCA) und Bypass.

Weiterhin können die erfindungsgemäßen Verbindungen der allgemeine Formel (I) auch Bedeutung für cerebrovaskuläre Erkrankungen haben. Hierzu zählen beispielsweise cerebrale Ischämie, Hirnschlag, Reperfusionsschäden, Hirntrauma, Ödeme, cerebrale Thrombose, Demenz und Alzheimer'sche Erkrankung

Die relaxierende Wirkung auf glatte Muskulatur macht sie geeignet für die Behandlung von Erkrankungen des Urogenitalsystems wie Prostatahypertrophie, Inkontinenz sowie insbesondere zur Behandlung der erektilen Dysfunktion und der weiblichen sexuellen Dysfunktion.

### Aktivität der Phosphordiesterasen (PDE's)

Die cGMP-stimulierbare PDE II, die cGMP-hemmbare PDE III und die cAMP-spezifische PDE IV wurden entweder aus Schweine- oder Rinderherzmyokard isoliert. Die Ca²⁺-Calmodulin stimulierbare PDEI wurde aus Schweineaorta, Schweinehirn oder bevorzugt aus Rinderaorta isoliert. Die c-GMP spezifische PDE V wurde aus Schweinedünndarm, Schweineaorta, humanen Blutplättchen und bevorzugt aus Rinderaorta gewonnen. Die Reinigung erfolgte durch Anionenanstauschchromatagraphie an MonoQ^{R} Pharmacia im wesentlichen nach der Methode von M. Hoey and Miles D. Houslay, Biochemical Pharmacology, Vol. 40, 193-202 (1990) und C. Lugman et al. Biochemical Pharmacology Vol. 35 1743-1751 (1986).

Die Bestimmung der Enzymaktivität erfolgt in einem Testansatz von 100 µl in 20 mM Tris/HCl-Puffer pH 7,5 der 5 mM MgCl₂, 0,1 mg/ml Rinderserumalbumin und entweder 800 Bq ³HcAMP oder ³HcGMP enthält. Die Endkonzentration der entsprechenden Nucleotide ist 10⁻⁶ mol/l. Die Reaktion wird durch Zugabe des Enzyms gestartet, die Enzymmenge ist so bemessen, dass während der Inkubationszeit von 30 min ca. 50% des Substrates umgesetzt werden. Um die cGMP stimulierbare PDE II zu testen, wird als Substrat ³HcAMP verwendet und dem Ansatz 10⁻⁶ mol/l nicht markiertes cGMP zugesetzt. Um die Ca²⁺-Calmodulinabhängige PDE I zu testen, werden dem Reaktionsansatz noch 1 µM CaCl₂ und 0,1 µM Calmodulin zugesetzt. Die Reaktion wird durch Zugabe von 100 µl Acetonitril, das 1 mM cAMP und 1 mM AMP enthält gestoppt. 100 µl des Reaktionsansatzes werden mittels HPLC getrennt und die Spaltprodukte "Online" mit einem DurchfluBscintillationszähler quantitativ bestimmt. Es wird die Substanzkonzentration gemessen, bei der die Reaktionsgeschwindigkeit um 50 % vermindert ist. Zusätzlich wurde zur Testung der "Phosphodiesterase [³H] cAMP-SPA enzyme assay" und der "Phosphodiesterase [³H] cGMP-SPA enzyme assay" der Firma Amersham Life Science verwendet. Der Test wurde nach dem vom Hersteller angegebenen Versuchsprotokoll durchgeführt. Für die Aktivitätsbestimmung der PDE II wurde der [³H] cAMP SPA assay verwendet, wobei dem Reaktionsansatz 10⁻⁶ M cGMP zur Aktivierung des Enzyms zugegeben wurde. Für die Messung der PDE I wurden 10⁻⁷ M Calmodulin und 1 µM CaCl₂ zum Reaktionsansatz zugegeben. Die PDE V wurde mit dem [³H] cGMP SPA assay gemessen.

Grundsätzlich führt die Inhibition einer oder mehrerer Phosphodiesterasen dieses Typs zu einer Erhöhung der cGMP-Konzentration. Dadurch sind die Verbindungen interessant für alle Therapien, in denen eine Erhöhung der cGMP-Konzentration als heilsam angenommen werden kann.

Die Untersuchung der kardiovaskulären Wirkungen wurden an normotonen und an SH-Ratten und an Hunden durchgeführt. Die Substanzen wurden intravenös oder oral appliziert.

Die Untersuchung auf erektionsauslösende Wirkung wurde am wachen Kaninchen durchgeführt [H. Naganuma, T. Egashira, J. Fuji, Clinical and Experimental Pharmacology and Physiology 20, 177-183 (1993)]. Die Substanzen wurden oral oder. parenteral appliziert.

Die neuen Wirkstoffe sowie ihre physiologisch unbedenklichen Salze (z. B. Hydrochloride, Maleinate oder Lactate) können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d. h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z. B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral, transdermal oder parenteral, z. B. perlingual , buccal, intravenös, nasal, rektal oder inhalativ.

Für die Anwendung beim Menschen werden bei oraler Administration im allgemeinen Dosierungen von 0,001 bis 50 mg/kg vorzugsweise 0,01 mg/kg - 20 mg/kg verabreicht. Bei parenteraler Administration, wie z. B. über Schleimhäute nasal, buccal, inhalativ, ist eine Dosierung von 0,001 mg/kg - 0,5 mg/kg sinnvoll.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der oben genannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die erfindungsgemäßen Verbindungen sind auch zur Anwendung in der Tiermedizin geeignet. Für Anwendungen in der Tiermedizin können die Verbindungen oder ihre nicht toxischen Salze in einer geeigneten Formulierung in Übereinstimmung mit den allgemeinen tiermedizinischen Praxen verabreicht werden. Der Tierarzt kann die Art der Anwendung und die Dosierung nach Art des zu behandelnden Tieres festlegen.

In den folgenden Herstellungsbeispielen der Vorstufen und Endprodukte ist in Strukturformeln mit einer oder mehreren ungesättigten Valenzen am Stickstoff- oder Sauerstoffatom stets ein Wasserstoff zu ergänzen.

D. h. Strukturen z. B. mit einem Strukturelement "-N-" meint eigentlich "-NH-" und Strukturen z. B. mit einem Strukturelement mit "-O" meint eigentlich "-OH".

### Herstellung der Vorstufen

### Beispiel I

### 4-Amino-5-cyclopentyl-4H-1,2,4-triazol-3-thiol

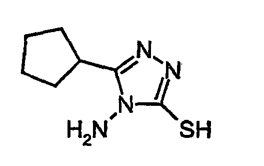

34.29 g (323 mmol) feingemörsertes Thiocarbohydrazid werden in 38.5 ml (355.3 mmol) Cyclopentancarbonsäure suspendiert und 20 min auf 165°C erhitzt. Bis zum Auftreten eines gelblichen Kondensates wird dabei entstehendes Reaktionswasser abdestilliert. Nach Erkalten wird die Suspension mit 250 ml Dichlormethan/Methanol 95:5 versetzt und es wird vom Niederschlag abfiltriert. Das Filtrat wird konzentriert und an Silicagel säulenfiltriert (Dichlormethan/Methanol 98:2). Nach Trocknen im Hochvakuum wird das Produkt als farbloser Feststoff erhalten.
Ausbeute: 34.37 g, 75 % Reinheit (43.3 % der Theorie)
MS (ESI-pos.): m/z (%) = 185 (M+H) (53), 184 (M⁺) (100), 143 (85)
¹H-NMR (200 MHz, CDCl₃): δ = 1.58-2.17 (m, 8 H); 2.70-2.82 (m, 1 H); 3.20-3.35 (m, 1 H); 4.51 (s, 2 H).

### Beispiel II

### 3-Cyclopentyl-4H-1,2,4-triazol-4-amin

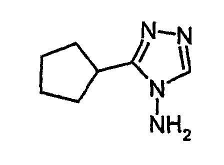

34.4 g (75 % Reinheit, 140 mmol) der Verbindung aus Beispiel I werden in 250 ml Essigsäure vorgelegt und unter Rückfluß mit 66 ml 30 %iger WasserstoffperoxidLösung portionsweise versetzt. Nach Ende der Zugabe wird 30 min bei Rückfluß gerührt, nach Abkühlen wird konzentriert und mit 3 N Natriumhydroxidlösung basisch gestellt. Die wässrige Phase wird sechsmal mit Dichlormethan extrahiert. Nach Vereinigung der organischen Phasen wird mit wenig gesättigter Natriumchloridlösung gewaschen, über Magnesiumsulfat getrocknet und konzentriert. Der anfallende leicht gelbliche Feststoff wird aus Dichlormethan/Ether kristallisiert.
Ausbeute: 3.99 g (15.4 % der Theorie)
MS (DCI, NH₃): m/z (%) = 153 (M+H) (100)
¹H-NMR (400 MHz, CDCl₃): δ = 1.65-1.98 (m, 7 H); 2.03-2.12 (m, 2 H); 3.27 (qui, 1 H); 4.86 (s, 2 H); 8.10 (s, 1 H).

### Beispiel III

N-(3-Cyclopentyl-4H-1,2,4-triazol-4-yl)-2-ethoxybenzolearboximid-amid

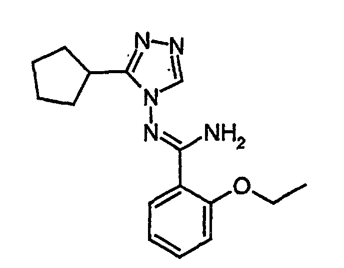

Zu einer Suspension von 0.34 g (60 %, 8.42 mmol) NaH in 24 ml trockenem 1,4-Dioxan (ausgeheizter Kolben, unter Argon) wird die Verbindung aus Beispiel II als Feststoff (1.22 g, 8.02 mmol) gegeben. Die Suspension wird 30 min bei 90°C gerührt, bevor 1.30 g (8.82 mmol) 2-Ethoxybenzonitril hinzugefügt werden. Die resultierende Suspension wird über Nacht bei 90°C gerührt. Nach Zugabe von Wasser wird mit Dichlormethan extrahiert (viermal). Die vereinigten organischen Phasen werden mit wenig gesättigter Natriumchloridlösung gewaschen und über Magnesiumsulfat getrocknet. Nach Einengen auf circa 20 ml wird Cyclohexan zugesetzt und der ausgefallene Feststoff durch Abfiltrieren isoliert.
Ausbeute: 1.58 g (65.8 % der Theorie)
MS (DCI, NH₃): m/z (%) = 300 (M+H) (100)
¹H-NMR (200 MHz, CDCl₃): δ = 1.51 (t, 3 H); 1.56-2.12 (m, 8 H); 3.14 (qui, 1 H); 4.22 (q, 2 H); 6.49 (bs, 2 H); 7.02-7.15 (m, 2 H); 7.49 (dt, 1 H); 8.04 (s, 1 H); 8.18 (dd, 1 H).

### Beispiel IV

### Ethyl-[(3-cyclopentyl-4H-1,2,4-triazol-4-yl)-imido]-(2-ethoxyphenyl)-methylcarbamat

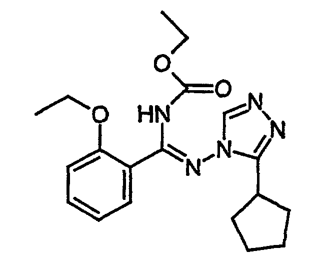

Zu einer Suspension von 0.23 g (60 %, 5.8 mmol) Natriumhydrid in 26 ml trockenem 1,4-Dioxan (ausgeheizter Kolben, Argon) werden 1.58 g (5.28 mmol) der Verbindung aus Beispiel III als Feststoff gegeben und 1.02 ml (8.4 mmol) Diethylcarbonat zugetropft. Die Suspension wird bei 90°C über Nacht gerührt. Nach Abkühlen werden weitere 120 mg Natriumhydrid und 1.02 ml Diethylcarbonat zugefügt und die Mischung wird zusätzliche 4 h bei 90°C gerührt, bevor nach Abkühlen mit 1 N Salzsäurelösung neutralisiert und anschließend im Vakuum konzentriert wird. Der Rückstand wird mit wenig Wasser behandelt und mit Dichlormethan extrahiert (viermal). Die vereinigten organischen Phasen werden mit wenig gesättigter Natriumchloridlösung gewaschen, über Magnesiumsulfat getrocknet, eingeengt und am Hochvakuum getrocknet.
Ausbeute: 2.14 g, 90 % Reinheit (98.2 % der Theorie)
MS (DCI, NH₃): m/z (%) = 372 (M+H) (100)

### Beispiel V

### 3-Cyclopentyl-6-(2-ethoxyphenyl)[1,2,4]triazolo[3,4-f][1,2,4]triazin-8(7H)-on

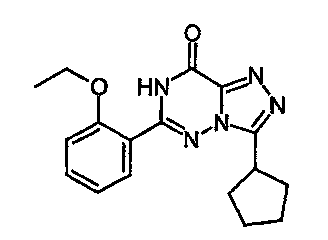

Eine Lösung aus 2.14 g (90 %, 5.19 mmol) der Verbindung aus Beispiel IV in 20 ml 2-Ethoxyethanol wird über Nacht unter Rückfluß erhitzt. Nach Abkühlen wird die Mischung am Hochvakuum einrotiert und an der Ölpumpe getrocknet. Der feste Rückstand wird mit heißem Ether behandelt, der ausgefallene Feststoff wird abfiltriert und am Hochvakuum getrocknet.
Ausbeute: 1.367 g (81 % der Theorie)
MS (DCI, NH₃): m/z (%) = 326 (M+H) (100)
¹H-NMR (200 MHz, CDCl₃): δ = 1.62 (t, 3 H); 1.72-2.30 (m, 8 H); 3.68 (qui, 1 H); 4.34 (q, 2 H); 7.08-7.21 (m, 2 H); 7.04 (dt, 1 H); 8.25 (dd, 1 H); 10.85 (bs, 1 H).

### Beispiel VI

### 3-(3-Cyclopentyl-8-oxo-7,8-dihydro[1,2,4]triazolo[3,4-f][1,2,4]triazin-6-yl)-4-ethoxybenzolsulfonylchlorid

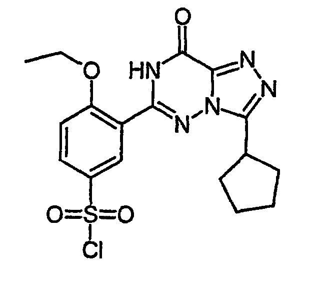

In 1.68 ml (25.2 mmol) eisgekühlte Chlorsulfonsäure werden 683 mg (2.1 mmol) der Verbindung aus Beispiel V portionsweise eingetragen. Die Mischung wird nach Erwärmen auf Raumtemperatur über Nacht nachgerührt. Nach Abkühlen auf 0°C wird mit Dichlormethan verdünnt und auf Eiswasser gegossen. Die organische Phase wird abgetrennt. Man extrahiert die wässrige Phase nochmals mit Dichlormethan, vereinigt die organischen Phasen, wäscht mit wenig gesättigter Natriumchloridlösung, trocknet über Magnesiumsulfat und dampft ein.
Ausbeute: 801 mg (90 % der Theorie)
MS (DCI, NH₃): m/z (%) = 424 (M+H) (100)
¹H-NMR (200 MHz, CDCl₃): δ = 1.65 (t, 3 H); 1.72-2.32 (m, 8 H); 3.71 (qui, 1 H); 4.47 (q, 2 H); 7.30 (d, 1 H); 8.22 (dd, 1 H); 8.77 (d, 1 H); 10.76 (bs, 1 H).

### Herstellung der Wirkstoffe

### Beispiel 1

### 3-Cyclopentyl-6-(2-ethoxy-5-{[4-(2-hydroxyethyl)piperazino]sulfonyl}phenyl)-[1,2,4]triazolo[3,4-f][1,2,4]triazin-8-(7H)-on

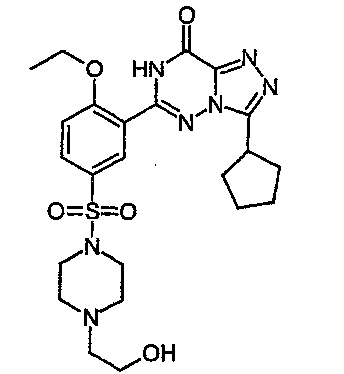

Zu einer Suspension aus 395 mg (0.92 mmol) des Sulfonsäurechlorids aus Beispiel VI in 3 ml Dichlormethan werden 394 mg (2.8 mmol) N-Hydroxyethylpiperazin und eine kleine Spatelspitze 4-*N*-Dimethylaminopyridin (DMAP) gegeben, die resultierende klare Lösung wird bei Raumtemperatur gerührt, bevor nach 7 Stunden mit Dichlormethan verdünnt wird, mit wenig Wasser sowie gesättigter Natriumchlorid gewaschen, über Magnesiumchlorid getrocknet und im Vakuum konzentriert wird. Der Rückstand wird aus wenig Dichlormethan/Ether kristallisiert.
Ausbeute: 368 mg (72.5 % der Theorie)
MS (DCI, NH₃): m/z (%) = 518 (M+H) (100)
¹H-NMR (200 MHz, CDCl₃): δ = 1.62 (t, 3 H); 1.68-2.30 (m, 8 H); 2.52-2.70 (m, 6 H); 3.05-3.17 (m, 4 H); 3.52-3.71 (m, 3 H); 4.49 (q, 2 H); 7.22 (d, 1 H); 7.91 (dd, 1 H); 8.43 (bs, 1 H); 10.64 (bs, 1 H).

### Beispiel 2

### 3-(3-Cyclopentyl-8-oxo-7,8-dihydro[1,2,4]triazolo[3,4-f][1,2,4]triazin-6-yl)-N-(3,4-dimethoxyphenethyl)-4-ethoxy-N-methylbenzolsulfonamid

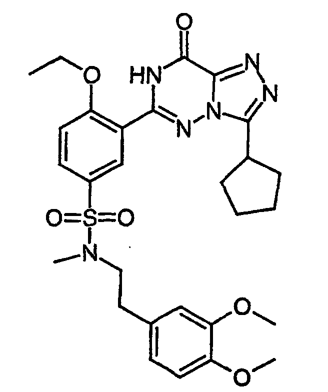

Zu einer Suspension aus 395 mg (0.93 mmol) des Sulfonsäurechlorids aus Beispiel VI in 3 ml Dichlormethan werden 546 mg (2.8 mmol) *N*-Methylhomoveratrylamin und eine kleine Spatelspitze 4-DMAP gegeben. Die resultierende klare Lösung wird bei Raumtemperatur gerührt, bevor nach 7 h mit Dichlormethan verdünnt wird, mit 1 N Salzsäurelösung (zweimal) sowie gesättigter Natriumchlorid gewaschen, über Magnesiumchlorid getrocknet und im Vakuum konzentriert wird. Der Rückstand wird aus wenig Dichlormethan/Ether kristallisiert.
Ausbeute: 299 mg (55.1 % der Theorie)
MS (DCI, NH₃): m/z (%) = 583 (M+H) (100)
¹H-NMR (200 MHz, CDCl₃): δ = 1.63 (t, 3 H); 1.68-2.23 (m, 8 H); 2.78-2.90 (m, 2 H); 2.82 (s, 3 H); 3.32 (t, 2 H); 3.63 (qui, 1 H); 3.84 (s, 6 H); 4.39 (q, 2 H); 6.68-6.80 (in, 3 H); 7.17 (d, 1 H); 7.89 (dd, 1 H); 8.49 (d, 1 H); 10.66 (bs, 1 H).

Die in den folgenden Tabellen aufgeführten Sulfonamide wurden mittels automatisierter Parallelsynthese aus dem entsprechenden Sulfonsäurechlorid (Beispiel VI) und den entsprechenden Aminen nach einer der drei folgenden Standardvorschriften hergestellt.

Die Reinheit der Endprodukte wurde mittels HPLC bestimmt, ihre Charakterisierungen durch LC-MS Messung vorgenommen. Der in der Spalte % (HPLC) angegebene Zahlenwert gibt den Gehalt des durch den Molpeak charakterisierten Endprodukts an. Standardvorschrift A wurde angewendet bei Aminen mit aciden Funktionalitäten, Standardvorschrift B bei Aminen mit neutralen Funktionalitäten, Standardvorschrift C bei Aminen mit zusätzlichen basischen Funktionalitäten.

Bei Verbindungen, die in den folgenden Tabellen aufgeführt sind und die optisch eine freie Stickstoffvalenz aufzeigen, sind diese grundsätzlich als -NH-Rest zu verstehen.

### Standardvorschrift A: Umsetzung von Aminen mit aciden Funktionalitäten

Zunächst werden 0,05 mmol Amin, 0,042 mmol Sulfonsäurechlorid und 0,10 mmol Na₂CO₃ vorgelegt und 0,5 ml eines Gemisches aus THF/H₂O von Hand zupipettiert. Nach 24 h bei RT wird mit 0,5 ml 1 M H₂SO₄-Lösung versetzt und über eine zweiphasige Kartusche filtriert (500 mg Extrelut (Oberphase) und 500 mg SiO₂, Laufmittel Essigester). Nach dem Einengen des Filtrates im Vakuum erhält man das Produkt.

### Standardvorschrift B: Umsetzung von Aminen mit neutralen Funktionalitäten

Zunächst werden 0,125 mmol Amin vorgelegt und vom Synthesizer 0,03 mmol Sulfonsäurechlorid als Lösung in 1,2-Dichlorethan zupipettiert. Nach 24 h wird das Gemisch mit 0,5 ml 1 M H₂SO₄ versetzt und über eine zweiphasige Kartusche (500 mg Extrelut (Oberphase) und 500 mg SiO₂, Laufmittel: Essigester) filtriert. Das Filtrat wird im Vakuum eingeengt.

### Standardvorschrift C: Umsetzung von Aminen mit basischen Funktionalitäten

Zunächst werden 0,05 mmol Amin vorgelegt und vom Synthesizer 0,038 mmol Sulfonsäurechlorid als Lösung in 1,2-Dichlorethan und 0,05 mmol Triethylamin als Lösung in 1,2-Dichlorethan zupipettiert. Nach 24 h wird zunächst mit 3 ml gesättigter NaHCO₃-Lösung versetzt und das Reaktionsgemisch über eine zweiphasige Kartusche nitriert. Nach dem Einengen des Filtrates im Vakuum erhält man das Produkt.

Alle Reaktionen werden dünnschichtchromatographisch verfolgt. Für den Fall das nach 24 Stunden bei RT keine vollständige Umsetzung erfolgt ist, wird für weitere 12 Stunden auf 60°C erhitzt und im Anschluß der Versuch beendet.

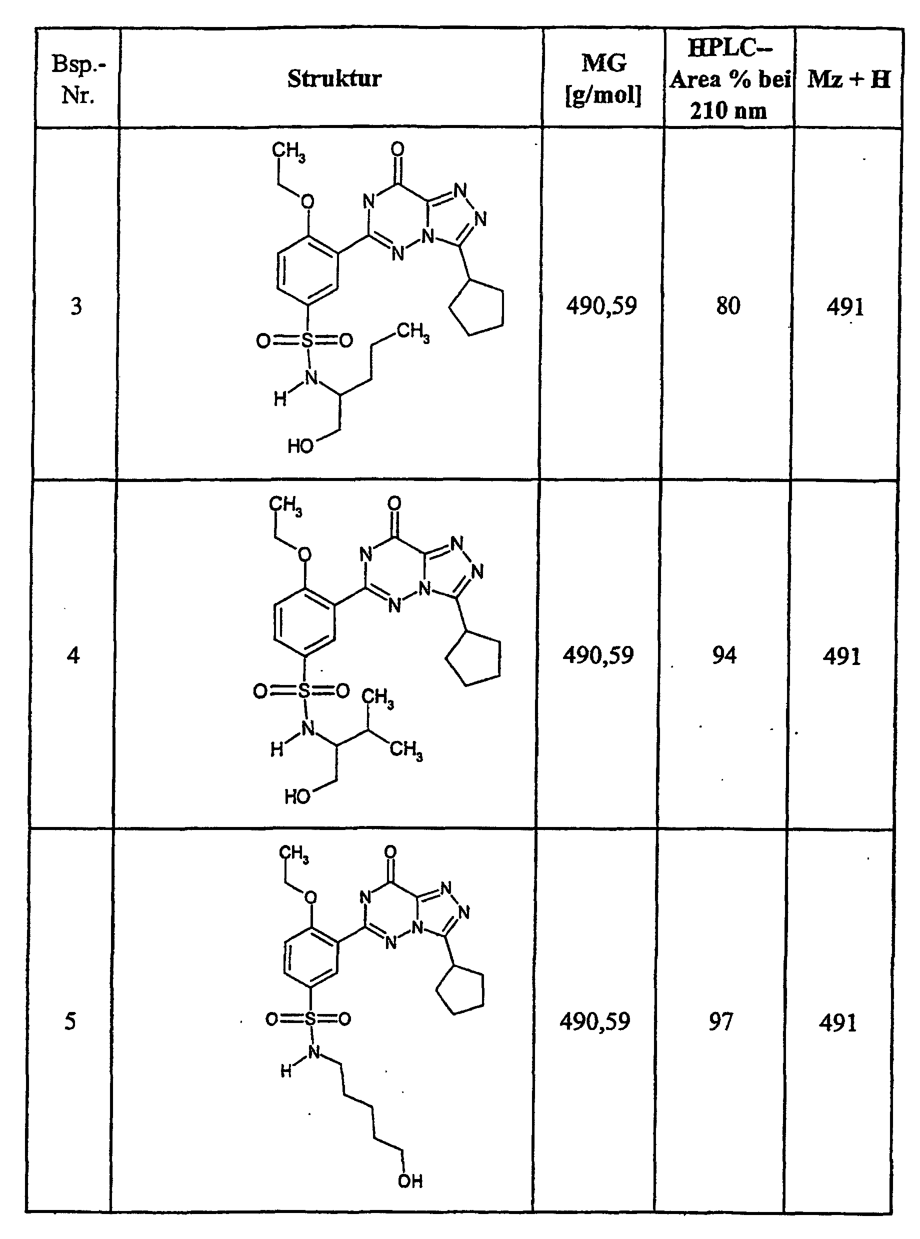

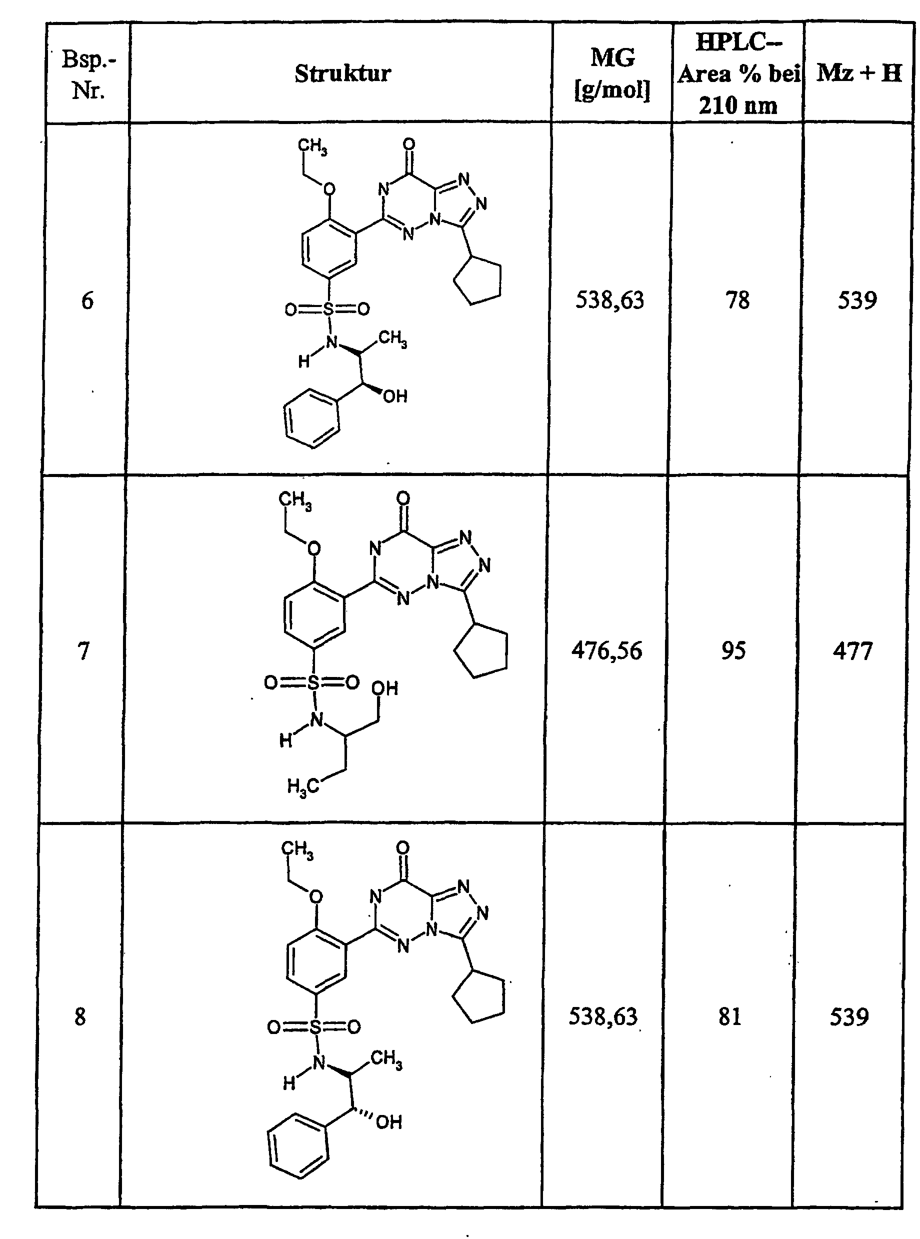

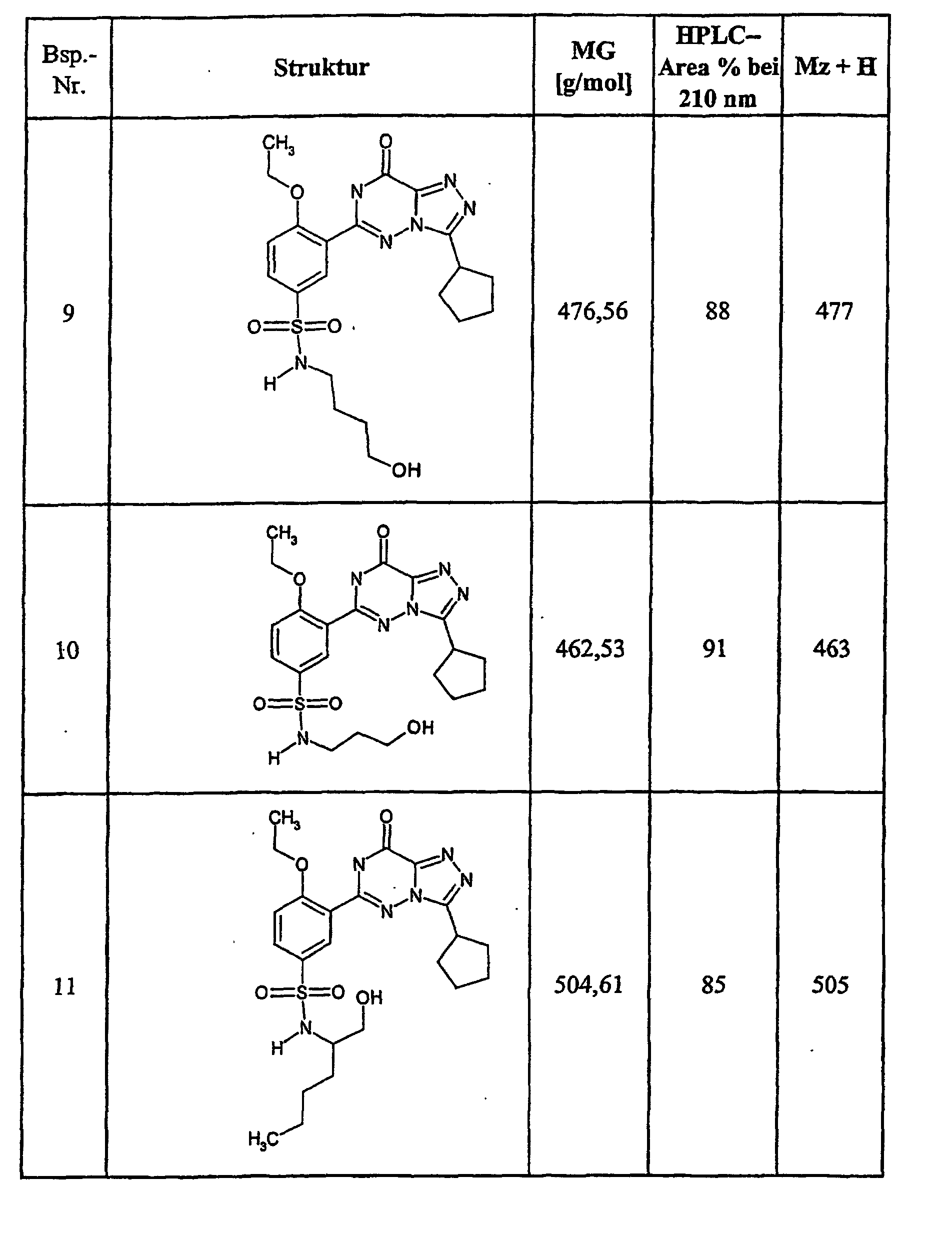

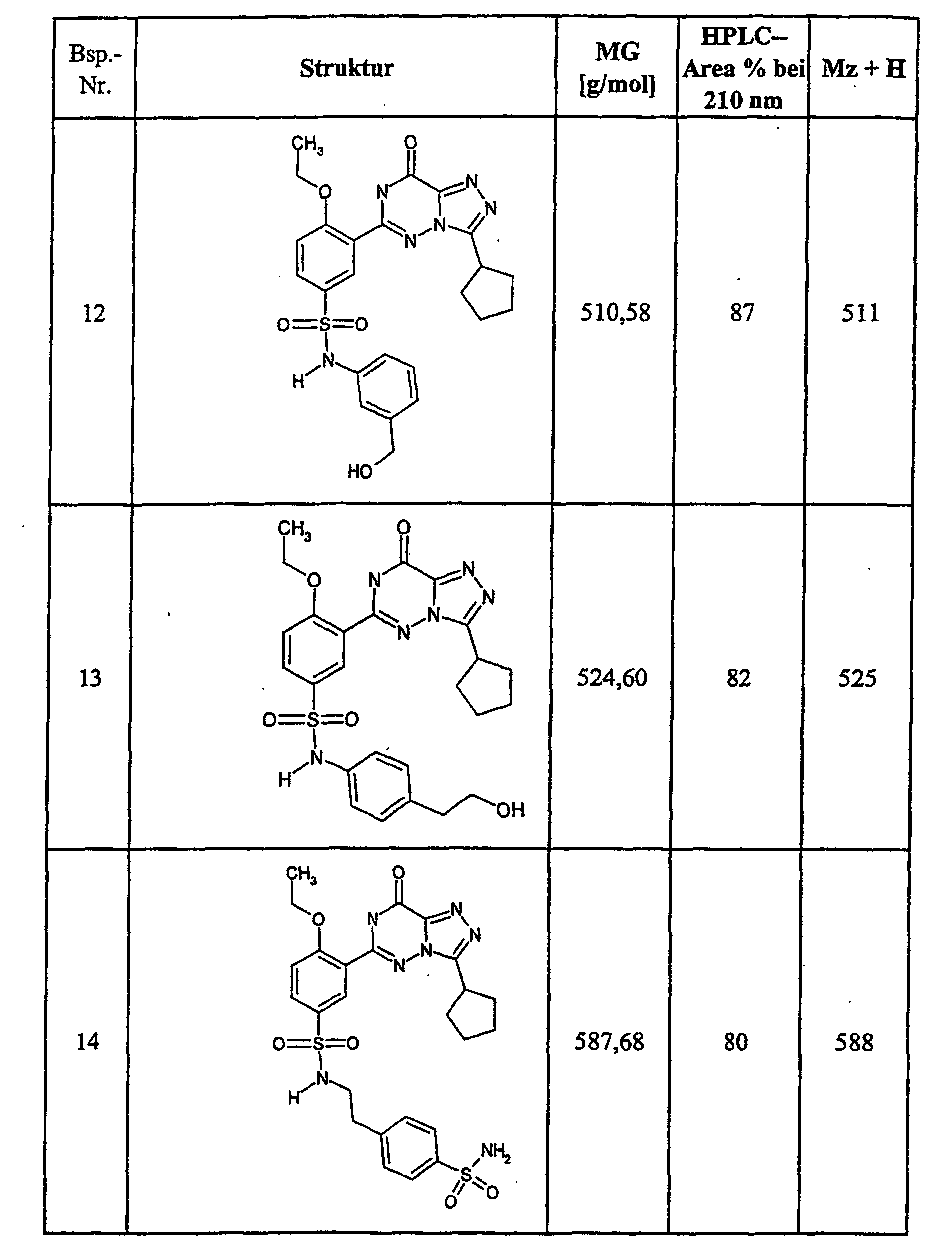

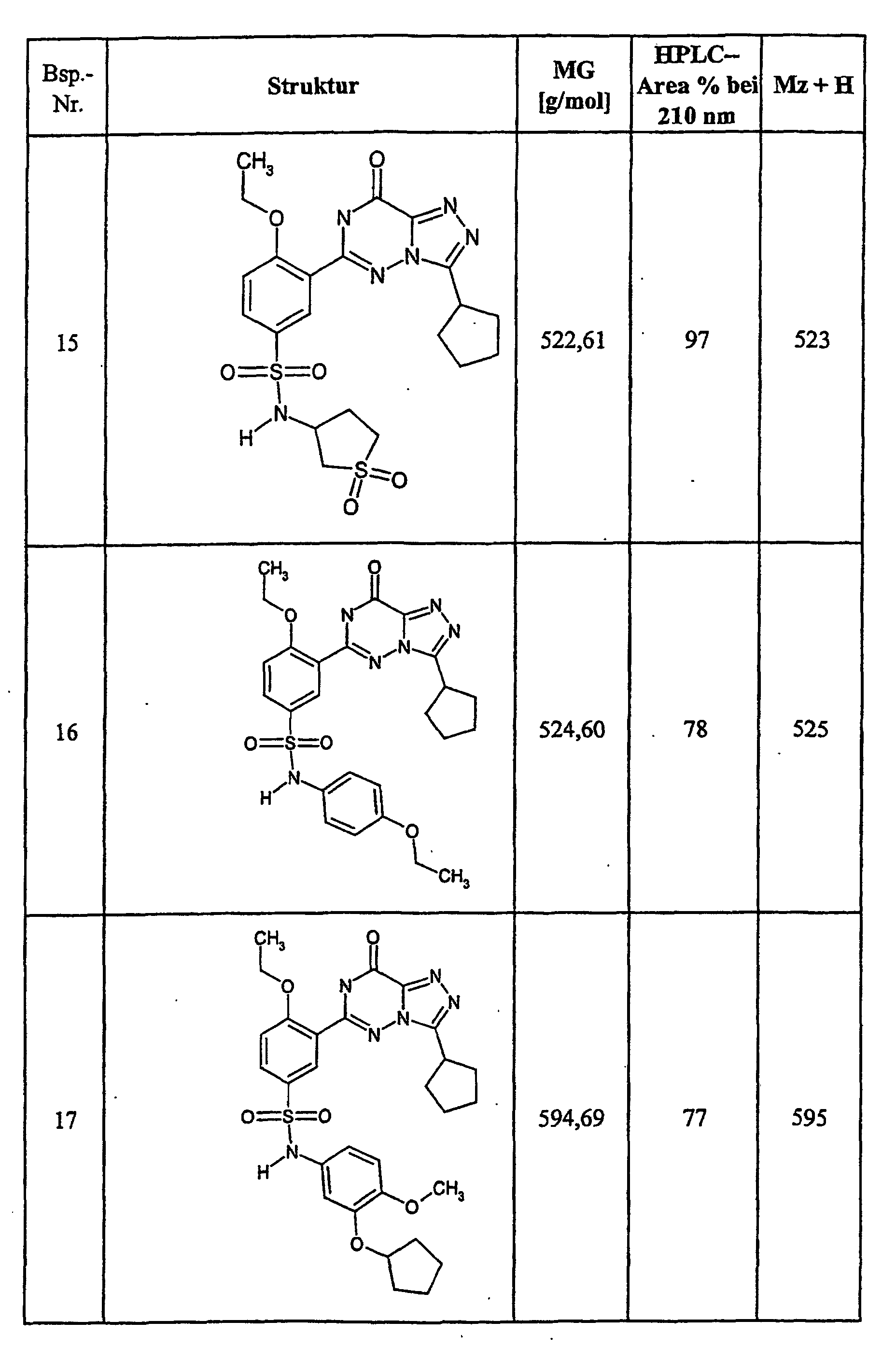

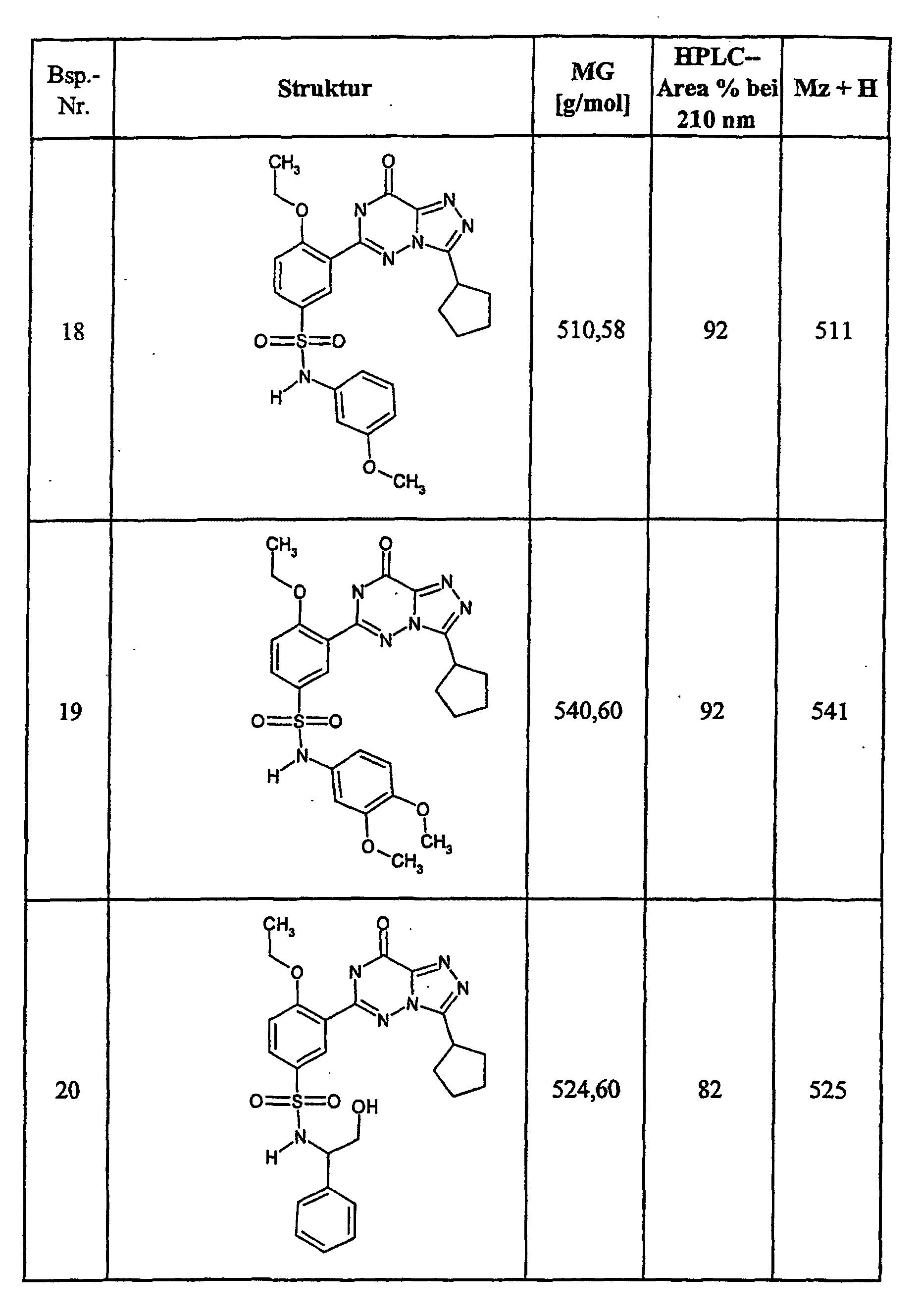

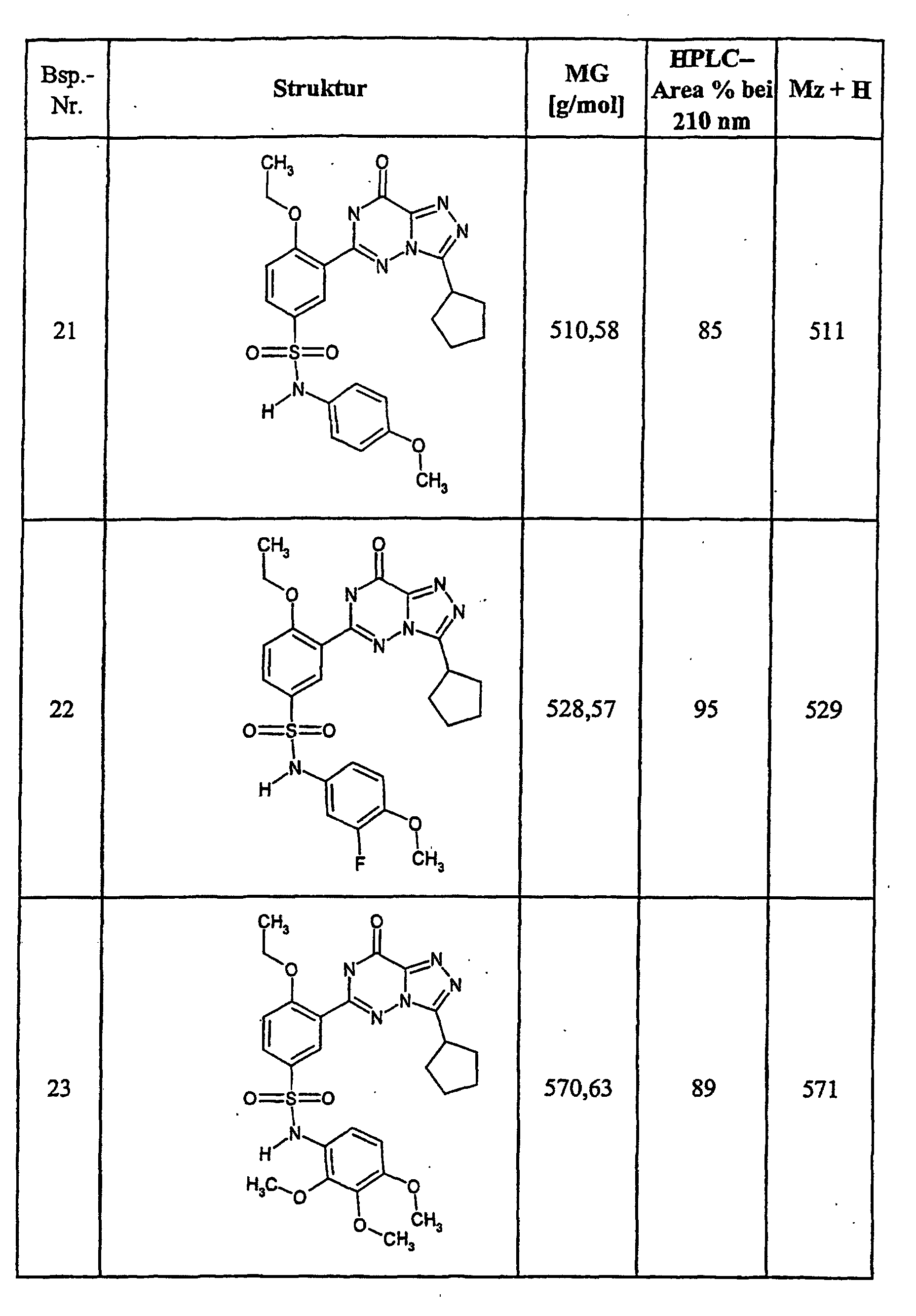

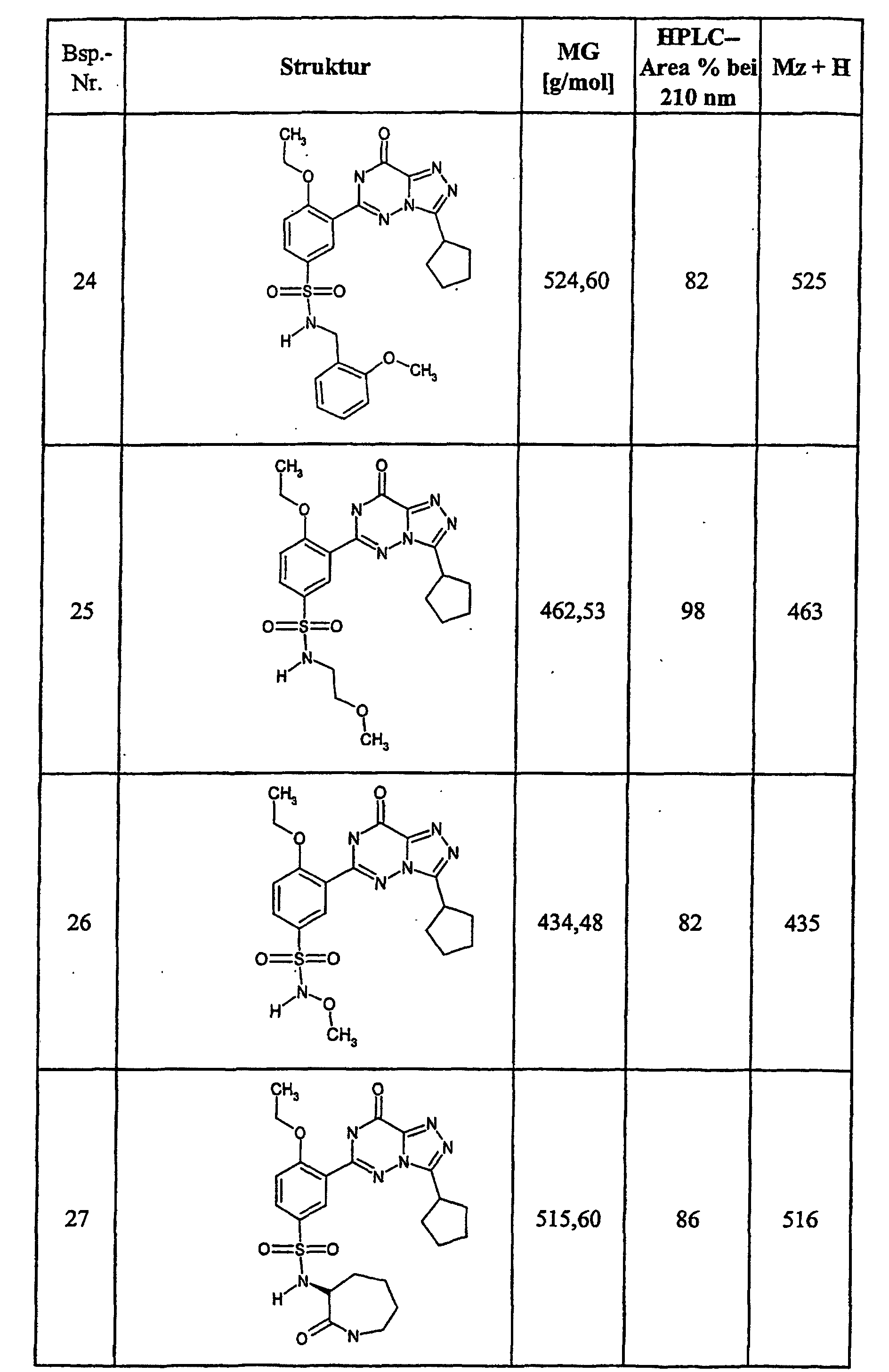

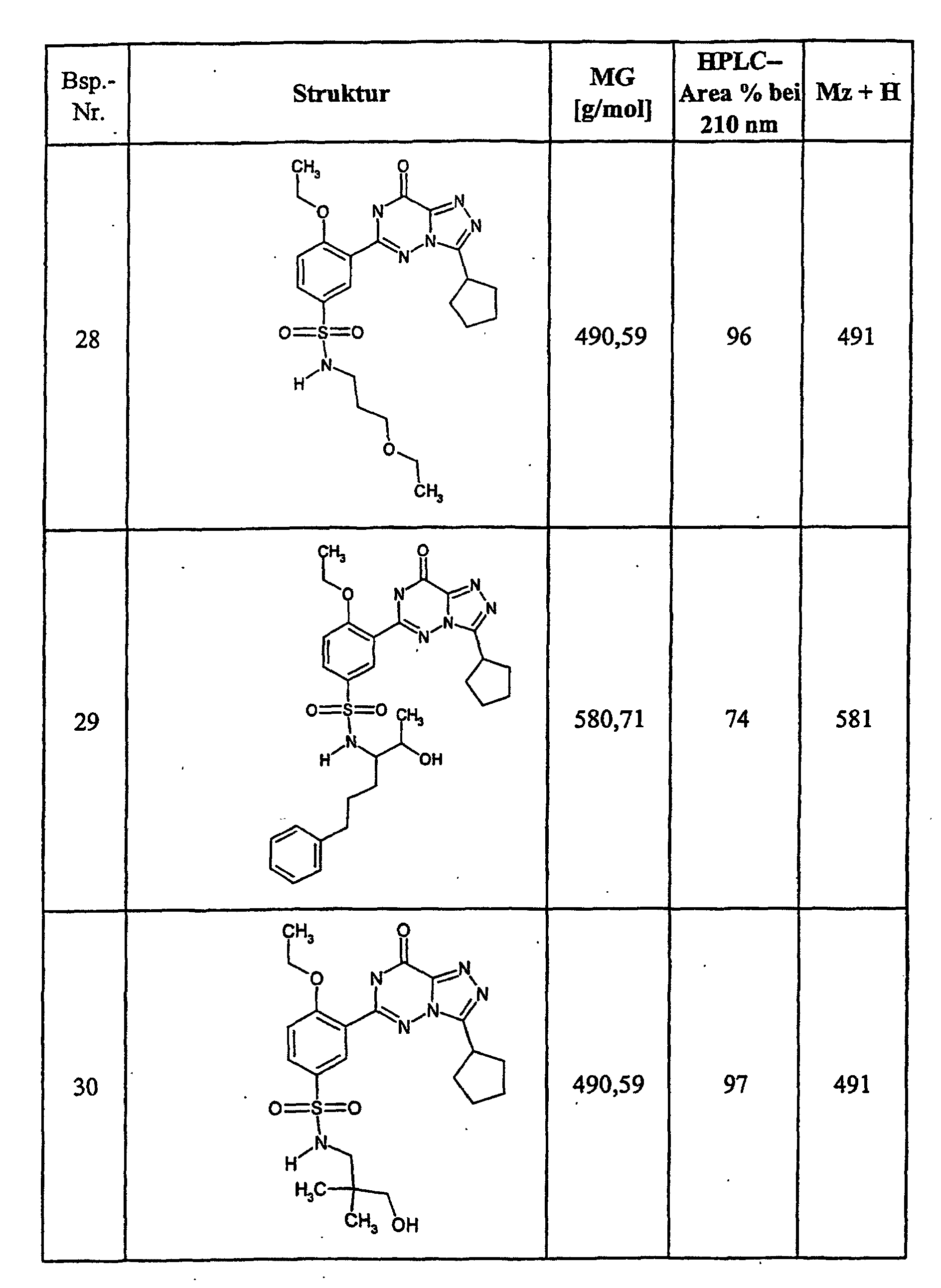

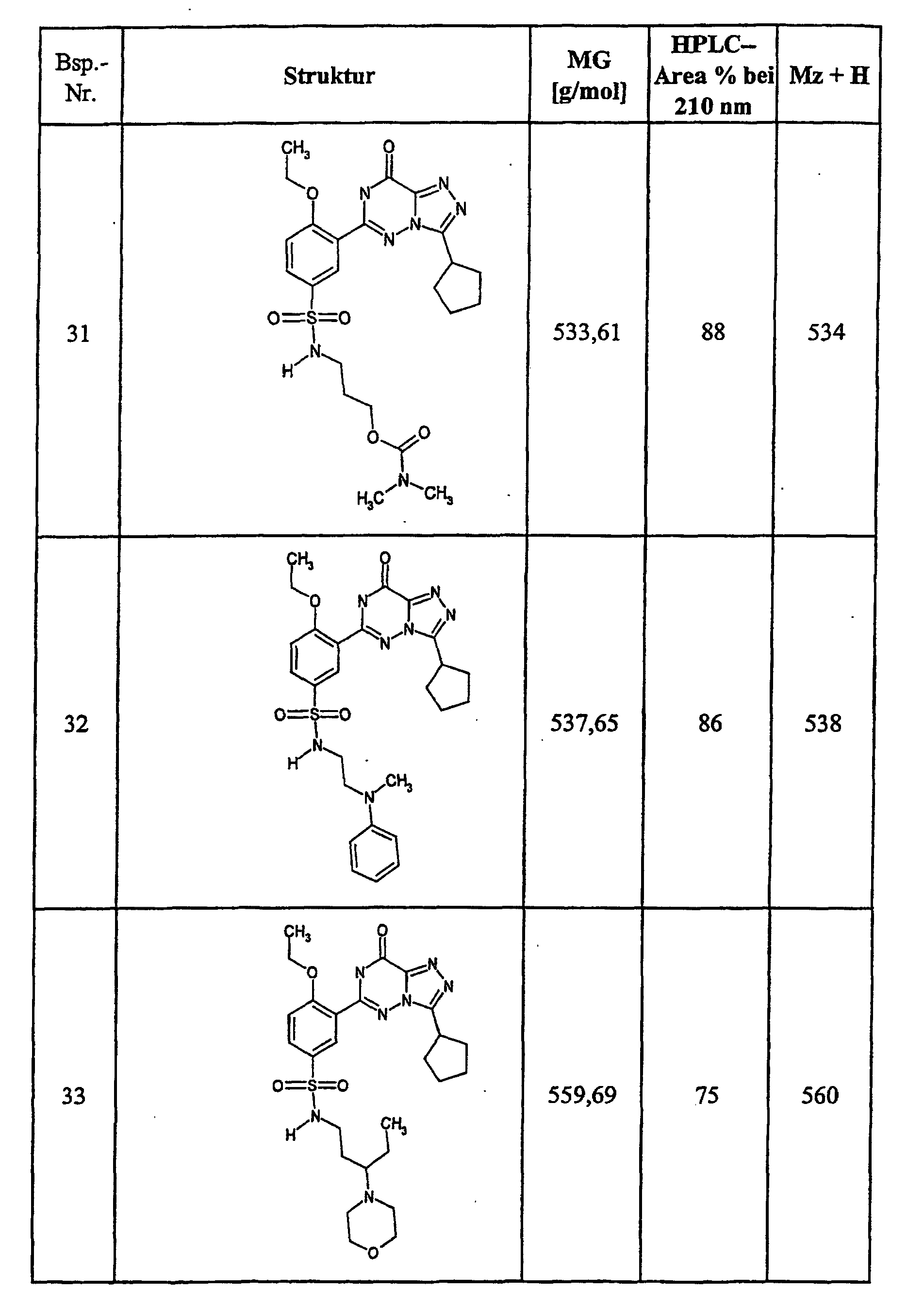

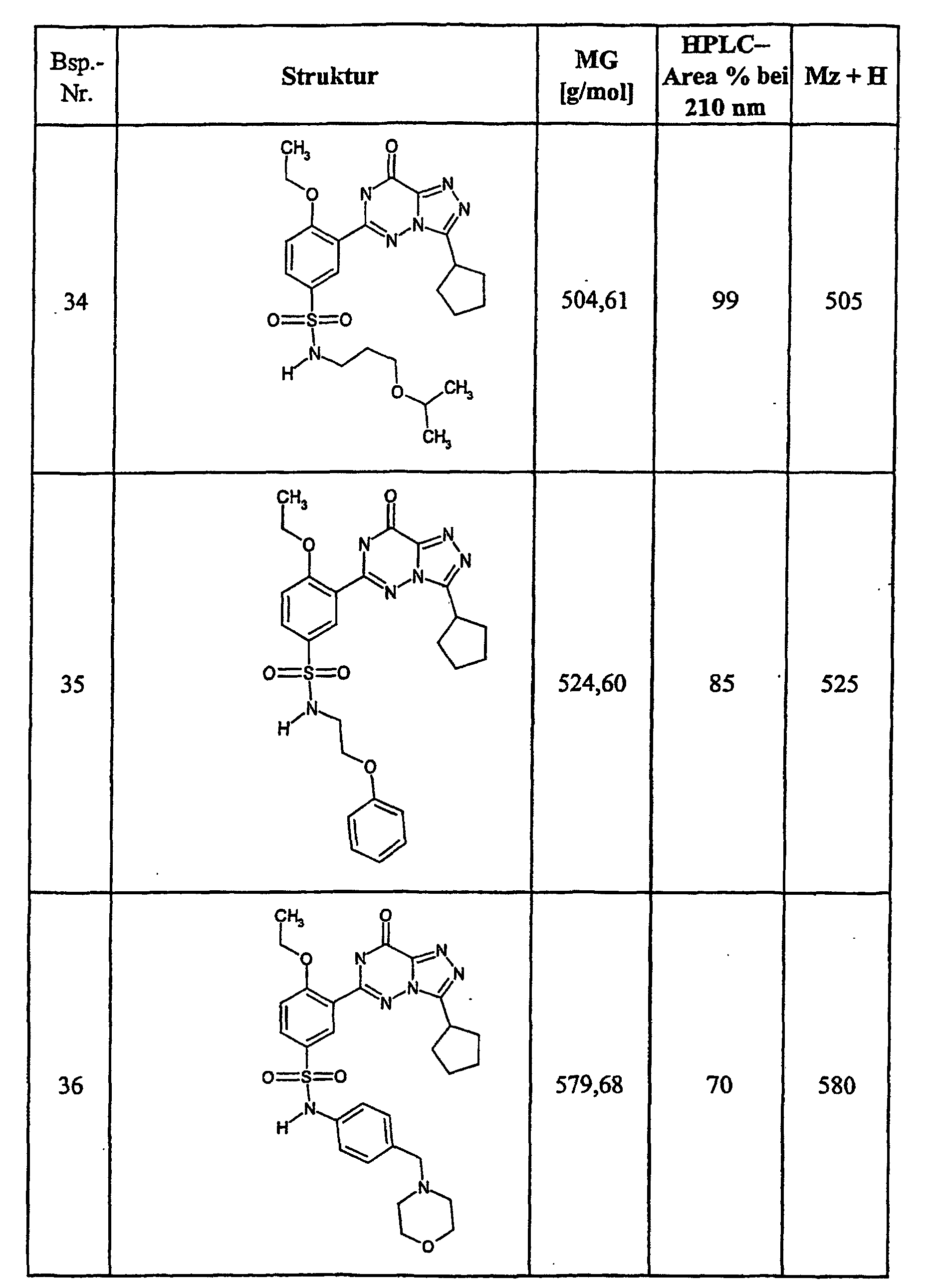

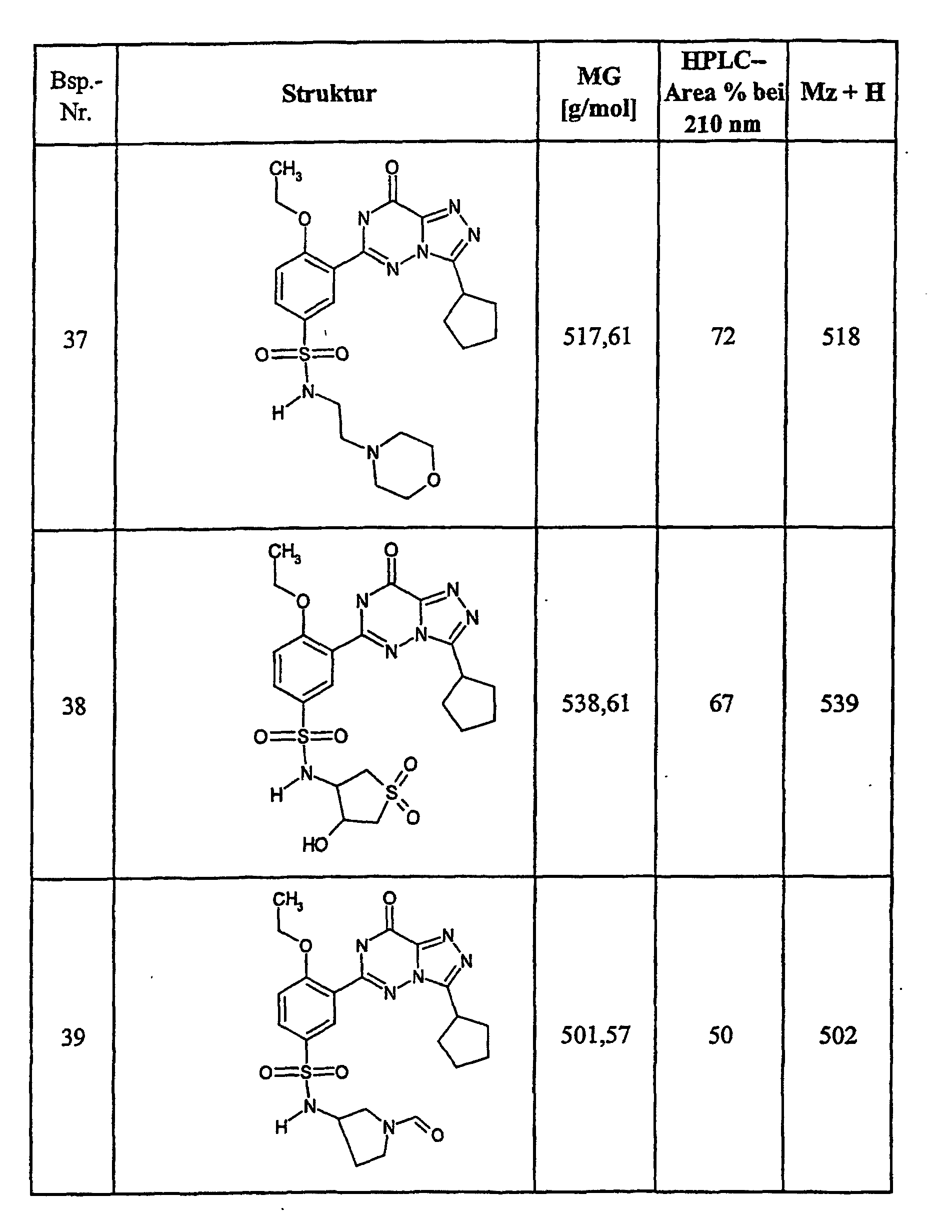

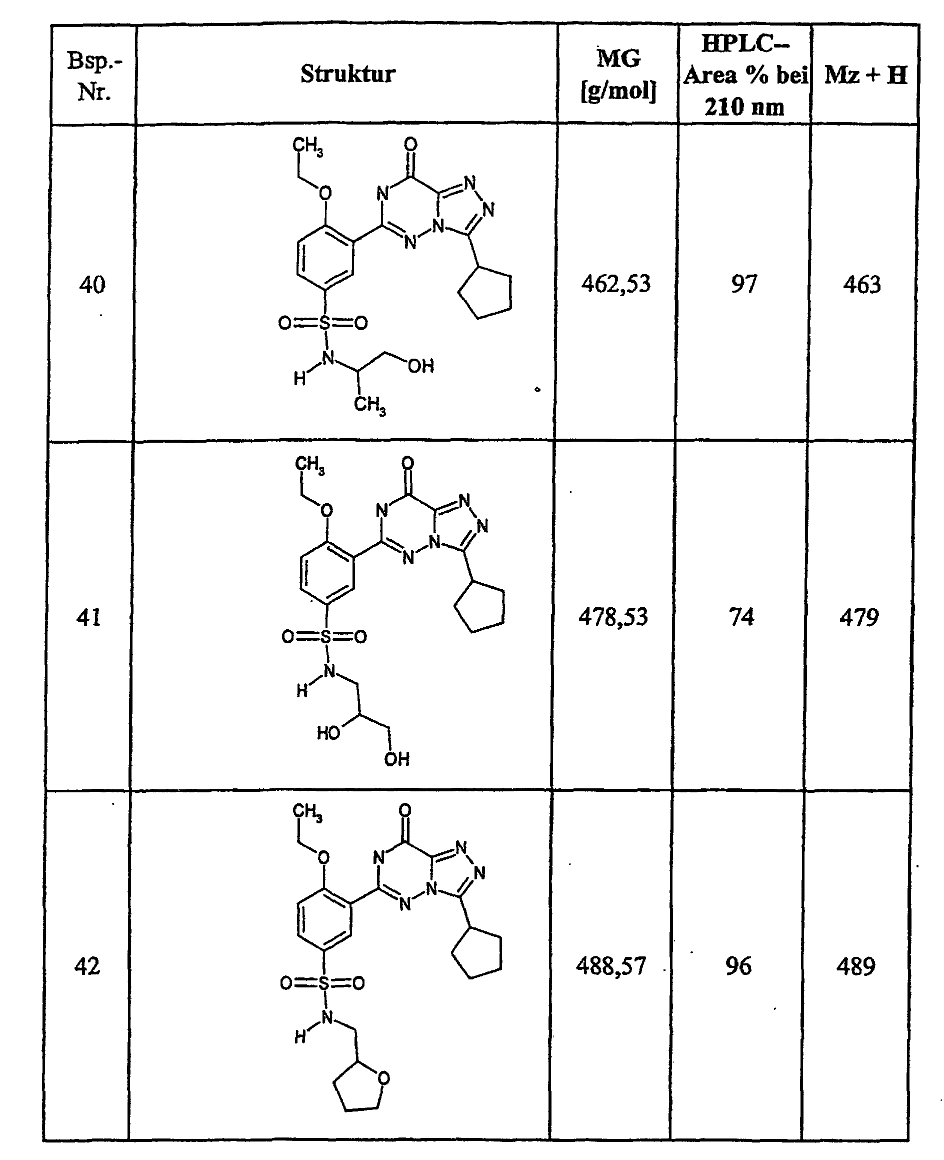

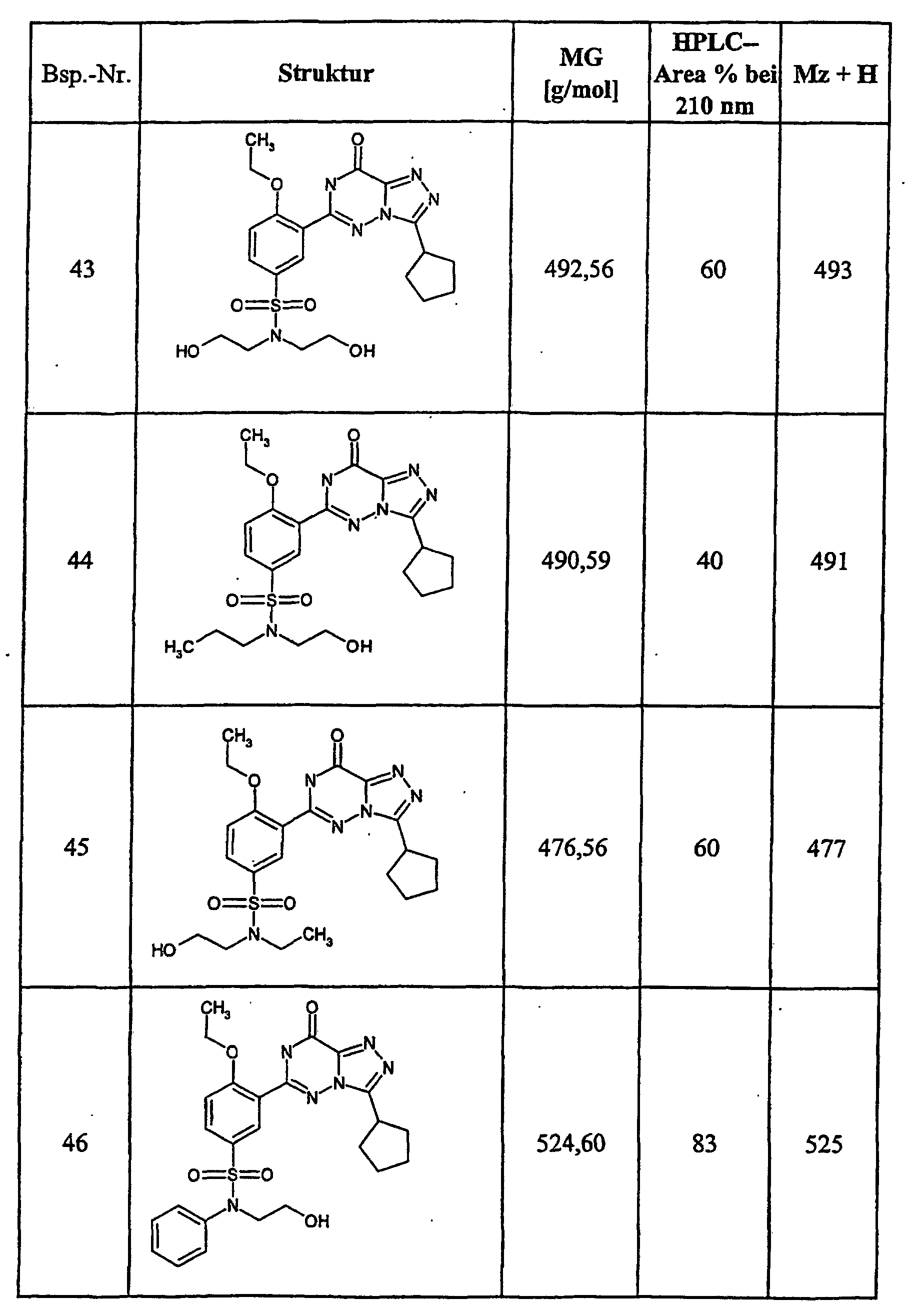

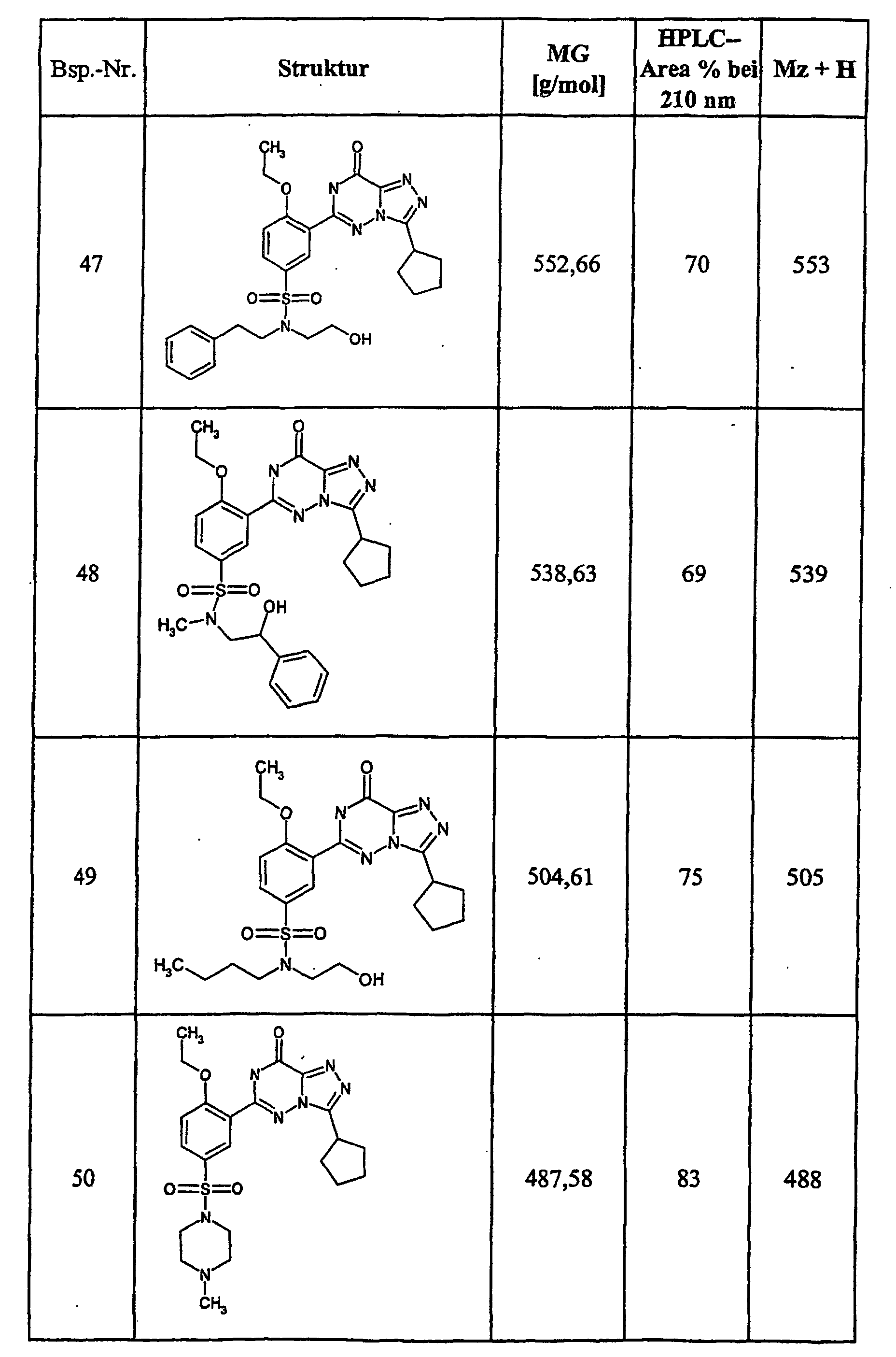

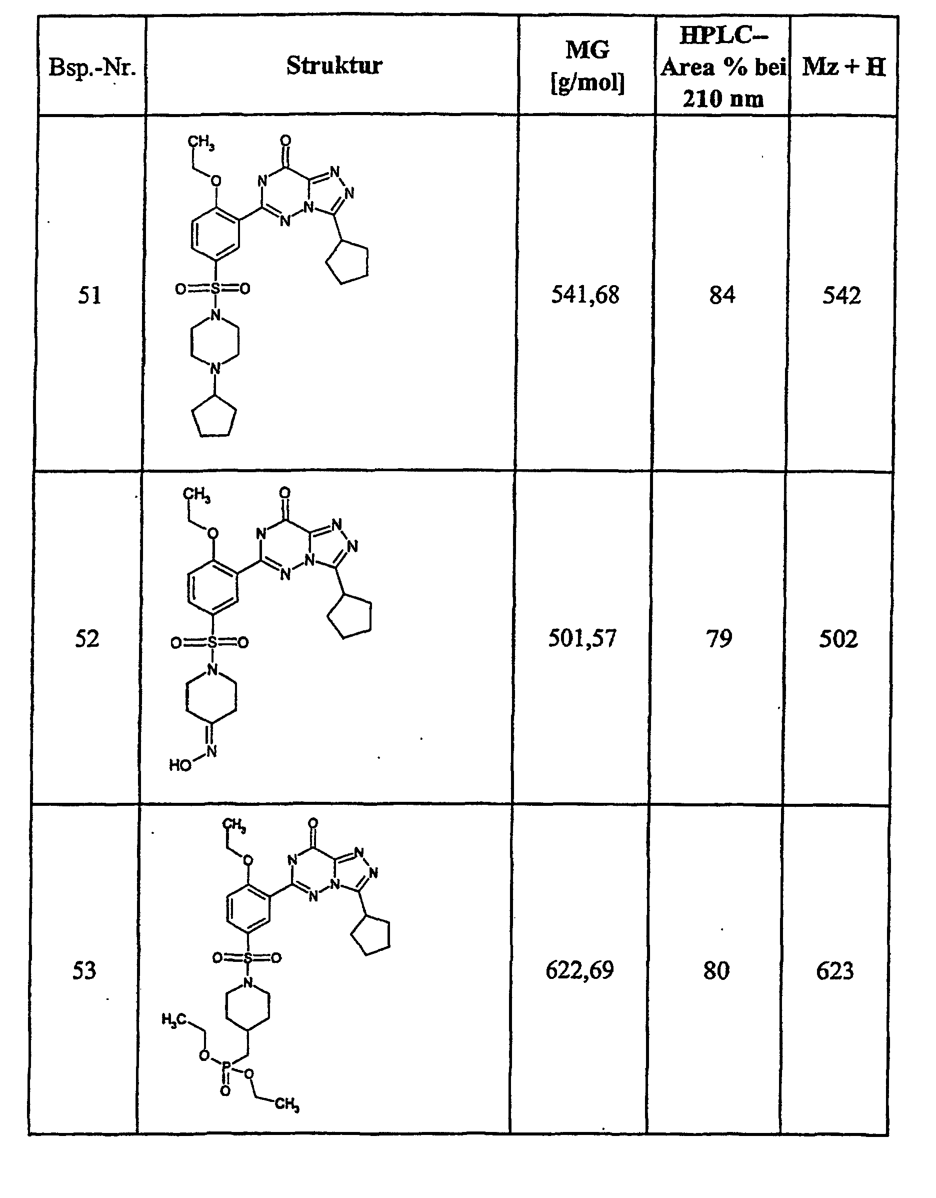

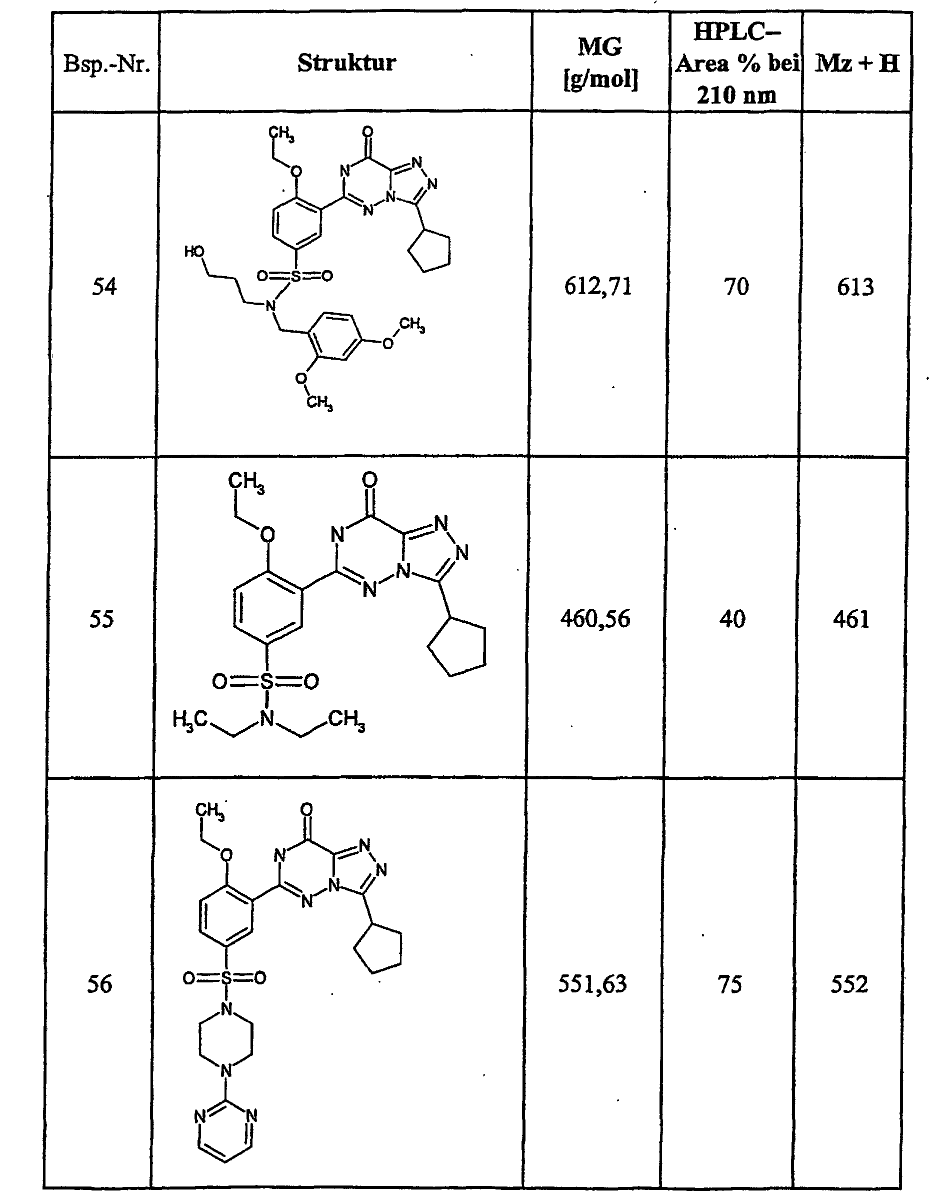

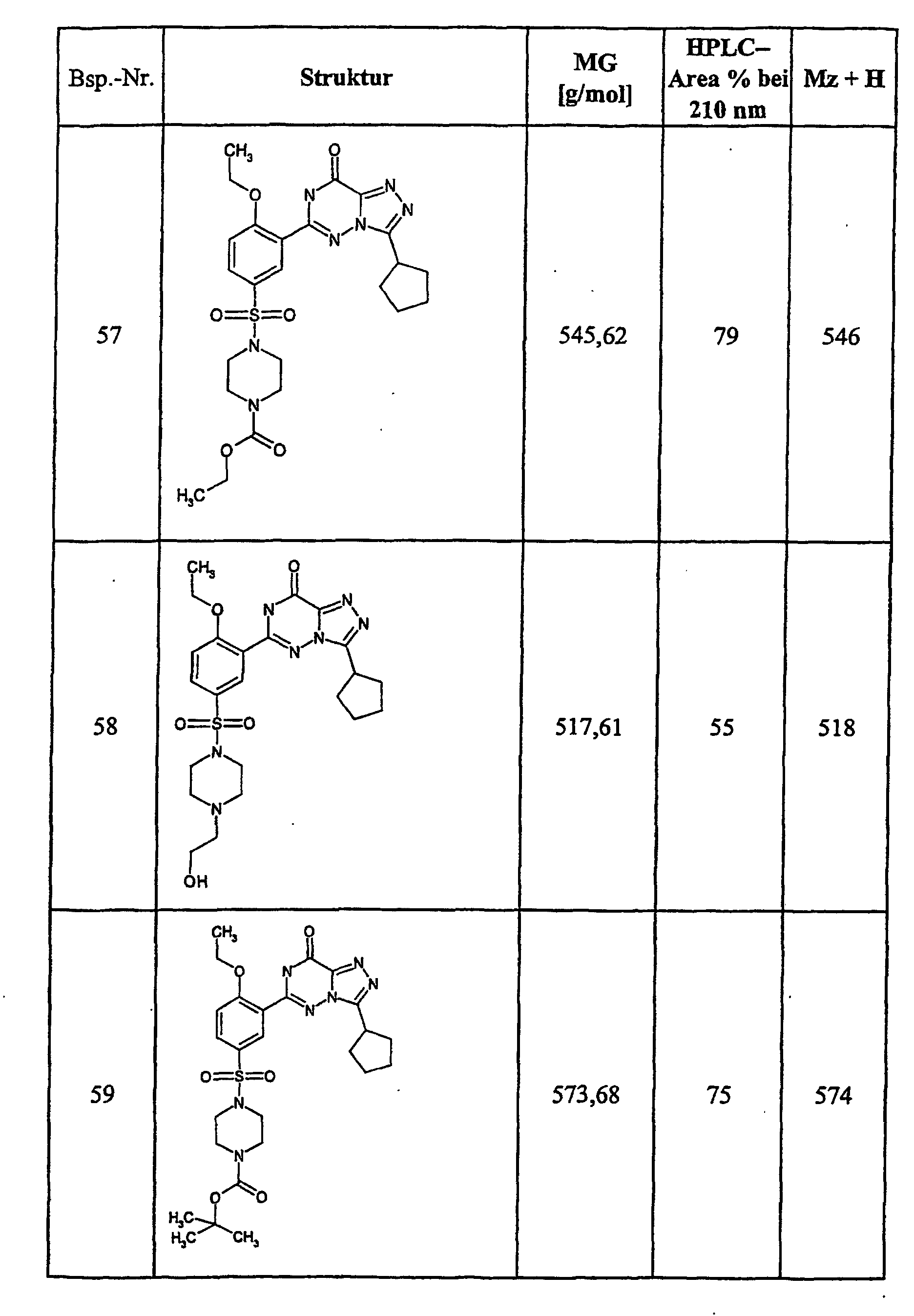

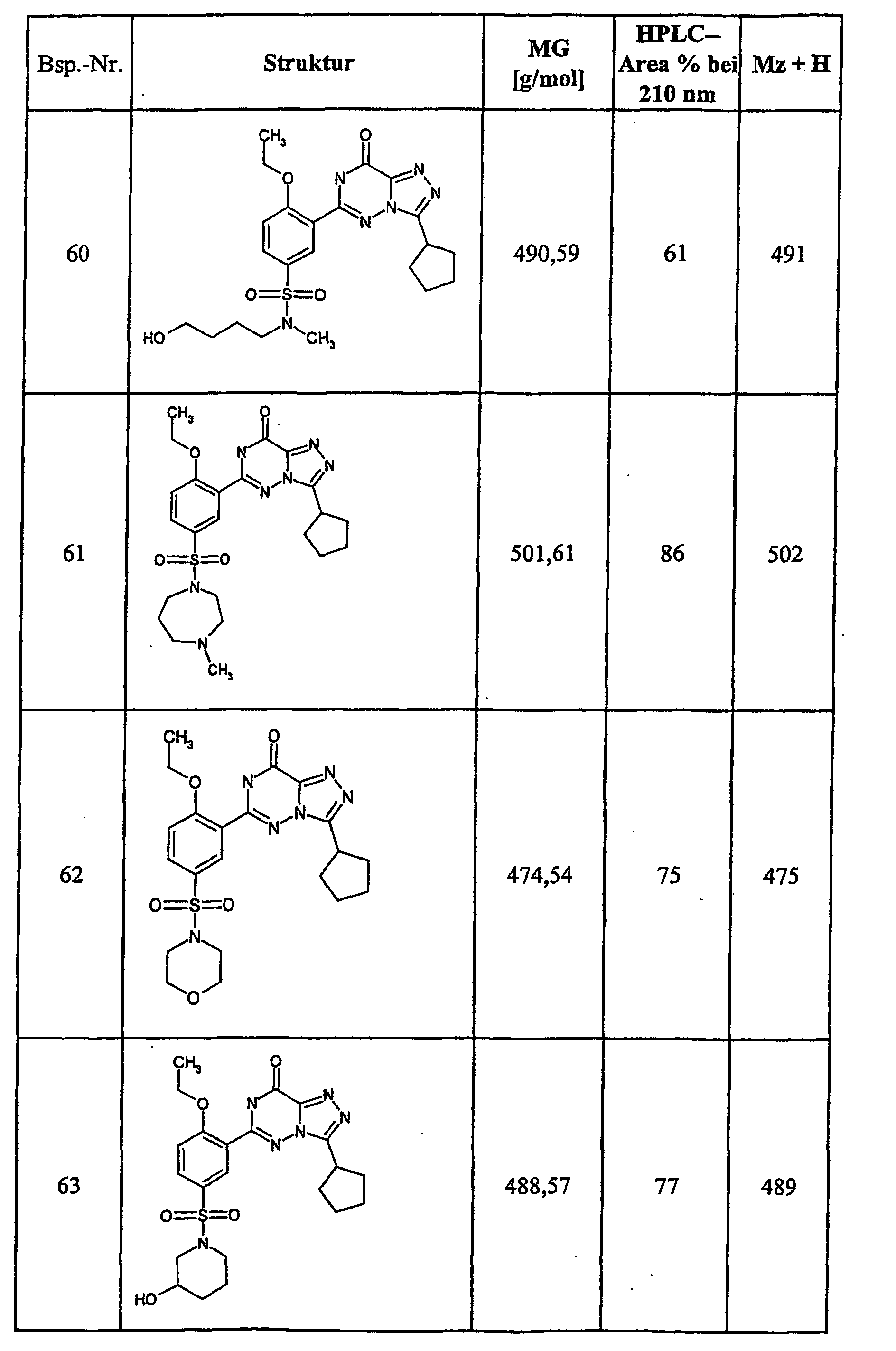

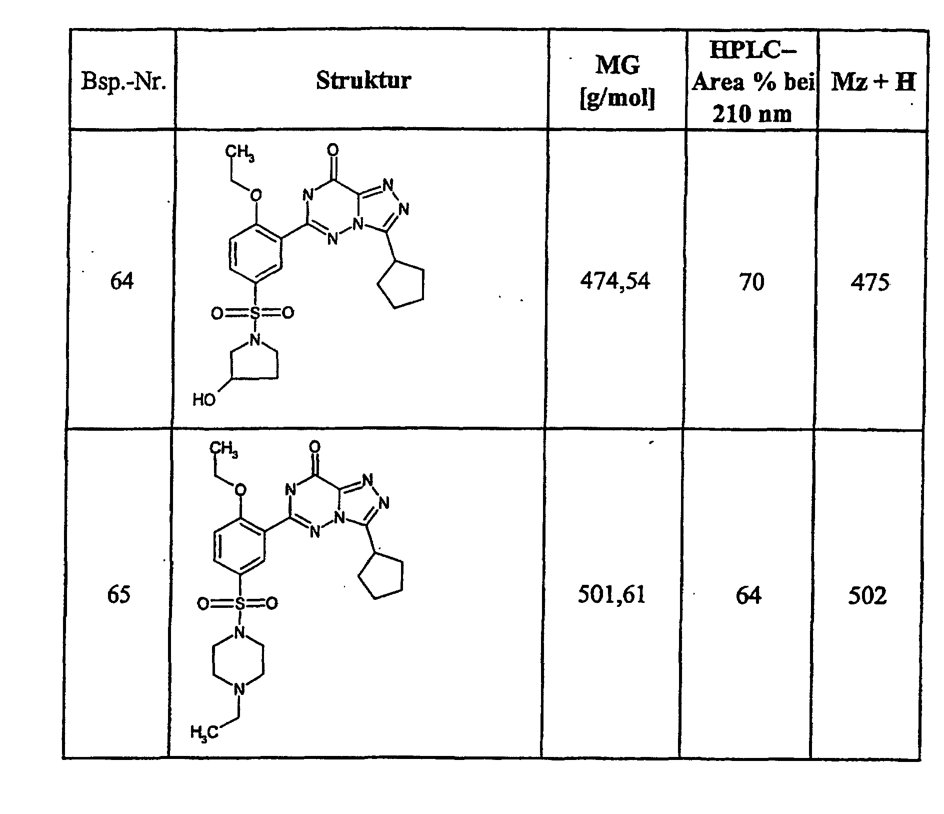

## Patentansprüche

1. Neue Triazolotriazinone der allgemeinen Formel (I) 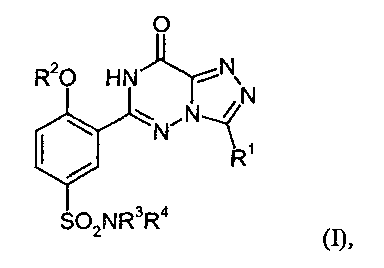 in welcher
R¹ für (C₃-C₈)-Cycloalkyl steht,
R² für Wasserstoff oder für geradkettiges oder verzweigtes Alkyl mit bis zu 6 Kohlenstoffatomen steht,
R³ und R⁴ gleich oder verschieden sind und für Wasserstoff, (C₁-C₆)-Alkoxy oder für (C₁-C₆)-Alkyl stehen, das gegebenenfalls bis zu 3-fach, gleich oder verschieden durch Hydroxy, (C₁-C₅)-Alkoxy, Phenoxy oder durch Reste der Formeln
―O-CO-NR⁵R⁶,
―NR⁷R⁸
oder 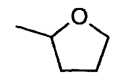 substituiert ist,
worin
R⁵, R⁶, R⁷ und R⁸ gleich oder verschieden sind und Wasserstoff, (C₁-C₆)-Alkyl oder Phenyl bedeuten,
oder
R⁷ und R⁸ gemeinsam mit dem Stickstoffatom, an das sie gebunden sind, einen 5- bis 6-gliedrigen, gesättigten Heterocyclus bilden, der noch ein weiteres Heteroatom aus der Reihe S und O enthalten kann,
und/oder seinerseits (C₁-C₆)-Alkyl gegebenenfalls durch Phenyl substituiert ist, das gegebenenfalls bis zu 3-fach gleich oder verschieden durch Hydroxy, (C₁-C₆)-Alkoxy, Halogen oder durch (C₁-C₆)-Alkyl substituiert ist, das seinerseits wiederum durch Hydroxy oder (C₁-C₆)-Alkoxy substituiert ist, oder Phenyl gegebenenfalls durch Reste der Formeln -SO₂-NR⁹R¹⁰ oder -NR¹¹R¹² substituiert ist,
worin
R⁹, R¹⁰, R¹¹ und R¹² gleich oder verschieden sind und Wasserstoff, (C₁-C₆)-Alkyl oder Phenyl bedeuten,
oder
R¹¹ und R¹² gemeinsam mit dem Stickstoffatom, an das sie gebunden sind; einen 5- bis 6-gliedrigen, gesättigten Heterocyclus bilden, der noch ein weiteres Heteroatom aus der Reihe S und O enthalten kann,
oder
R³ für Wasserstoff oder (C₁-C₆)-Alkyl steht,
und
R⁴ für Reste der Formel 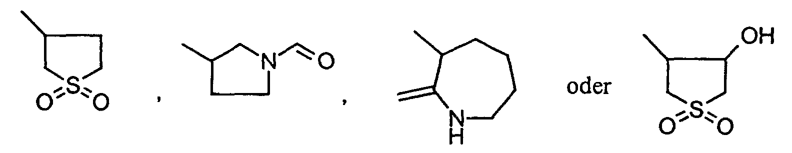
steht,
oder
für Phenyl steht, das gegebenenfalls bis zu 3-fach, gleich oder verschieden durch Halogen, (C₁-C₆)-Alkoxy, Hydroxy, durch einen Rest der Formel 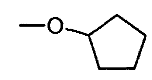 oder durch (C₁-C₆)-Alkyl substituiert ist, das seinerseits durch Hydroxy oder (C₁-C₆)-Alkoxy substituiert sein kann,
oder
R³ und R⁴ gemeinsam mit dem Stickstoffatom, an das sie gebunden sind, einen Rest der Formel 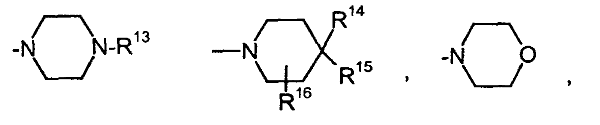 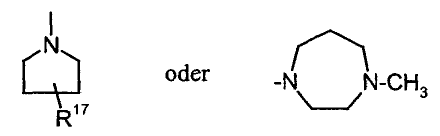 bilden,
worin
R¹³ Wasserstoff, (C₁-C₆)-Alkoxycarbonyl, (C₃-C₆)-Cycloalkyl, Pyridyl, Pyrimidyl oder (C₁-C₆)-Alkyl bedeutet, das gegebenenfalls durch Hydroxy substituiert ist,
R¹⁴ und R¹⁵ gleich oder verschieden sind und Wasserstoff, Hydroxy oder (C₁-C₆)-Alkyl bedeuten, das gegebenenfalls durch Hydroxy oder durch einen Rest der Formel -P(O)(OR¹⁸)(OR¹⁹) substituiert ist,
worin
R¹⁸ und R¹⁹ gleich oder verschieden sind und Wasserstoff oder (C₁-C₆)-Alkyl bedeuten,
oder
R¹⁴ und R¹⁵ gemeinsam einen Rest der Formel =N-OH bilden,
R¹⁶ und R¹⁷ gleich oder verschieden sind und Wasserstoff oder (C₁-C₆)-Alkyl bedeuten, das gegebenenfalls durch Hydroxy substituiert ist,
und deren Salze, N-Oxide und stereoisomere Formen.

2. Neue Triazolotriazinone der allgemeinen Formel (I) gemäß Anspruch 1,
in welcher
R¹ für Cyclopropyl, Cyclopentyl oder Cyclohexyl steht,
R² für geradkettiges oder verzweigtes Alkyl mit bis zu 4 Kohlenstoffatomen steht,
R³ und R⁴ gleich oder verschieden sind und für Wasserstoff, Methoxy oder für (C₁-C₅)-Alkyl stehen, das gegebenenfalls bis zu 3-fach, gleich oder verschieden durch Hydroxy, (C₁-C₄)-Alkoxy, Phenoxy oder durch Gruppen der Formeln
―O-CO-NR⁵R⁶ ,
―NR⁷R⁸
oder 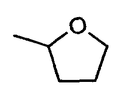 substituiert ist, worin
R⁵, R⁶, R⁷ und R⁸ gleich oder verschieden sind und Wasserstoff, (C₁-C₄)-Alkyl oder Phenyl bedeuten,
oder
R⁷ und R⁸ gemeinsam mit dem Stickstoffatom, an das sie gebunden sind, einen Morpholin-, Piperidin- oder Pyrrolidinring bilden,
und/oder seinerseits (C₁-C₅)-Alkyl gegebenenfalls durch Phenyl substituiert ist, das gegebenenfalls bis zu 3-fach gleich oder verschieden durch Hydroxy, (C₁-C₄)-Alkoxy oder durch (C₁-C₄)-Alkyl substituiert sein kann, das seinerseits wiederum durch Hydroxy oder (C₁-C₄)-Alkoxy substituiert ist, oder Phenyl gegebenenfalls durch Reste der Formeln -SO₂-NR⁹R¹⁰ oder -NR¹¹R¹² substituiert ist,
worin
R⁹, R¹⁰, R¹¹ und R¹² gleich oder verschieden sind und Wasserstoff, (C₁-C₄)-Alkyl oder Phenyl bedeuten,
oder
R¹¹ und R¹² gemeinsam mit dem Stickstoffatom, an das sie gebunden sind, einen Morpholin-, Piperidin- oder Pyrrolidinring bilden,
oder
R³ für Wasserstoff oder (C₁-C₄)-Alkyl steht,
und
R⁴ für Reste der Formel 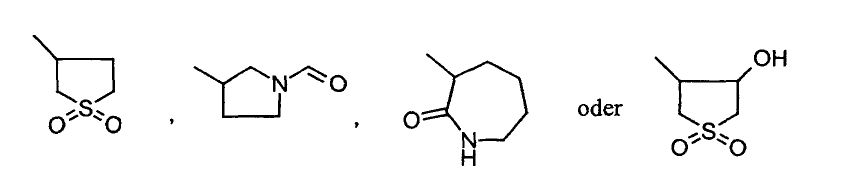
steht,
oder
für Phenyl steht, das gegebenenfalls bis zu 3-fach, gleich oder verschieden durch Fluor, (C₁-C₄)-Alkoxy, Hydroxy, durch einen Rest der Formel 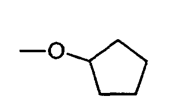 oder durch (C₁-C₄)-Alkyl substituiert ist, das seinerseits durch Hydroxy oder (C₁-C₃)-Alkoxy substituiert sein kann,
oder
R³ und R⁴ gemeinsam mit dem Stickstoffatom, an das sie gebunden sind, einen Rest der Formel 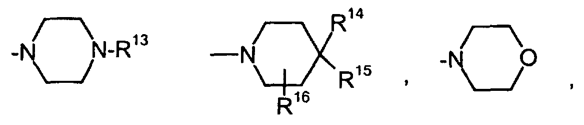 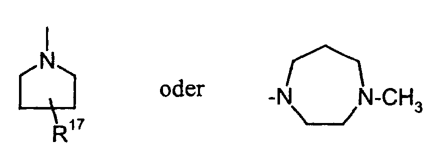 bilden,
worin
R¹³ Wasserstoff, (C₁-C₄)-Alkoxycarbonyl, Cyclopentyl, Cyclohexyl, Pyridyl, Pyrimidyl oder (C₁-C₅)-Alkyl bedeutet, das gegebenenfalls durch Hydroxy substituiert ist,
R¹⁴ und R¹⁵ gleich oder verschieden sind und Wasserstoff oder (C₁-C₅)-Alkyl bedeuten, das gegebenenfalls durch Hydroxy oder durch einen Rest der Formel -P(O)(OR¹⁸)(OR¹⁹) substituiert ist,
worin
R¹⁸ und R¹⁹ gleich oder verschieden sind und Wasserstoff, Methyl oder Ethyl bedeuten,
oder
R¹⁴ und R¹⁵ gemeinsam einen Rest der Formel =N-OH bilden,
R¹⁶ und R¹⁷ gleich oder verschieden sind und Wasserstoff, Hydroxy oder (C₁-C₃)-Alkyl bedeuten, das gegebenenfalls durch Hydroxy substituiert ist,
und deren Salze, N-Oxide und stereoisomere Formen.

3. Neue Triazolotriazinone der allgemeinen Formel (I) gemäß Anspruch 1,
in welcher
R¹ für Cyclopentyl steht,
R² für geradkettiges oder verzweigtes Alkyl mit bis zu 3 Kohlenstoffatomen steht,
R³ und R⁴ gleich oder verschieden sind und für Wasserstoff, Methoxy oder für (C₁-C₄)-Alkyl stehen, das gegebenenfalls bis zu 3-fach, gleich oder verschieden durch Hydroxy, (C₁-C₄)-Alkoxy, Phenoxy oder durch Gruppen der Formeln
―O-CO-NR⁵R⁶ ,
―NR⁷R⁸
oder 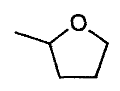 substituiert ist,
worin
R⁵, R⁶, R⁷ und R⁸ gleich oder verschieden sind und Wasserstoff, (C₁-C₃)-Alkyl oder Phenyl bedeuten,
oder
R⁷ und R⁸ gemeinsam mit dem Stickstoffatom, an das sie gebunden sind, einen Morpholin-, Piperidin- oder Pyrrolidinring bilden,
und/oder seinerseits (C₁-C₄)-Alkyl gegebenenfalls durch Phenyl substituiert ist, das gegebenenfalls bis zu 3-fach gleich oder verschieden durch Hydroxy, (C₁-C₃)-Alkoxy, Fluor oder durch (C₁-C₃)-Alkyl substituiert ist, das seinerseits wiederum durch Hydroxy oder (C₁-C₄)-Alkoxy substituiert ist, oder Phenyl gegebenenfalls durch Reste der Formeln -SO₂-NR⁹R¹⁰ oder -NR¹¹R¹² substituiert ist,
worin
R⁹, R¹⁰, R¹¹ und R¹² gleich oder verschieden sind und Wasserstoff, (C₁-C₃)-Alkyl oder Phenyl bedeuten,
oder
R¹¹ und R¹² gemeinsam mit dem Stickstoffatom, an das sie gebunden sind, einen Morpholin-, Piperidin- oder Pyrrolidinring bilden,
oder
R³ für Wasserstoff oder Methyl steht,
und
R⁴ für Reste der Formel 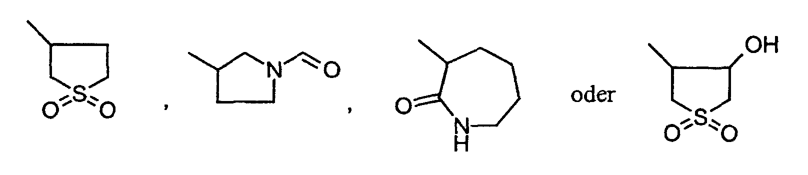
steht,
oder
für Phenyl steht, das gegebenenfalls bis zu 3-fach, gleich oder verschieden durch Fluor, Methoxy, Hydroxy, durch einen Rest der Formel 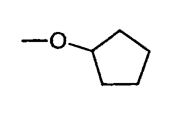 oder durch (C₁-C₄)-Alkyl substituiert ist, das seinerseits durch Hydroxy oder Methoxy oder Ethoxy substituiert sein kann,
oder
R³ und R⁴ gemeinsam mit dem Stickstoffatom, an das sie gebunden sind, einen Rest der Formel 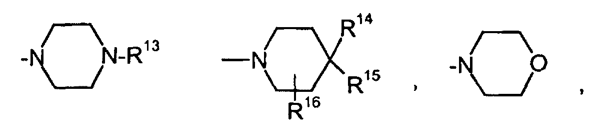 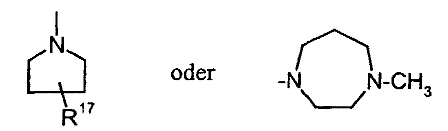 bilden,
worin
R¹³ Wasserstoff, (C₁-C₄)-Alkoxycarbonyl, Cyclopentyl, Pyrimidyl oder (C₁-C₃)-Alkyl bedeutet, das gegebenenfalls durch Hydroxy substituiert ist,
R¹⁴ und R¹⁵ gleich oder verschieden sind und (C₁-C₃)-Alkyl bedeuten, das gegebenenfalls durch Hydroxy oder durch einen Rest der Formel -P(O)(OR¹⁸)(OR¹⁹) substituiert ist,
worin
R¹⁸ und R¹⁹ Ethyl bedeuten,
oder
R¹⁴ und R¹⁵ gemeinsam einen Rest der Formel =N-OH bilden,
R¹⁶ und R¹⁷ gleich oder verschieden sind und Wasserstoff oder (C₁-C₃)-Alkyl bedeuten, das gegebenenfalls durch Hydroxy substituiert ist,
und deren Salze, N-Oxide und stereoisomere Formen.

4. Neue Triazolotriazinone der allgemeinen Formel (I), gemäß Anspruch 1 bis 3 mit folgenden Strukturen:

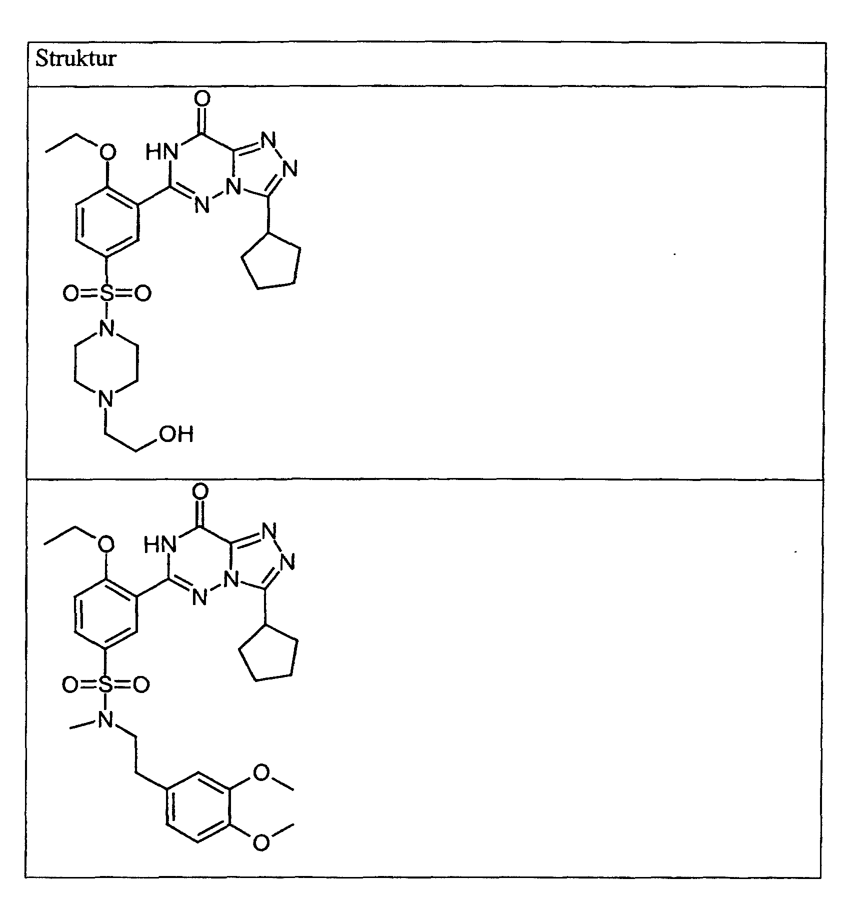

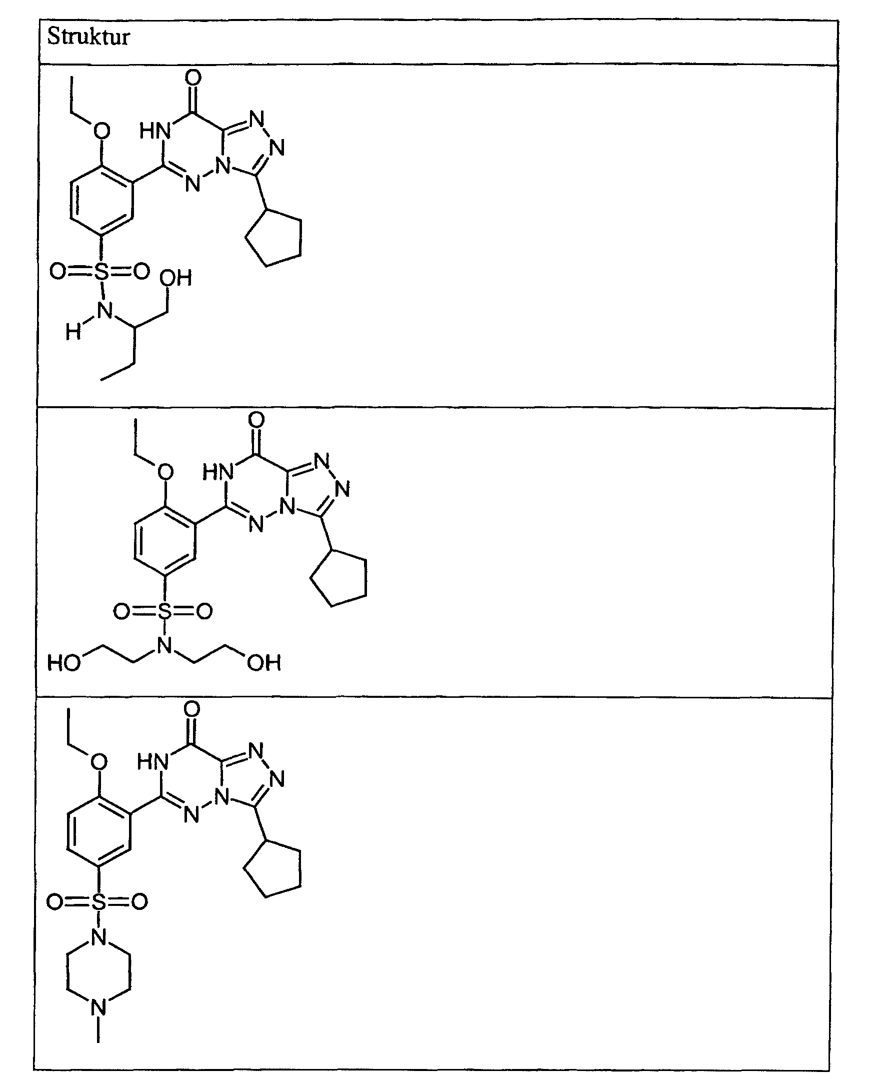

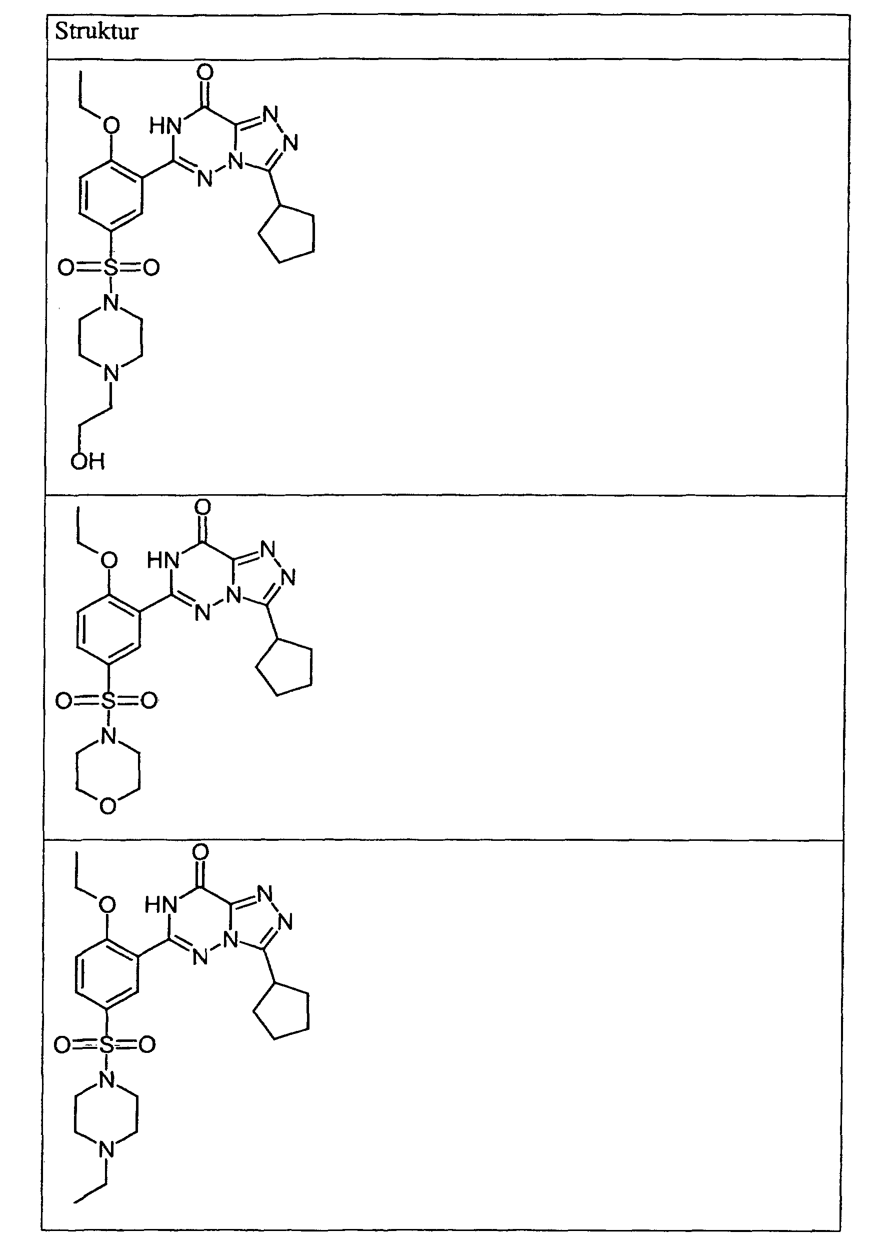

5. Verfahren zur Herstellung von Triazolotriazinonen gemäß Ansprüchen 1 bis 4, **dadurch gekennzeichnet, dass** man Verbindungen der allgemeinen Formel (II) 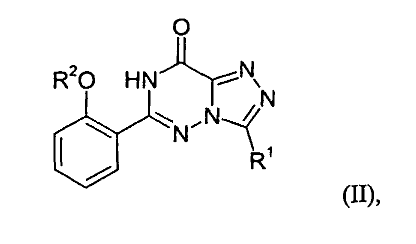 in welcher
R¹ und R² die oben angegebene Bedeutung haben,
mit Chlorsulfonsäure (ClSO₃H) gegebenenfalls in inerten Lösemitteln, gegebenenfalls in Anwesenheit einer Base zu den Verbindungen der allgemeinen Formel (III) 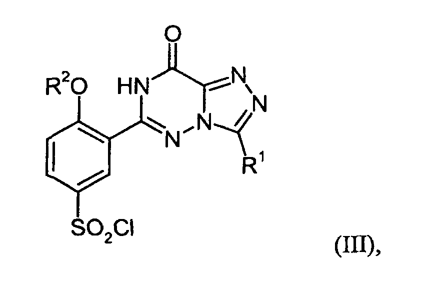 in welcher
R¹ und R² die oben angegebene Bedeutung haben,
umsetzt und abschließend mit Aminen der allgemeinen Formel (IV)
HNR³R⁴ (IV),
in welcher
R³ und R⁴ die oben angegebene Bedeutung haben,
umsetzt.

6. Verbindungen der allgemeinen Formel (I) gemäß Ansprüchen 1 bis 4 zur Prophylaxe und/oder Behandlung von Erkrankungen.

7. Arzneimittel oder pharmazeutische Zusammensetzung, enthaltend mindestens eine Verbindung der allgemeinen Formel (I) gemäß einem der Ansprüche 1 bis 4 sowie einen oder mehrere pharmakologisch unbedenkliche Hilfs- und Trägerstoffe.

8. Arzneimittel oder pharmazeutische Zusammensetzung gemäß Anspruch 7 zur Prophylaxe und/oder Behandlung von Erkrankungen, die im Zusammenhang mit cGMP-regulierten Vorgängen stehen ('cGMP-related diseases').

9. Arzneimittel oder pharmazeutische Zusammensetzung gemäß Anspruch 7 oder 8 zur Prophylaxe und/oder Behandlung von kardiovaskulären Erkrankungen, Erkrankungen des Urogenitalsystems sowie cerebrovaskulären Erkrankungen.

10. Arzneimittel oder pharmazeutische Zusammensetzung gemäß einem der Ansprüche 7 bis 9 zur Prophylaxe und/oder Behandlung von kardiovaskulären Erkrankungen wie Bluthochdruck, neuronale Hypertonie, stabile und instabile Angina, periphere und kardiale Gefäßerkrankungen, Arrhythmien, thromboembolische Erkrankungen und Ischämien wie Myokardinfarkt, Himschlag, transistorische und ischämische Attacken, Angina pectoris, periphere Durchblutungsstörungen, Verhinderung von Restenosen nach Thrombolysetherapie, percutaner transluminaler Angioplastie (PTA), percutan transluminaler Koronarangioplastien (PTCA) und Bypass.

11. Arzneimittel oder pharmazeutische Zusammensetzung gemäß einem der Ansprüche 7 bis 9 zur Prophylaxe und/oder Behandlung von cerebrovaskulären Erkrankungen wie cerebrale Ischämie, Himschlag, Reperfusionsschäden, Himtrauma, Ödeme, cerebrale Thrombose, Demenz und Alzheimer'sche Erkrankung.

12. Arzneimittel oder pharmazeutische Zusammensetzung gemäß einem der Ansprüche 7 bis 9 zur Prophylaxe und/oder Behandlung von Erkrankungen des Urogenitalsystems wie Prostatahypertrophie, Inkontinenz sowie insbesondere erektile Dysfunktion und weibliche sexuelle Dysfunktion.

13. Arzneimittel oder pharmazeutische Zusammensetzung gemäß einem der Ansprüche 7 bis 12, **dadurch gekennzeichnet, dass** das Arzneimittel oder die pharmazeutische Zusammensetzung intravenös oder oral appliziert wird.

14. Verwendung der Verbindungen der allgemeinen Formel (I) gemäß einem der Ansprüche 1 bis 4 zur Herstellung von Arzneimitteln oder pharmazeutischen Zusammensetzungen zur Prophylaxe und/oder Behandlung von Krankheiten.

15. Verwendung gemäß Anspruch 14 zur Herstellung eines Arzneimittels oder einer pharmazeutischen Zusammensetzung zur Prophylaxe und/oder Behandlung von Erkrankungen, die im Zusammenhang mit cGMP-regulierten Vorgängen stehen ('cGMP-related diseases').

16. Verwendung gemäß Anspruch 14 oder 15 zur Herstellung eines Arzneimittels oder einer pharmazeutischen Zusammensetzung zur Prophylaxe und/oder Behandlung von kardiovaskulären Erkrankungen, Erkrankungen des Urogenitalsystems sowie cerebrovaskulären Erkrankungen.

17. Verwendung gemäß einem der Ansprüche 14 bis 16 zur Herstellung eines Arzneimittels oder einer pharmazeutischen Zusammensetzung zur Prophylaxe und/oder Behandlung von kardiovaskulären Erkrankungen wie Bluthochdruck, neuronale Hypertonie, stabile und instabile Angina, periphere und kardiale Gefäßerkrankungen, Arrhythmien, thromboembolische Erkrankungen und Ischämien wie Myokardinfarkt, Himschlag, transistorische und ischämische Attacken, Angina pectoris, periphere Durchblutungsstörungen, Verhinderung von Restenosen nach Thrombolysetherapie, percutaner transluminaler Angioplastie (PTA), percutan transluminaler Koronarangioplastien (PTCA) und Bypass.

18. Verwendung gemäß einem der Ansprüche 14 bis 16 zur Herstellung eines Arzneimittels oder einer pharmazeutischen Zusammensetzung zur Prophylaxe und/oder Behandlung von cerebrovaskulären Erkrankungen wie cerebrale Ischämie, Himschlag, Reperfusionsschäden, Himtrauma, Ödeme, cerebrale Thrombose, Demenz und Alzheimer'sche Erkrankung.

19. Verwendung gemäß einem der Ansprüche 14 bis 16 zur Herstellung eines Arzneimittels oder einer pharmazeutischen Zusammensetzung zur Prophylaxe und/oder Behandlung von Erkrankungen des Urogenitalsystems wie Prostatahypertrophie, Inkontinenz sowie insbesondere erektile Dysfunktion und weibliche sexuelle Dysfunktion.

20. Verwendung gemäß einem der Ansprüche 14 bis 19, **dadurch gekennzeichnet, dass** die Arzneimittel oder Zusammensetzungen intravenös oder oral appliziert werden.

## Claims

1. Novel triazolotriazinone of the general formula (I) 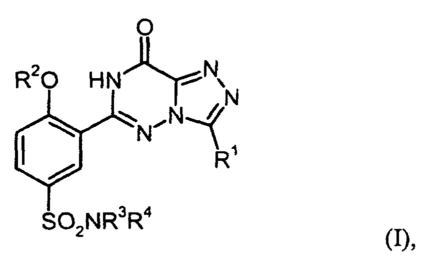 in which
R¹ represents (C₃-C₈)-cycloalkyl,
R² represents hydrogen or represents straight-chain or branched alkyl having up to 6 carbon atoms,
R³ and R⁴ are identical or different and represent hydrogen or (C₁-C₆)-alkoxy or represent (C₁-C₆)-alkyl which is optionally substituted, up to 3 times, identically or differently, by hydroxyl, (C₁-C₅)-alkoxy or phenoxy or by radicals of the formulae
―O-CO-NR⁵R⁶ ,
―NR⁷R⁸
or 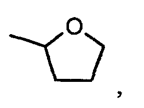 in which
R⁵, R⁶, R⁷ and R⁸ are identical or different and denote hydrogen, (C₁-C₆)-alkyl or phenyl,
or
R⁷ and R⁸, together with the nitrogen atom to which they are bonded, form a 5- to 6-membered, saturated heterocycle which can additionally contain a further heteroatom from the series S and O,
and/or (C₁-C₆)-alkyl is, for its part, optionally substituted by phenyl which is optionally substituted, up to 3 times, identically or differently, by hydroxyl, (C₁-C₆)-alkoxy or halogen or by (C₁-C₆)-alkyl which, for its part, is in turn substituted by hydroxyl or (C₁-C₆)-alkoxy, or phenyl is optionally substituted by radicals of the formulae -SO₂-NR⁹R¹⁰ or -NR¹¹R¹²,
in which
R⁹, R¹⁰, R¹¹ and R¹² are identical or different and denote hydrogen, (C₁-C₆)-alkyl or phenyl,
or
R¹¹ and R¹², together with the nitrogen atom to which they are bonded, form a 5- to 6-membered, saturated heterocycle which can additionally contain a further heteroatom from the series S and O,
or
R³ represents hydrogen or (C₁-C₆)-alkyl,
and
R⁴ represents radicals of the formula 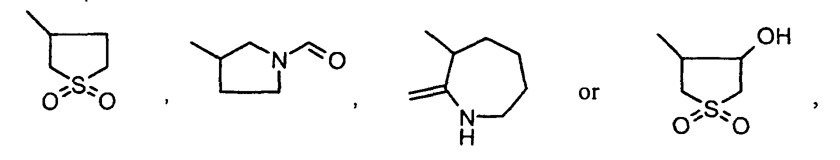
or
represents phenyl which is optionally substituted, up to 3 times, identically or differently, by halogen, (C₁-C₆)-alkoxy or hydroxyl or by a radical of the formula
or by (C₁-C₆)-alkyl which, for its part, can be substituted by hydroxyl or (C₁-C₆)-alkoxy,
or
R³ and R⁴, together with the nitrogen atom to which they are bonded, form a radical of the formula 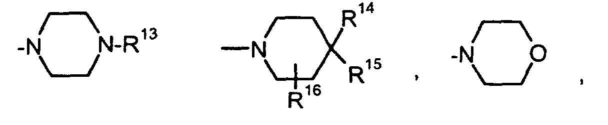 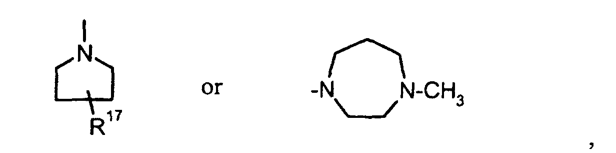 in which
R¹³ denotes hydrogen, (C₁-C₆)-alkoxycarbonyl, (C₃-C₆)-cycloalkyl, pyridyl, pyrimidyl or (C₁-C₆)-alkyl which is optionally substituted by hydroxyl,
R¹⁴ and R¹⁵ are identical or different and denote hydrogen, hydroxyl or (C₁-C₆)-alkyl which is optionally substituted by hydroxyl or by a radical of the formula -P(O)(OR¹⁸)(OR¹⁹),
in which
R¹⁸ and R¹⁹ are identical or different and denote hydrogen or (C₁-C₆)-alkyl,
or
R¹⁴ and R¹⁵ together form a radical of the formula =N-OH,
R¹⁶ and R¹⁷ are identical or different and denote hydrogen or (C₁-C₆)-alkyl which is optionally substituted by hydroxyl,
and the salts, N-oxides and stereoisomeric forms thereof.

2. Novel triazolotriazinone of the general formula (I) according to Claim 1,
in which
R¹ represents cyclopropyl, cyclopentyl or cyclohexyl,
R² represents straight-chain or branched alkyl having up to 4 carbon atoms,
R³ and R⁴ are identical or different and represent hydrogen or methoxy or represent (C₁-C₅)-alkyl which is optionally substituted, up to 3 times, identically or differently, by hydroxyl, (C₁-C₄)-alkoxy or phenoxy or by groups of the formulae
―O-CO-NR⁵R⁶ ,
―NR⁷R⁸
or 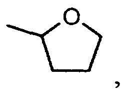 in which
R⁵, R⁶, R⁷ and R⁸ are identical or different and denote hydrogen, (C₁-C₄)-alkyl or phenyl,
or
R⁷ and R⁸, together with the nitrogen atom to which they are bonded, form a morpholine, piperidine or pyrrolidine ring,
and/or (C₁-C₅)-alkyl is, for its part, optionally substituted by phenyl which can be optionally substituted, up to 3 times, identically or differently, by hydroxyl or (C₁-C₄)-alkoxy or by (C₁-C₄)-alkyl which, for its part, is in turn substituted by hydroxyl or (C₁-C₄)-alkoxy, or phenyl is optionally substituted by radicals of the formulae -SO₂-NR⁹R¹⁰ or -NR¹¹R¹²,
in which
R⁹, R¹⁰, R¹¹ and R¹² are identical or different and denote hydrogen, (C₁-C₄)-alkyl or phenyl,
or
R¹¹ and R¹², together with the nitrogen atom to which they are bonded, form a morpholine, piperidine or pyrrolidine ring,
or
R³ represents hydrogen or (C₁-C₄)-alkyl,
and
R⁴ represents radicals of the formula 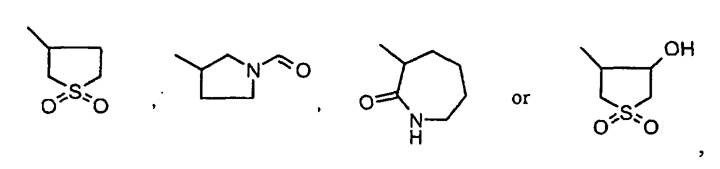
or
represents phenyl which is optionally substituted, up to 3 times, identically or differently, by fluorine, (C₁-C₄)-alkoxy or hydroxyl, by a radical of the formula 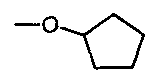 or by (C₁-C₄)-alkyl which can, for its part, be substituted by hydroxyl or (C₁-C₃)-alkoxy,
or
R³ and R⁴, together with the nitrogen atom to which they are bonded, form a radical of the formula 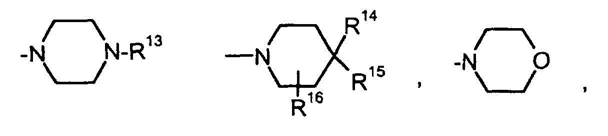 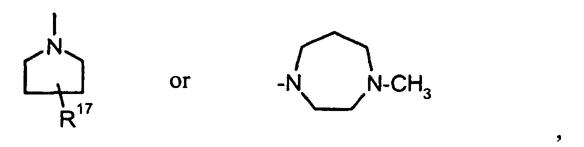 in which
R¹³ denotes hydrogen, (C₁-C₄)-alkoxycarbonyl, cyclopentyl, cyclohexyl, pyridyl, pyrimidyl or (C₁-C₅)-alkyl which is optionally substituted by hydroxyl,
R¹⁴ and R¹⁵ are identical or different and denote hydrogen or (C₁-C₅)-alkyl which is optionally substituted by hydroxyl or by a radical of the formula -P(O)(OR¹⁸)(OR¹⁹),
in which
R¹⁸ and R¹⁹ are identical or different and denote hydrogen, methyl or ethyl,
or
R¹⁴ and R¹⁵ together form a radical of the formula =N-OH,
R¹⁶ and R¹⁷ are identical or different and denote hydrogen, hydroxyl or (C₁-C₃)-alkyl which is optionally substituted by hydroxyl,
and the salts, N-oxides and stereoisomeric forms thereof.

3. Novel triazolotriazinone of the general formula (I) according to Claim 1,
in which
R¹ represents cyclopentyl,
R² represents straight-chain or branched alkyl having up to 3 carbon atoms,
R³ and R⁴ are identical or different and represent hydrogen or methoxy or represent (C₁-C₄)-alkyl which is optionally substituted, up to 3 times, identically or differently, by hydroxyl, (C₁-C₄)-alkoxy or phenoxy or by groups of the formulae
―O-CO-NR⁵R⁶ ,
―NR⁷R⁸
or 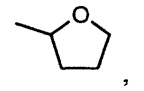 in which
R⁵, R⁶, R⁷ and R⁸ are identical or different and denote hydrogen, (C₁-C₃)-alkyl or phenyl,
or
R⁷ and R⁸, together with the nitrogen atom to which they are bonded, form a morpholine, piperidine or pyrrolidine ring,
and/or (C₁-C₄)-alkyl is, for its part, optionally substituted by phenyl which is optionally substituted, up to 3 times, identically or differently, by hydroxyl, (C₁-C₃)-alkoxy or fluorine or by (C₁-C₃)-alkyl which is for its part in turn substituted by hydroxyl or (C₁-C₄)-alkoxy, or phenyl is optionally substituted by radicals of the formulae -SO₂-NR⁹R¹⁰ or -NR¹¹R¹²,
in which
R⁹, R¹⁰, R¹¹ and R¹² are identical or different and denote hydrogen, (C₁-C₃)-alkyl or phenyl,
or
R¹¹ and R¹², together with the nitrogen atom to which they are bonded, form a morpholine, piperidine or pyrrolidine ring,
or
R³ represents hydrogen or methyl,
and
R⁴ represents radicals of the formula 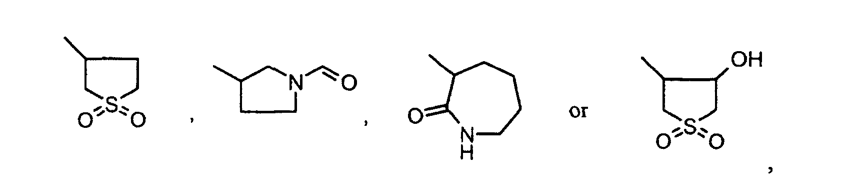
or
represents phenyl which is optionally substituted, up to 3 times, identically or differently, by fluorine, methoxy or hydroxyl, by a radical of the formula 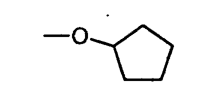 or by (C₁-C₄)-alkyl which, for its part, can be substituted by hydroxyl or methoxy or ethoxy,
or
R³ and R⁴, together with the nitrogen atom to which they are bonded, form a radical of the formula 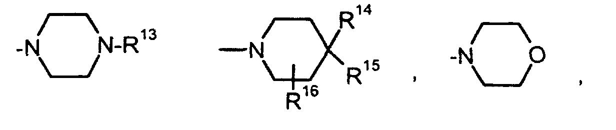 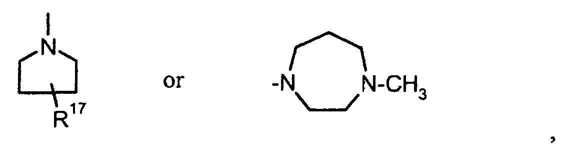 in which
R¹³ denotes hydrogen, (C₁-C₄)-alkoxycarbonyl, cyclopentyl, pyrimidyl or (C₁-C₃)-alkyl which is optionally substituted by hydroxyl,
R¹⁴ and R¹⁵ are identical or different and denote (C₁-C₃)-alkyl which is optionally substituted by hydroxyl or by a radical of the formula -P(O)(OR¹⁸)(OR¹⁹),
in which
R¹⁸ and R¹⁹ denote ethyl,
or
R¹⁴ and R¹⁵ together form a radical of the formula =N-OH,
R¹⁶ and R¹⁷ are identical or different and denote hydrogen or (C₁-C₃)-alkyl which is optionally substituted by hydroxyl,
and the salts, N-oxides and stereoisomeric forms thereof.

4. Novel triazolotriazinone of the general formula (I) according to Claims 1 to 3 and possessing one of the following structures:

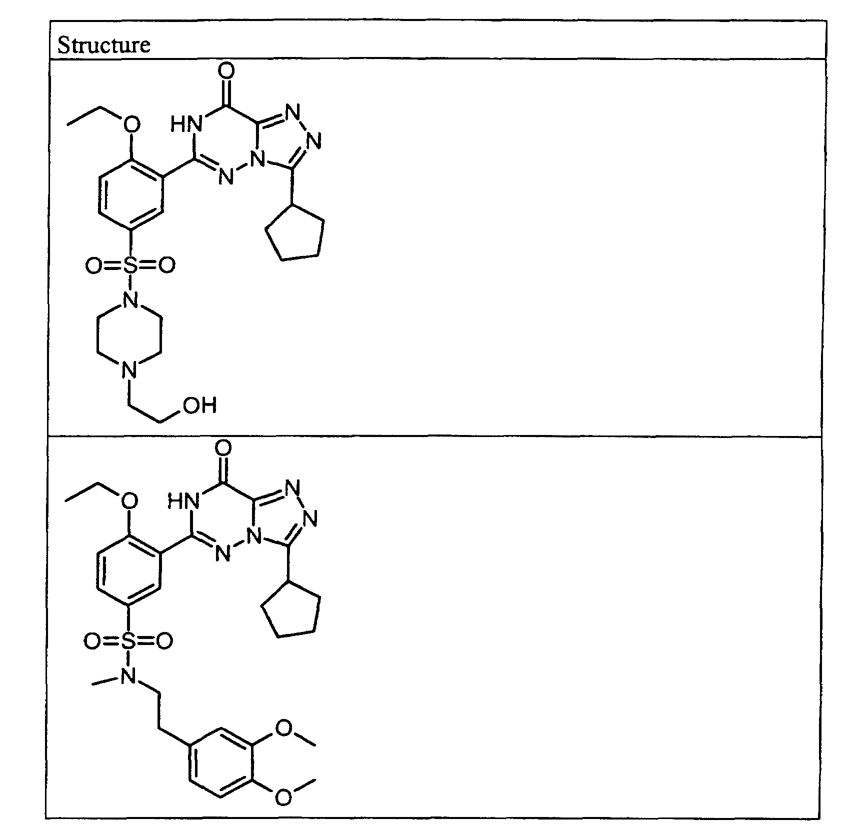

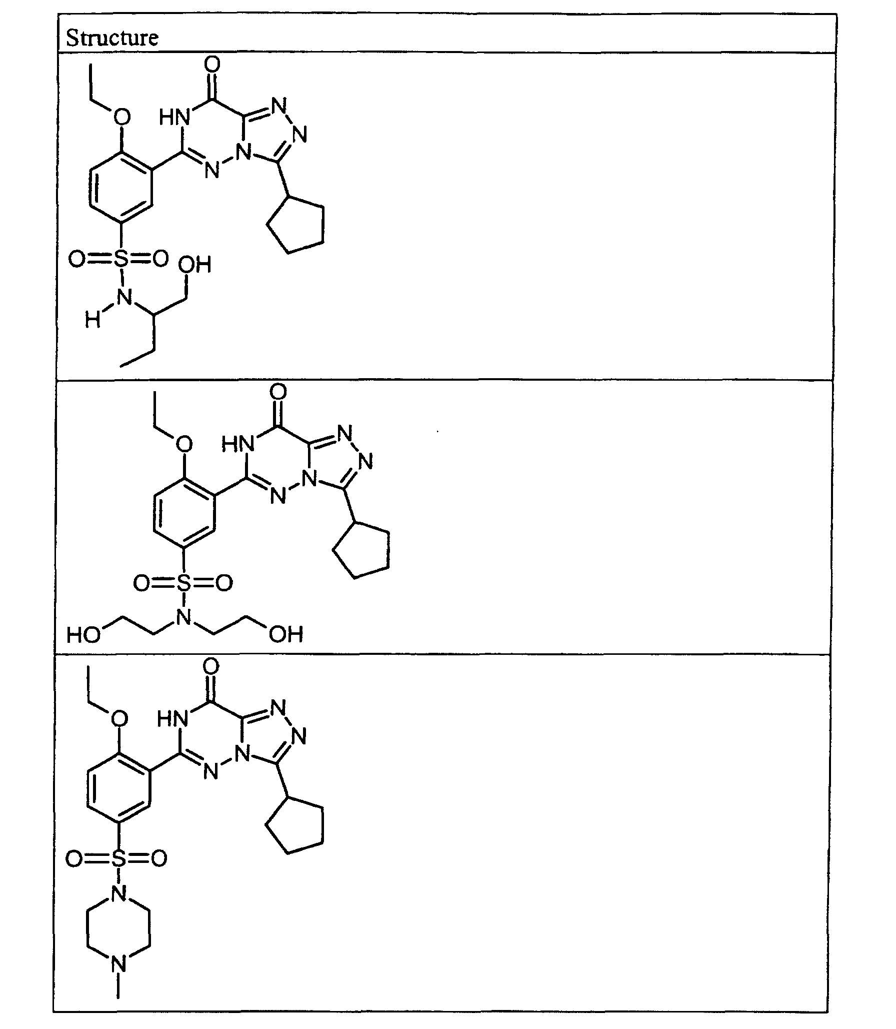

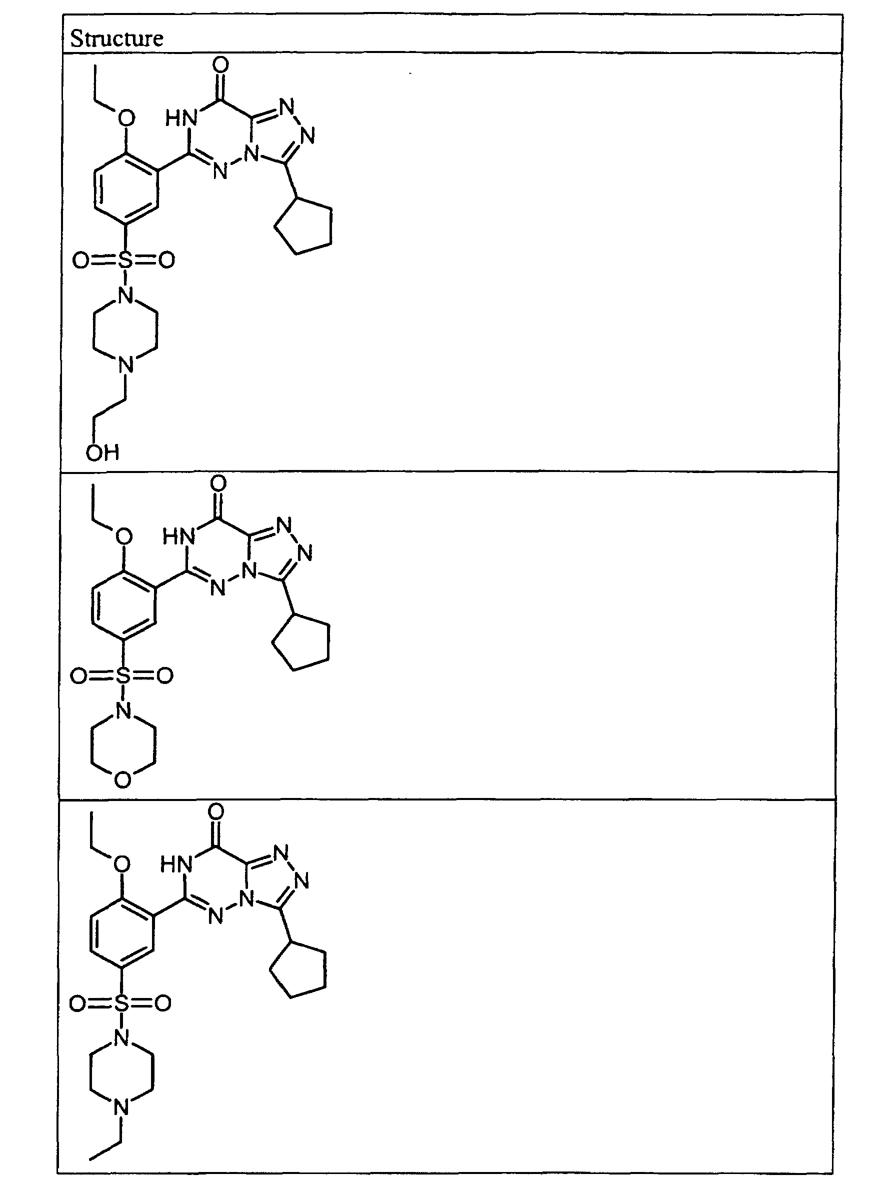

5. Process for preparing triazolotriazinones according to Claims 1 to 4, **characterized in that** compounds of the general formula (II) 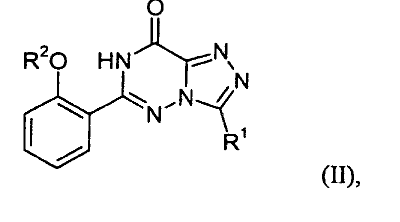 in which
R¹ and R² have the abovementioned meaning,
are reacted with chlorosulfonic acid (ClSO₃H), where appropriate in inert solvents and where appropriate in the presence of a base, to give the compounds of the general formula (III) 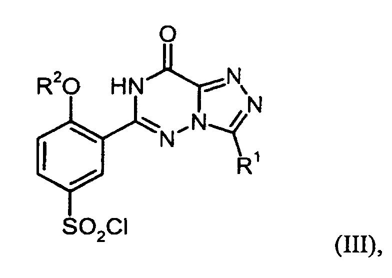 in which
R¹ and R² have the abovementioned meaning,
and subsequently reacted with amines of the general formula (IV)
HNR³R⁴ (IV),
in which
R³ and R⁴ have the abovementioned meaning.

6. Compound of the general formula (I) according to Claims 1 to 4 for the prophylaxis and/or treatment of diseases.

7. Medicament or pharmaceutical composition which comprises at least one compound of the general formula (I) according to one of Claims 1 to 4 and also one or more pharmacologically harmless auxiliary substances and carrier substances.

8. Medicament or pharmaceutical composition according to Claim 7 for the prophylaxis and/or treatment of diseases which are connected to cGMP-regulated processes (cGMP-related diseases).

9. Medicament or pharmaceutical composition according to Claim 7 or 8 for the prophylaxis and/or treatment of cardiovascular diseases, diseases of the urogenital system and cerebrovascular diseases.

10. Medicament or pharmaceutical composition according to one of Claims 7 to 9 for the prophylaxis and/or treatment of cardiovascular diseases such as high blood pressure, neuronal hypertension, stable and unstable angina, peripheral and cardiac vascular diseases, arrhythmias, thromboembolic diseases and ischemias such as myocardial infarction, stroke, transistory and ischemic attacks, angina pectoris, peripheral circulatory disturbances, prevention of restenoses following thrombolysis therapy, percutaneous transluminal angioplasty (PTA), percutaneous transluminal corony arangioplasties (PTCA) and bypass.

11. Medicament or pharmaceutical composition according to one of Claims 7 to 9 for the prophylaxis and/or treatment of cerebrovascular diseases such as cerebral ischemia, stroke, reperfusion damage, brain trauma, edemas, cerebral thrombosis, dementia and Alzheimer's disease.

12. Medicament or pharmaceutical composition according to one of Claims 7 to 9 for the prophylaxis and/or treatment of diseases of the urogenital system such as prostatate hypertrophy, incontinence and, in particular, erectile dysfunction and female sexual dysfunction.

13. Medicament or pharmaceutical composition according to one of Claims 7 to 12, **characterized in that** the medicament or the pharmaceutical composition is administered intravenously or orally.

14. Use of the compounds of the general formula (I) according to one of Claims 1 to 4 for producing medicaments or pharmaceutical compositions for the prophylaxis and/or treatment of diseases.

15. Use according to Claim 14 for producing a medicament or a pharmaceutical composition for the prophylaxis and/or treatment of diseases which are connected to cGMP-regulated processes (cGMP-related diseases).

16. Use according to Claim 14 or 15 for producing a medicament or a pharmaceutical composition for the prophylaxis and/or treatment of cardiovascular diseases, diseases of the urogenital system and cerebrovascular diseases.

17. Use according to one of Claims 14 to 16 for producing a medicament or a pharmaceutical composition for the prophylaxis and/or treatment of cardiovascular diseases such as high blood pressure, neuronal hypertension, stable and unstabile angina, peripheral and cardiac vascular diseases, arrhythmias, thromboembolic diseases and ischemias such as myocardial infarction, stroke, transistory and ischemic attacks, angina pectoris, peripheral circulatory disturbances, prevention of restenoses following thrombolysis therapy, percutaneous transluminal angioplasty (PTA), percutaneous transluminal coronary angioplasties (PTCA) and bypass.

18. Use according to one of Claims 14 to 16 for producing a medicament or a pharmaceutical composition for the prophylaxis and/or treatment of cerebrovascular diseases such as cerebral ischemia, stroke, reperfusion damage, brain trauma, edemas, cerebral thrombosis, dementia and Alzheimer's disease.

19. Use according to one of Claims 14 to 16 for producing a medicament or a pharmaceutical composition for the prophylaxis and/or treatment of diseases of the urogenital system such as prostate hypertrophy, incontinence and, in particular, erectile dysfunction and female sexual dysfunction.

20. Use according to one of Claims 14 to 19, **characterized in that** the medicaments or compositions are administered intravenously or orally.

## Revendications

1. Nouvelles triazolotriazinones de formule générale (I) 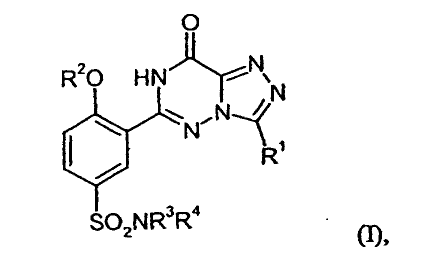 dans laquelle
R¹ est un reste cycloalkyle en C₃ à C₈,
R² représente l'hydrogène ou un reste alkyle linéaire ou ramifié ayant jusqu'à 6 atomes de carbone,
R³ et R⁴ sont identiques ou différents et représentent l'hydrogène, un reste alkoxy en C₁ à C₆ ou un reste alkyle en C₁ à C₆, qui est éventuellement substitué jusqu'à trois fois identiques ou différentes par un radical hydroxy, alkoxy en C₁ à C₅, phénoxy ou par des restes de formules
―O-CO-NR⁵R⁶ ,
―NR⁷R⁸
ou 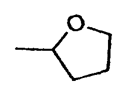 où
R⁵, R⁶, R⁷ et R⁸ sont identiques ou différents et représentent l'hydrogène, un reste alkyle en C₁ à C₆ ou un reste phényle,
ou bien
R⁷ et R⁸ forment, conjointement avec l'atome d'azote auquel ils sont liés, un hétérocycle saturé pentagonal ou hexagonal qui peut encore contenir un autre hétéroatome de la série S et O,
et/ou le reste alkyle en C₁ à C₆ est lui-même éventuellement substitué par un radical phényle qui est éventuellement substitué jusqu'à trois fois identiques ou différentes par un radical hydroxy, alkoxy en C₁ à C₆, halogéno ou par un radical alkyle en C₁ à C₆ qui est lui-même substitué à son tour par un radical hydroxy ou alkoxy en C₁ à C₆, ou bien le radical phényle est éventuellement substitué par des restes de formules -SO₂-NR⁹R¹⁰ ou -NR¹¹R¹²,
où
R⁹, R¹⁰, R¹¹ et R¹² sont identiques ou différents et représentent l'hydrogène, un radical alkyle en C₁ à C₆ ou phényle,
ou bien
R¹¹ et R¹² forment, conjointement avec l'atome d'azote auquel ils sont liés, un hétérocycle saturé pentagonal ou hexagonal qui peut encore contenir un autre hétéroatome de la série S et O;
ou bien
R³ représente l'hydrogène ou un reste alkyle en C₁ à C₆,
et
R⁴ représente des restes de formule 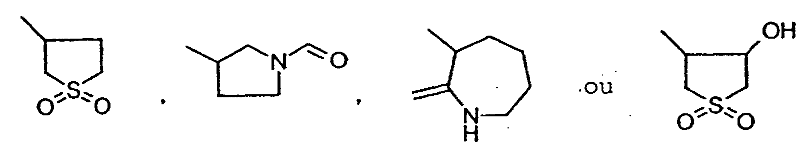
ou bien
un reste phényle, qui est éventuellement substitué jusqu'à trois fois identiques ou différentes par un radical halogéno, alkoxy en C₁ à C₆, hydroxy, par un reste de formule 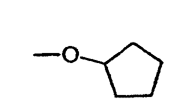 ou par un reste alkyle en C₁ à C₆ qui peut lui-même être substitué par un radical hydroxy ou alkoxy en C₁ à C₆,
ou bien
R³ et R⁴ forment, conjointement avec l'atome d'azote auquel ils sont liés, un reste de formule 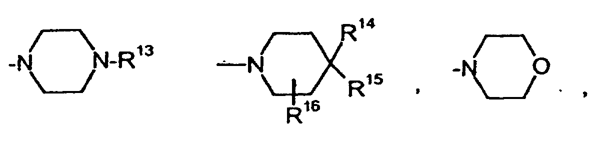 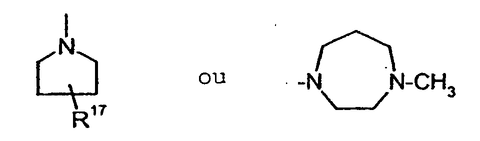 où
R¹³ représente l'hydrogène, un reste (alkoxy en C₁ à C₆)carbonyle, cycloalkyle en C₃ à C₆, pyridyle, pyrimidyle ou alkyle en C₁ à C₆, qui est éventuellement substitué par un radical hydroxy,
R¹⁴ et R¹⁵ sont identiques ou différents et représentent l'hydrogène, un groupe hydroxy ou un reste alkyle en C₁ à C₆, qui est éventuellement substitué par un radical hydroxy ou par un reste de formule -P(O)(OR¹⁸)(OR¹⁹),
où
R¹⁸ et R¹⁹ sont identiques ou différents et représentent l'hydrogène ou un reste alkyle en C₁ à C₆,
ou bien
R¹⁴ et R¹⁵ forment ensemble un reste de formule =N-OH, R¹⁶ et R¹⁷ sont identiques ou différents et représentent l'hydrogène ou un reste alkyle en C₁ à C₆ qui est éventuellement substitué par un radical hydroxy,
et leurs sels, leurs N-oxydes et leurs formes stéréo-isomères.

2. Nouvelles triazolotriazinones de formule générale (I) suivant la revendication 1,
formule dans laquelle
R¹ est un reste cyclopropyle, cyclopentyle ou cyclohexyle,
R² est un reste alkyle linéaire ou ramifié ayant jusqu'à 4 atomes de carbone,
R³ et R⁴ sont identiques ou différents et représentent l'hydrogène, un reste méthoxy ou un reste alkyle en C₁ à C₅ qui est éventuellement substitué jusqu'à trois fois, identiques ou différentes, par un radical hydroxy, alkoxy en C₁ à C₄, phénoxy ou par des groupes de formules
―O-CO-NR⁵R⁶ ,
―NR⁷R⁸
ou 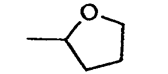
où
R⁵, R⁶, R⁷ et R⁸ sont identiques ou différents et représentent l'hydrogène, un reste alkyle en C₁ à C₄ ou un reste phényle,
ou bien
R⁷ et R⁸ forment, conjointement avec l'atome d'azote auquel ils sont liés, un noyau morpholine, pipéridine ou pyrrolidine,
et/ou le reste alkyle en C₁ à C₅ est lui-même éventuellement substitué par un radical phényle qui peut éventuellement être substitué jusqu'à trois fois identiques ou différentes par un radical hydroxy, alkoxy en C₁ à C₄ ou par un radical alkyle en C₁ à C₄ qui est lui-même à son tour substitué par un radical hydroxy ou alkoxy en C₁ à C₄, ou bien le radical phényle est éventuellement substitué par des restes de formules -SO₂-NR⁹R¹⁰ ou -NR¹¹R¹², où
R⁹, R¹⁰, R¹¹ et R¹² sont identiques ou différents et représentent l'hydrogène, un reste alkyle en C₁ à C₄ ou phényle,
ou bien
R¹¹ et R¹² forment, conjointement avec l'atome d'azote auquel ils sont liés, un noyau morpholine, pipéridine ou pyrrolidine,
ou bien
R³ représente l'hydrogène ou un reste alkyle en C₁ à C₄,
et
R⁴ représente des restes de formule 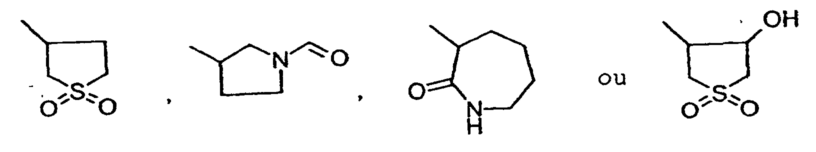
ou représente un reste phényle, qui est éventuellement substitué jusqu'à trois fois identiques ou différentes par du fluor, un reste alkoxy en C₁ à C₄, hydroxy, par un reste de formule 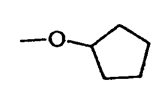 ou par un reste alkyle en C₁ à C₄ qui peut lui-même être substitué par un radical hydroxy ou alkoxy en C₁ à C₃,
ou bien,
R³ et R⁴ forment, conjointement avec l'atome d'azote auquel ils sont liés, un reste de formule 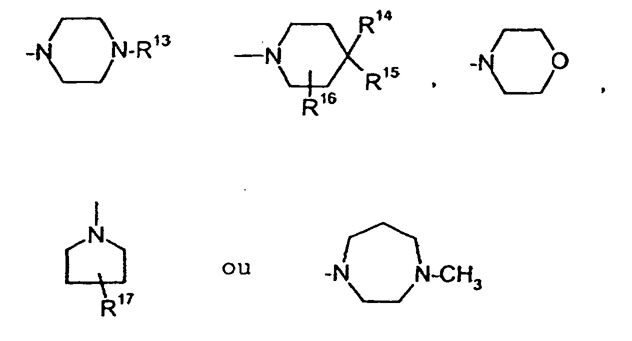 où
R¹³ représente l'hydrogène, un reste (alkoxy en C₁ à C₄)carbonyle, cyclopentyle, cyclohexyle, pyridyle, pyrimidyle ou alkyle en C₁ à C₅, qui est éventuellement substitué par un radical hydroxy,
R¹⁴ et R¹⁵ sont identiques ou différents et représentent l'hydrogène ou un reste alkyle en C₁ à C₅ qui est éventuellement substitué par un radical hydroxy ou par un reste de formule -P(O) (OR¹⁸) (OR¹⁹),
où
R¹⁸ et R¹⁹ sont identiques ou différents et représentent l'hydrogène, un reste méthyle ou éthyle,
ou bien
R¹⁴ et R¹⁵ forment ensemble un reste de formule =N-OH,
R¹⁶ et R¹⁷ sont identiques ou différents et représentent l'hydrogène, un reste hydroxy ou un reste alkyle en C₁ à C₃ qui est éventuellement substitué par un radical hydroxy,
et leurs sels, leurs N-oxydes et leurs formes stéréo-isomères.

3. Nouvelles triazolotriazinones de formule générale (I) suivant la revendication 1,
formule dans laquelle
R¹ est un reste cyclopentyle,
R² est un reste alkyle linéaire ou ramifié ayant jusqu'à 3 atomes de carbone,
R³ et R⁴ sont identiques ou différents et représentent l'hydrogène, un reste méthoxy ou un reste alkyle en C₁ à C₄ qui est substitué éventuellement jusqu'à trois fois identiques ou différentes par un radical hydroxy, alkoxy en C₁ à C₄, phénoxy ou par des groupes de formules
―O-CO-NR⁵R⁶ ,
―NR⁷R⁸
ou 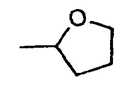 où
R⁵, R⁶, R⁷ et R⁸ sont identiques ou différents et représentent l'hydrogène, un reste alkyle en C₁ à C₃ ou un reste phényle,
ou bien
R⁷ et R⁸ forment, conjointement avec l'atome d'azote auquel ils sont liés, un noyau morpholine, pipéridine ou pyrrolidine,
et/ou le reste alkyle en C₁ à C₄ est lui-même éventuellement substitué par un radical phényle qui est éventuellement substitué jusqu'à trois fois identiques ou différentes par un radical hydroxy, alkoxy en C₁ à C₃, fluoro ou par un radical alkyle en C₁ à C₃ qui est lui-même substitué à son tour par un radical hydroxy ou alkoxy en C₁ à C₄, ou bien le radical phényle est éventuellement substitué par des restes de formules -SO₂-NR⁹R¹⁰ ou -NR¹¹R¹²,
où
R⁹, R¹⁰, R¹¹ et R¹² sont identiques ou différents et représentent l'hydrogène, un reste alkyle en C₁ à C₃ ou phényle,
ou bien
R¹¹ et R¹² forment, conjointement avec l'atome d'azote auquel ils sont liés, un noyau morpholine, pipéridine ou pyrrolidine,
ou bien
R³ est l'hydrogène ou un reste méthyle,
et
R⁴ représente des restes de formule 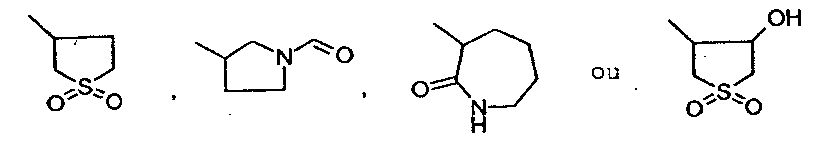
ou bien représente un reste phényle qui est éventuellement substitué jusqu'à trois fois identiques ou différentes par du fluor, un radical méthoxy, hydroxy, ou par un reste de formule 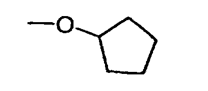 ou par un radical alkyle en C₁ à C₄ qui peut lui-même être substitué par un radical hydroxy ou méthoxy ou éthoxy,
ou bien
R³ et R⁴ forment, conjointement avec l'atome d'azote auquel ils sont liés, un reste de formule 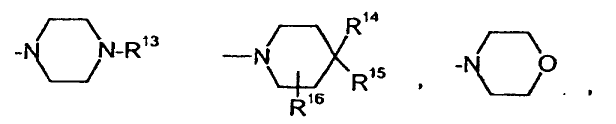 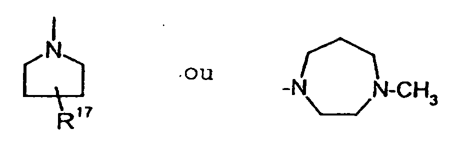 où
R¹³ représente l'hydrogène, un reste (alkoxy en C₁ à C₄)carbonyle, cyclopentyle, pyrimidyle ou un reste alkyle en C₁ à C₃ qui est éventuellement substitué par un radical hydroxy,
R¹⁴ et R¹⁵ sont identiques ou différents et représentent un reste alkyle en C₁ à C₃ qui est éventuellement substitué par un radical hydroxy ou par un reste de formule -P(O)(OR¹⁸)(OR¹⁹),
où
R¹⁸ et R¹⁹ représentent des restes éthyle,
ou bien
R²⁴ et R¹⁵ forment ensemble un reste de formule =N-OH,
R¹⁶ et R¹⁷ sont identiques ou différents et représentent l'hydrogène ou un reste alkyle en C₁ à C₃ qui est éventuellement substitué par un radical hydroxy,
et leurs sels, leurs N-oxydes et leurs formes stéréo-isomères.

4. Nouvelles triazolotriazinones de formule générale (I), suivant la revendication 1 à 3, de structures suivantes :

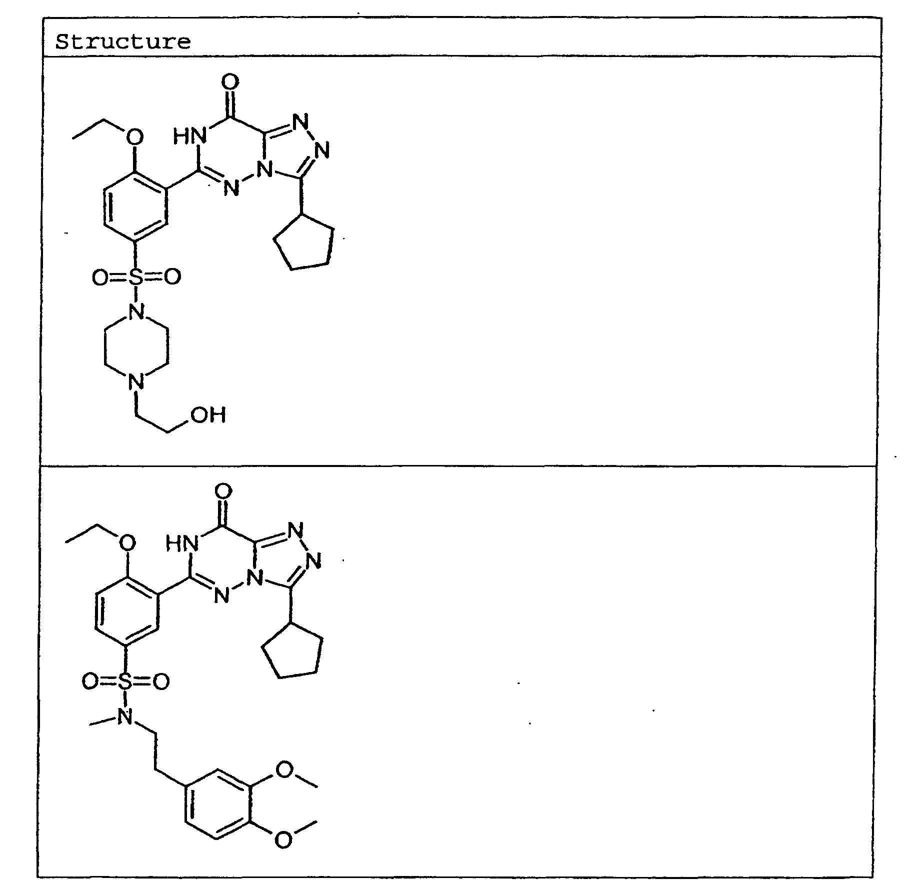

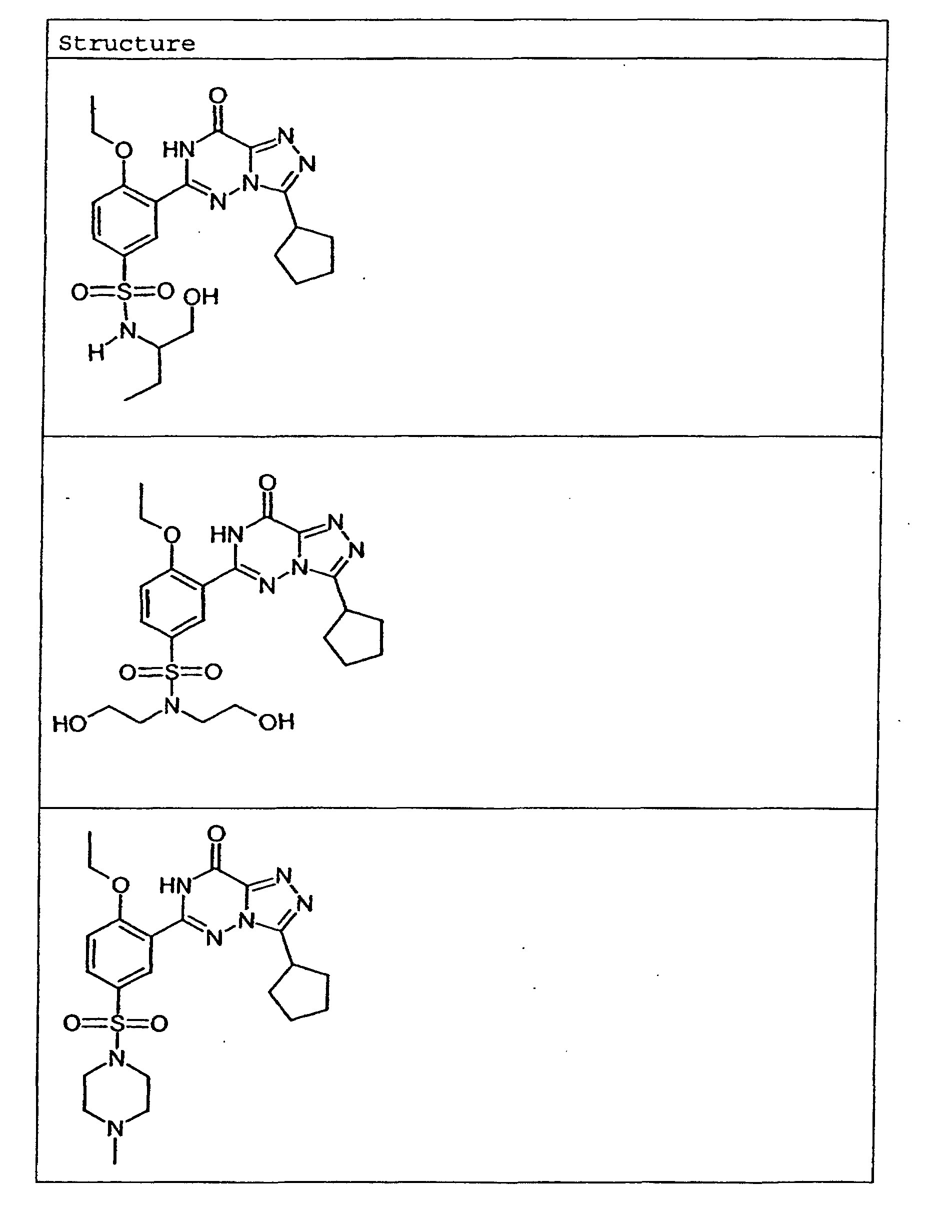

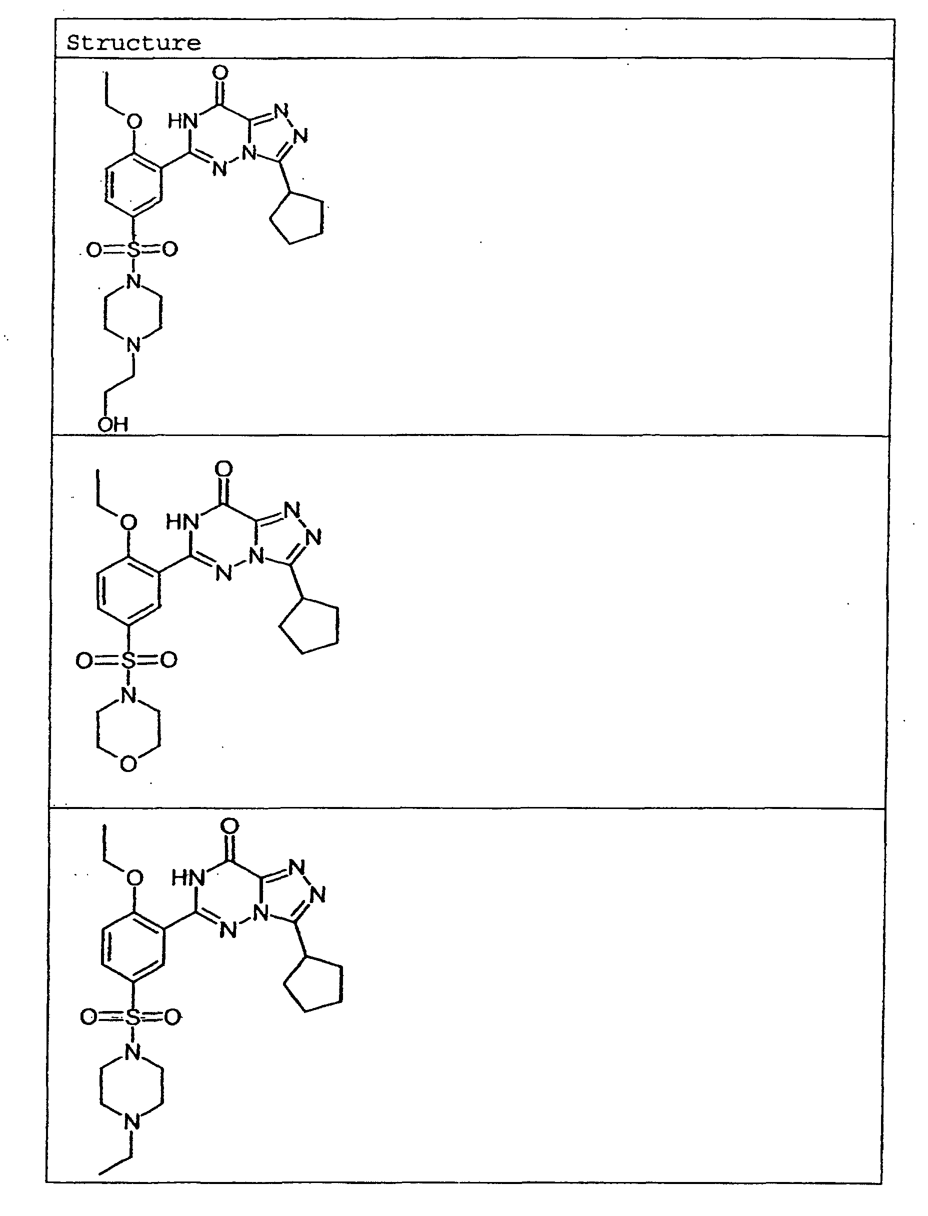

5. Procédé de production de triazolotriazinones suivant les revendications 1 à 4, **caractérisé en ce qu'**on fait réagir des composés de formule générale (II) 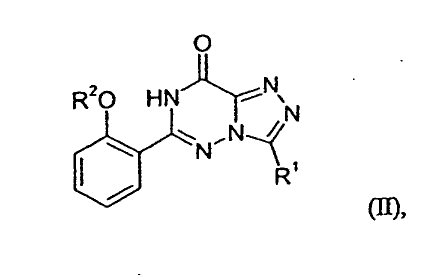 dans laquelle
R¹ et R² ont la définition indiquée ci-dessus,
avec l'acide chlorosulfonique (ClSO₃H), éventuellement dans des solvants inertes, le cas échéant en présence d'une base, pour obtenir les composés de formule générale (III) 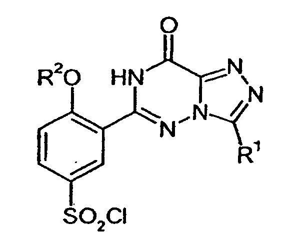 dans laquelle
R¹ et R² ont la définition indiquée ci-dessus,
qu'on fait réagir finalement avec des amines de formule générale (IV)
HNR³R⁴ (IV),
dans laquelle
R³ et R⁴ ont la définition indiquée ci-dessus.

6. Composés de formule générale (I) suivant la revendication 1 à 4, destinés à la prophylaxie et/ou au traitement de maladies.

7. Médicament ou préparation pharmaceutique, contenant au moins un composé de formule générale (I) suivant l'une des revendications 1 à 4 ainsi qu'une ou plusieurs substances auxiliaires et un ou plusieurs supports acceptables du point de vue pharmacologique.

8. Médicament ou préparation pharmaceutique suivant la revendication 7, destiné à la prophylaxie et/ou au traitement de maladies qui sont en rapport avec des processus régulés par le cGMP ("cGMP-related diseases").

9. Médicament ou préparation pharmaceutique suivant la revendication 7 ou 8, destiné à la prophylaxie et/ou au traitement de maladies cardiovasculaires, de maladies du système urogénital ainsi que de maladies cérébrovasculaires.

10. Médicament ou préparation pharmaceutique suivant l'une des revendications 7 à 9, destiné à la prophylaxie et/ou au traitement de maladies cardiovasculaires telles que l'hypertension, l'hypertonie neuronale, l'angor stable et instable, les maladies vasculaires périphériques et cardiaques, les arythmies, des maladies thromboemboliques et des ischémies telles que l'infarctus du myocarde, l'apoplexie cérébrale, les attaques transitoires et ischémiques, l'angine de poitrine, des troubles de l'irrigation périphérique, l'inhibition de resténoses après une thrombolyse, l'angiopathie transluminale percutanée (PTA), des angioplasties coronariennes transluminales percutanées (PTCA) et un pontage.

11. Médicament ou préparation pharmaceutique suivant l'une des revendications 7 à 9, destiné à la prophylaxie et/ou au traitement de maladies cérébrovasculaires telles que l'ischémie cérébrale, l'apoplexie cérébrale, les atteintes liées à la reperfusion, le traumatisme cérébral, l'oedème, la thrombose cérébrale, la démence et la maladie d'Alzheimer.

12. Médicament ou préparation pharmaceutique suivant l'une des revendications 7 à 9, destiné à la prophylaxie et/ou au traitement de maladies des voies urogénitales telles que l'hypertrophie de la prostate, l'incontinence ainsi que, notamment, les troubles de l'érection et le dysfonctionnement sexuel chez la femme.

13. Médicament ou préparation pharmaceutique suivant l'une des revendications 7 à 12, **caractérisé en ce que** le médicament ou la préparation pharmaceutique est administré par voie intraveineuse ou orale.

14. Utilisation de composés de formule générale (I) suivant l'une des revendications 1 à 4 pour la préparation de médicaments ou de préparations pharmaceutiques destinés à la prophylaxie et/ou au traitement de maladies.

15. Utilisation suivant la revendication 14, pour la préparation d'un médicament ou d'une préparation pharmaceutique destiné à la prophylaxie et/ou au traitement de maladies qui sont en rapport avec des processus régulés par le cGMP ("cGMP-related diseases").

16. Utilisation suivant la revendication 14 ou 15, pour la préparation d'un médicament ou d'une préparation pharmaceutique destiné à la prophylaxie et/ou au traitement de maladies cardiovasculaires, de maladies des voies urogénitales ainsi que de maladies cérébrovasculaires.

17. Utilisation suivant l'une des revendications 14 à 16, pour la préparation d'un médicament ou d'une préparation pharmaceutique destiné à la prophylaxie et/ou au traitement de maladies cardiovasculaires telles que l'hypertension, l'hypertonie neuronale, l'angor stable et instable, les maladies vasculaires périphériques et cardiaques, les arythmies, des maladies thromboemboliques et des ischémies telles que l'infarctus du myocarde, l'apoplexie cérébrale, les attaques transitoires et ischémiques, l'angine de poitrine, des troubles de l'irrigation périphérique, l'inhibition de resténoses après une thrombolyse, l'angiopathie transluminale percutanée (PTA), des angioplasties coronariennes transluminales percutanées (PTCA) et un pontage.

18. Utilisation suivant l'une des revendications 14 à 16, pour la préparation d'un médicament ou d'une préparation pharmaceutique destiné à la prophylaxie et/ou au traitement de maladies cérébrovasculaires telles que l'ischémie cérébrale, l'apoplexie cérébrale, les atteintes consécutives à la reperfusion, le traumatisme cérébral, l'oedème, la thrombose cérébrale, la démence et la maladie d'Alzheimer.

19. Utilisation suivant l'une des revendications 14 à 16, pour la préparation d'un médicament ou d'une préparation pharmaceutique destiné à la prophylaxie et/ou au traitement de maladies des voies urogénitales telles que l'hypertrophie de la prostate, l'incontinence ainsi que, notamment, les troubles de l'érection et le dysfonctionnement sexuel chez la femme.

20. Utilisation suivant l'une des revendications 14 à 19, **caractérisée en ce que** le médicament ou les préparations sont administrés par voie intraveineuse ou orale.
